# EUROPEAN PATENT APPLICATION

(11) **EP 3 799 884 A1**
(43) Date of publication of application: **07.04.2021**
(21) Application number: 19200878.7
(22) Date of filing: 01.10.2019
(51) Int. Cl.: A61K 39/02, C07K 14/235

(54) **IMMUNOGENIC COMPOSITIONS**

(71) Applicant: GlaxoSmithKline Biologicals S.A., 1330 Rixensart (BE)
(72) Inventor: The designation of the inventor has not yet been filed
(74) Representative: Evans, Stephen John Eves

(57) **Abstract**

The invention relates to bacterial strains, particularly for use in the field of vaccines, in particular to the field of the prevention or treatment of infections caused by bacterium of the *Bordetella* genus.

SEQUENCE LISTING

<110>  GlaxoSmithKline Biologicals S.A.

<120>  Immunogenic Compositions

<130>  VB66722P EP

<160>  21

<170>  PatentIn version 3.5

<210>  1
<211>  264
<212>  PRT
<213>  Bordetella pertussis

<400>  1

Met Ser Gly Asn Ile His Pro Thr Ala Val Val Asp Pro Ala Ala Gln
1               5                   10                  15

Ile Asp Ser Ser Val Val Ile Gly Pro Tyr Ser Val Val Gly Pro Gly
            20                  25                  30

Val Ser Ile Ala Ala Gly Thr Glu Val Gly Ala His Cys Val Leu Asp
        35                  40                  45

Gly Val Thr Ser Ile Gly Arg Asp Asn Arg Phe Tyr Arg Phe Cys Ser
    50                  55                  60

Ile Gly Gly Met Pro Gln Asp Lys Lys Tyr Ser Gly Glu Pro Thr Arg
65                  70                  75                  80

Leu Val Ile Gly Asp Arg Asn Thr Val Arg Glu Phe Thr Thr Phe Asn
                85                  90                  95

Thr Gly Thr Val Gln Asp Gly Gly Val Thr Ser Ile Gly Asp Asp Asn
            100                 105                 110

Trp Ile Met Ala Tyr Val His Ile Ala His Asp Cys His Ile Gly Asn
        115                 120                 125

Asn Thr Ile Leu Ala Asn Ser Val Gln Leu Gly Gly His Val Gln Val
    130                 135                 140

Gly Asp Trp Ala Ile Val Gly Gly Leu Thr Gly Val His Gln Phe Ala
145                 150                 155                 160

Lys Ile Gly Ala His Ser Met Thr Gly Gly Asn Ser Ser Leu Met Gln
                165                 170                 175

Asp Ala Pro Pro Phe Val Leu Ala Ala Gly Asn Pro Cys Arg Pro Val
            180                 185                 190

Gly Val Asn Val Glu Gly Leu Lys Arg Arg Gly Phe Ser Ala Ala Ala
        195                 200                 205

Ile Ser Ala Leu Arg Asp Ala Tyr Lys Ser Ile Tyr Arg Arg Gly Leu
    210                 215                 220

Ser Leu Asp Glu Gly Arg Ala Glu Leu Arg Ala Arg Gln Gln Ala Glu
225                 230                 235                 240

Pro Asp Val Ala Glu His Leu Gln Thr Met Leu Asp Phe Leu Asp Ala
                245                 250                 255

Ser Thr Arg Gly Ile Ile Arg Pro
            260

<210>  2
<211>  264
<212>  PRT
<213>  Bordetella parapertussis

<400>  2

Met Ser Gly Asn Ile His Pro Thr Ala Val Val Asp Pro Ala Ala Gln
1               5                   10                  15

Ile Asp Ser Ser Val Val Ile Gly Pro Tyr Ser Val Val Gly Pro Gly
            20                  25                  30

Val Ser Ile Ala Ala Gly Thr Glu Val Gly Ala His Cys Val Leu Asp
        35                  40                  45

Gly Val Thr Ser Ile Gly Arg Asp Asn Arg Phe Tyr Arg Phe Cys Ser
    50                  55                  60

Ile Gly Gly Met Pro Gln Asp Lys Lys Tyr Ser Gly Glu Pro Thr Arg
65                  70                  75                  80

Leu Val Ile Gly Asp Arg Asn Thr Val Arg Glu Phe Thr Thr Phe Asn
                85                  90                  95

Thr Gly Thr Val Gln Asp Gly Gly Val Thr Ser Ile Gly Asp Asp Asn
            100                 105                 110

Trp Ile Met Ala Tyr Val His Ile Ala His Asp Cys His Ile Gly Asn
        115                 120                 125

Asn Thr Ile Leu Ala Asn Ser Val Gln Leu Gly Gly His Val Gln Val
    130                 135                 140

Gly Asp Trp Ala Ile Val Gly Gly Leu Thr Gly Val His Gln Phe Ala
145                 150                 155                 160

Lys Ile Gly Ala His Ser Met Thr Gly Ser Asn Ser Ser Leu Met Gln
                165                 170                 175

Asp Ala Pro Pro Phe Val Leu Ala Ala Gly Asn Pro Cys Arg Pro Val
            180                 185                 190

Gly Val Asn Val Glu Gly Leu Lys Arg Arg Gly Phe Ser Ala Ala Ala
        195                 200                 205

Ile Ser Ala Leu Arg Asp Ala Tyr Lys Ser Ile Tyr Arg Arg Gly Leu
    210                 215                 220

Ser Leu Asp Glu Ala Arg Ala Glu Leu Arg Ala Arg Gln Gln Ala Glu
225                 230                 235                 240

Pro Asp Val Ala Glu His Leu Gln Thr Met Leu Asp Phe Leu Asp Ala
                245                 250                 255

Ser Thr Arg Gly Ile Ile Arg Pro
            260

<210>  3
<211>  333
<212>  PRT
<213>  Comamonas testosteroni

<400>  3

Met Ser Val Leu Leu Gly Gln Ile Leu Asp Ala Leu Gly Gly Glu Leu
1               5                   10                  15

Val Gly Gly Glu Arg Glu Met Pro Ile Ser Arg Ile Ala Pro Leu Asp
            20                  25                  30

Ser Ala Gly His Gly Asp Leu Ser Phe Leu Ser Asn Pro Arg Tyr Arg
        35                  40                  45

Gln Gln Leu Ala Ala Ser Gln Ala Ala Cys Val Ile Val Ala Pro Thr
    50                  55                  60

Met Arg Asp Glu Ala Ala Gln Arg Gly Ala Cys Ile Val Thr Ala Asp
65                  70                  75                  80

Pro Tyr Ala Tyr Phe Ala Arg Ala Thr Gln Trp Trp Lys Ala His Gln
                85                  90                  95

Gln Gly Gly Arg Pro Arg Gly Ile His Ala Ser Ala Val Val Asp Ala
            100                 105                 110

Thr Ala Gln Val His Glu Ser Ala Tyr Val Gly Pro Gln Cys Val Val
        115                 120                 125

Glu Ala Gly Ala Val Ile Gly Ala Asp Thr Val Leu Lys Ser Arg Val
    130                 135                 140

Thr Ile Ser Gln Gly Cys Val Leu Gly Glu Arg Cys Ile Leu His Pro
145                 150                 155                 160

Gly Val Val Ile Gly Ala Asp Gly Phe Gly Phe Ala Pro Ser Ala Gly
                165                 170                 175

Gln Trp Glu Lys Ile Glu Gln Leu Gly Ala Val Arg Ile Gly Asn Asp
            180                 185                 190

Val Glu Ile Gly Ala Asn Thr Cys Val Asp Arg Gly Ala Leu Asp Asp
        195                 200                 205

Thr Val Ile Glu Asp Gly Val Lys Ile Asp Asn Leu Val Gln Ile Ala
    210                 215                 220

His Asn Val His Ile Gly Ala His Thr Val Ile Ala Gly Asn Thr Gly
225                 230                 235                 240

Ile Ala Gly Ser Ala Arg Ile Gly Lys Arg Cys Gln Ile Gly Gly Ala
                245                 250                 255

Ala Asn Ile Leu Gly His Leu Thr Ile Ala Asp Gly Thr Val Ile Ser
            260                 265                 270

Pro Thr Ser Met Val Thr Arg Ser Leu Pro Lys Ala Gly Phe Tyr Thr
        275                 280                 285

Gly Ile Phe Pro Leu Gln Glu Asn Glu Gln Trp Glu Lys Asn Ala Ala
    290                 295                 300

Thr Phe Arg Gln Leu Tyr Thr Leu Arg Glu Arg Val Lys Lys Leu Glu
305                 310                 315                 320

Gln Ala Leu Ala Glu Gly Arg Gln Ser Asn Asn Gly Asn
                325                 330

<210>  4
<211>  353
<212>  PRT
<213>  Pseudomonas aeruginosa

<400>  4

Met Met Ser Thr Leu Ser Tyr Thr Leu Gly Gln Leu Ala Ala His Val
1               5                   10                  15

Gly Ala Glu Val Arg Gly Asp Ala Asp Leu Pro Ile Gln Gly Leu Ala
            20                  25                  30

Thr Leu Gln Glu Ala Gly Pro Ala Gln Leu Ser Phe Leu Ala Asn Pro
        35                  40                  45

Gln Tyr Arg Lys Tyr Leu Pro Glu Ser Arg Ala Gly Ala Val Leu Leu
    50                  55                  60

Thr Ala Ala Asp Ala Asp Gly Phe Ala Gly Thr Ala Leu Val Val Ala
65                  70                  75                  80

Asn Pro Tyr Leu Ala Tyr Ala Ser Leu Ser His Leu Phe Asp Arg Lys
                85                  90                  95

Pro Lys Ala Ala Ala Gly Ile His Pro Thr Ala Ile Val Ala Ala Asp
            100                 105                 110

Ala Glu Val Asp Pro Ser Ala Ser Val Gly Ala Tyr Ala Val Ile Glu
        115                 120                 125

Ser Gly Ala Arg Ile Gly Ala Gly Val Ser Ile Gly Ala His Cys Val
    130                 135                 140

Ile Gly Ala Arg Ser Val Ile Gly Glu Gly Gly Trp Leu Ala Pro Arg
145                 150                 155                 160

Val Thr Leu Tyr His Asp Val Thr Ile Gly Ala Arg Val Ser Ile Gln
                165                 170                 175

Ser Gly Ala Val Ile Gly Gly Glu Gly Phe Gly Phe Ala Asn Glu Lys
            180                 185                 190

Gly Val Trp Gln Lys Ile Ala Gln Ile Gly Gly Val Thr Ile Gly Asp
        195                 200                 205

Asp Val Glu Ile Gly Ala Asn Thr Thr Ile Asp Arg Gly Ala Leu Ser
    210                 215                 220

Asp Thr Leu Ile Gly Asn Gly Val Lys Leu Asp Asn Gln Ile Met Ile
225                 230                 235                 240

Ala His Asn Val Gln Ile Gly Asp His Thr Ala Met Ala Ala Cys Val
                245                 250                 255

Gly Ile Ser Gly Ser Ala Lys Ile Gly Arg His Cys Met Leu Ala Gly
            260                 265                 270

Gly Val Gly Leu Val Gly His Ile Glu Ile Cys Asp Asn Val Phe Val
        275                 280                 285

Thr Gly Met Thr Met Val Thr Arg Ser Ile Thr Glu Pro Gly Ser Tyr
    290                 295                 300

Ser Ser Gly Thr Ala Met Gln Pro Ala Ala Glu Trp Lys Lys Ser Ala
305                 310                 315                 320

Ala Arg Ile Arg Gln Leu Asp Asp Met Ala Arg Arg Leu Gln Gln Leu
                325                 330                 335

Glu Lys Arg Leu Ala Ala Val Thr Ser Ser Gly Asp Ala Ser Ser Asp
            340                 345                 350

Ala

<210>  5
<211>  178
<212>  PRT
<213>  Bordetella bronchiseptica

<400>  5

Met Gln Phe Leu Lys Lys Asn Lys Pro Leu Phe Gly Ile Val Thr Leu
1               5                   10                  15

Ala Leu Ala Cys Ala Thr Ala Gln Ala Gln Pro Thr Gln Gly Gly Val
            20                  25                  30

Ser Leu His Tyr Gly Ile Gly Asp His Tyr Gln Arg Val Thr Leu Asn
        35                  40                  45

Tyr Glu Thr Pro Thr Leu Trp Ser His Gln Phe Gly Gly Asn Trp Gly
    50                  55                  60

Arg Leu Asp Leu Thr Pro Glu Leu Gly Ala Ser Tyr Trp Trp Ala Asp
65                  70                  75                  80

Gly Ser Arg Ser Pro Gly His Val Trp Gln Ala Ser Ala Ile Pro Met
                85                  90                  95

Phe Arg Trp Trp Thr Gly Glu Arg Phe Tyr Ile Glu Ala Gly Ile Gly
            100                 105                 110

Ala Thr Val Phe Ser Ser Thr Ser Phe Ala Asp Lys Arg Ile Gly Ser
        115                 120                 125

Ala Phe Gln Phe Gly Asp His Ile Gly Leu Gly Phe Leu Leu Thr Pro
    130                 135                 140

Ser Asn Arg Ile Gly Leu Arg Tyr Ser His Phe Ser Asn Ala Gly Ile
145                 150                 155                 160

Lys Glu Pro Asn Pro Gly Leu Asp Ile Val Gln Leu Thr Tyr Thr Tyr
                165                 170                 175

Gln Phe

<210>  6
<211>  239
<212>  PRT
<213>  Francisella novicida

<400>  6

Met Leu Lys Gln Thr Leu Gln Thr Asn Phe Gln Gly Phe Lys Asp Ile
1               5                   10                  15

Phe Lys Lys Pro Lys Leu His Asn His Lys Leu Pro Arg Tyr Leu Gln
            20                  25                  30

Leu Lys Tyr Thr Phe Ile Pro Leu Leu Ile Leu Val Ile Phe Ala Tyr
        35                  40                  45

Tyr Asn Leu Asp Thr Pro Val Glu Asn Tyr Ile Lys His Ser Met Pro
    50                  55                  60

Asn Ile Val Gly Val Ile Phe Gly Lys Ile Thr Asp Val Gly Lys Ala
65                  70                  75                  80

Glu Tyr Ile Leu Ile Ile Cys Gly Val Ile Val Leu Ala Arg Leu Phe
                85                  90                  95

Thr Asp Ser Gln Lys Leu Ser Ala Asn Thr Arg Ala Met Phe Asp Lys
            100                 105                 110

Val Ser Ala Tyr Ala Gly Phe Ile Leu Ala Thr Val Ala Ile Ser Gly
        115                 120                 125

Ile Leu Gly Gln Ile Leu Lys Met Ile Ile Gly Arg Ala Arg Pro Lys
    130                 135                 140

Phe Phe Leu Glu Tyr Gly Ser His Tyr Phe Gln His Phe His Ala Pro
145                 150                 155                 160

Gly Tyr Asp Phe Ala Ser Met Pro Ser Gly His Ser Ile Thr Val Gly
                165                 170                 175

Ala Met Phe Ile Ala Phe Phe Tyr Ile Phe Pro Lys Leu Arg Tyr Phe
            180                 185                 190

Trp Tyr Leu Leu Ile Val Val Phe Ala Gly Ser Arg Ile Met Val Gly
        195                 200                 205

Ser His Tyr Pro Ser Asp Val Ile Phe Gly Val Ala Phe Gly Cys Tyr
    210                 215                 220

Cys Thr Ala Tyr Ile Tyr Tyr Trp Met Arg Asn Arg Glu Ile Ile
225                 230                 235

<210>  7
<211>  226
<212>  PRT
<213>  Rhizobium leguminosarum

<400>  7

Met Pro Pro Leu Arg Trp Gln Ala Cys Leu Leu Ile Thr Leu Asn Ala
1               5                   10                  15

Val Ile Leu Ser Met Leu Leu Phe Asp Ala Pro Ile Gly Ala Ser Glu
            20                  25                  30

Ala Pro Ala Pro Val Lys His Leu Gly Glu Ile Leu Thr Gly Phe Gly
        35                  40                  45

Asp Ser Ala Trp Leu Ile Tyr Thr Ser Ile Leu Leu Phe Phe Gln Gly
    50                  55                  60

Arg Ala Gly Tyr Lys Leu Val Lys Thr Ala Arg Ser Lys Ala Gln Ala
65                  70                  75                  80

Leu Tyr Val Ser Trp Ile Gly Ala Tyr Leu Phe Ile Thr Val Val Phe
                85                  90                  95

Ser Gly Leu Leu Ala Asn Leu Leu Lys Arg Ala Ile Gly Arg Ala Arg
            100                 105                 110

Pro Asp His Phe His Asp Tyr Gly Met Phe Ser Phe Thr Pro Phe Ser
        115                 120                 125

Gly His Ala Ala Phe Glu Ser Phe Pro Ser Gly His Ser Thr Thr Val
    130                 135                 140

Gly Ala Phe Phe Ala Ala Phe Ala Leu Leu Phe Pro Arg Tyr Arg Ile
145                 150                 155                 160

Ala Phe Ile Ala Cys Ala Ile Trp Leu Gly Met Thr Arg Val Met Val
                165                 170                 175

Gly Ala His Tyr Pro Ser Asp Val Ile Ala Gly Leu Ala Phe Gly Ala
            180                 185                 190

Trp Phe Ser Leu Leu Thr Ala Ile Val Phe Ala Arg Cys Gly Leu Leu
        195                 200                 205

Phe Lys Leu Ala Pro Asp Gly Trp Pro Leu Ser Lys Arg Leu Phe Arg
    210                 215                 220

Thr Ala
225

<210> 8
<211> 222
<212> PRT
<213> Francisella novicida

<400> 8

Met Ala Arg Phe His Ile Ile Leu Gly Leu Val Val Cys Phe Phe Ala
1               5                   10                  15

Trp Ile Phe Phe Leu Ile Phe Pro Asn Leu Asp Ile Gln Phe Ala Gly
            20                  25                  30

His Phe Tyr Asn Ser Ser Ala His Gln Phe Ile Gly Gly Tyr Asp Gly
        35                  40                  45

Phe Leu Gly Phe Leu His Trp Phe Ala Arg Phe Phe Pro Ile Phe Phe
    50                  55                  60

Ser Ile Ile Val Ile Leu Phe Leu Leu Gly Ser Leu Phe Ile Asp Lys
65                  70                  75                  80

Phe Lys Ile Lys Tyr Arg Lys Ala Ile Phe Phe Ile Ala Val Cys Leu
                85                  90                  95

Trp Ile Gly Pro Gly Leu Val Val Asn Tyr Val Phe Lys Asp His Trp
            100                 105                 110

Gly Arg Pro Arg Pro Val Met Val Glu Gln Phe Asn Gly Asp Lys Ile
        115                 120                 125

Phe Gln Pro Pro Phe Val Ile Ser Ser Gln Cys Asp Lys Asn Cys Ser
    130                 135                 140

Phe Val Cys Gly Asp Ala Ser Met Gly Phe Trp Leu Phe Ala Phe Met
145                 150                 155                 160

Pro Leu Leu Ala Thr Arg Lys Lys Lys Leu Val Ala Phe Ile Ala Ala
                165                 170                 175

Val Val Ala Gly Gly Gly Leu Gly Leu Met Arg Met Ser Gln Gly Gly
            180                 185                 190

His Phe Phe Ser Asp Val Val Phe Cys Gly Ile Phe Val Tyr Ile Ser
        195                 200                 205

Thr Trp Val Val Tyr Ala Leu Met Tyr Arg Lys Lys Glu Tyr
    210                 215                 220

<210>  9
<211>  795
<212>  DNA
<213>  Bordetella parapertussis

<400>  9
atgtcaggaa acatccatcc caccgccgtg gtcgatccgg cggcacagat cgacagttcg      60

gtcgtgatcg gcccatacag cgtggtgggg ccgggagtca gcattgccgc gggcaccgag     120

gtcggcgcgc actgcgtgct cgacggcgtg accagcatcg ggcgcgacaa ccgcttctac     180

cgcttttgct ccatcggcgg catgccgcag gacaagaagt acagcggcga accgacgcga     240

ctggtcatcg gcgaccgcaa cacggtgcgc gagttcacca cgttcaacac cggcacggtg     300

caggacggcg gcgtgaccag catcggcgac gacaactgga tcatggccta cgtgcacatc     360

gcgcacgact gccatatcgg caacaacacc atcctggcca actcggtgca gctgggcggc     420

cacgtgcagg tgggcgactg ggccatcgtg ggcggcctga ccggcgtgca ccagttcgcc     480

aagatcggcg cgcacagcat gaccggcagc aacagctcgc tgatgcagga tgcgcccccg     540

ttcgtgctgg cggcgggcaa tccgtgccgg cccgtgggcg tcaatgtcga aggcttgaag     600

cggcgcggtt tctcggcggc ggccatctcg gcgctgcgcg atgcgtacaa gtcgatctac 660

cggcgcggac tgtcgctgga cgaggcgcgc gccgagctgc gcgcgcgcca gcaggccgag 720

ccggatgtcg ccgagcatct gcagaccatg ctggacttcc tggacgcttc gacgcgcggc 780

atcatccggc catga 795

<210> 10
<211> 1002
<212> DNA
<213> Comamonas testosteroni

<400> 10
gtgagcgttc tattgggaca gattctcgat gcgctcggtg gagaactggt aggtggcgag 60

cgcgagatgc ctatctcgcg catcgctcca ctggactctg cgggccacgg tgatctgagc 120

tttttaagca atccccgcta tcgccagcaa ctggccgcct cacaggcggc ctgtgtcatt 180

gtggcgccga cgatgcgtga tgaagcagcg cagcgtggcg cctgcattgt cacggctgat 240

ccttatgcct atttcgcgcg agccacccag tggtggaagg cgcatcagca gggcggcagg 300

ccgcgcggca tccatgcgag tgcggtggtg gatgccacgg cccaggtgca tgaaagtgcc 360

tatgtggggc cgcaatgcgt ggtggaggca ggcgcggtca tcggtgccga tacggtgctg 420

aaatcgcgcg tcaccatcag ccagggctgt gtgctgggcg agcgctgcat tctgcatccc 480

ggtgtggtca tcggtgccga cggcttcggc ttcgcgccct cggcgggcca atgggaaaag 540

atagagcagt tgggggccgt gcgcatcggc aacgatgtgg aaatcggtgc caacacctgc 600

gtggatcgtg gggcacttga tgacaccgtc attgaagatg gcgtcaagat cgataatctg 660

gtgcagattg cccacaatgt gcacattggt gcgcatacgg tgattgccgg caataccggt 720

attgccggca gtgcgcgcat cggcaagcgc tgccagattg gtggagccgc caatattctg 780

gggcatttga cgattgcgga tggcacggtg atttcgccga cctccatggt cactcgctcc 840

ttgcccaagg caggttttta tacaggcatc tttccgttgc aagagaacga gcaatgggaa 900

aagaacgctg caaccttcag gcagttgtac acgctccggg agcgggtcaa gaagcttgaa 960

caagcgctgg ctgagggccg gcaaagcaac aacggaaatt ga 1002

<210> 11
<211> 1062
<212> DNA
<213> Pseudomonas aeruginosa

<400> 11
atgatgagta ccttgtccta caccctgggc cagttggccg cgcatgtcgg cgcggaagtc 60

cgtggcgacg cggacctgcc gatccagggc ctggcgacct tgcaggaggc cggaccggct 120

cagctgagct tcctggccaa cccgcagtat cgcaagtacc tgcccgaaag ccgtgccggc 180

gccgtcctgc tgacggctgc cgacgccgac gggttcgccg gtaccgcgct ggtggtggcc 240

aacccctacc tggcctatgc cagcctgtcg cacctgttcg atcgcaaacc gaaggcggcc 300

gccggtatcc atcctaccgc catcgtcgcg gcggacgccg aggtcgatcc gagcgccagc 360

gtcggcgcct atgcggtgat cgagagtggc gcccgcatcg gcgctggcgt cagcatcggc 420

gcgcactgcg tgatcggagc ccgtagcgtg atcggcgaag gcggctggct ggcgccgcga 480

gtcacgctct accacgacgt caccatcggc gcgcgggtga gtatccagtc gggcgcggtg 540

atcggcggcg aaggtttcgg tttcgccaac gagaagggcg tctggcagaa gatcgcccag 600

atcggcgggg tgaccatcgg tgacgacgtc gagatcggcg cgaacaccac catcgaccgt 660

ggagcactgt ccgatacgct gatcggcaac ggtgtgaagc tggacaacca gatcatgatc 720

gcgcacaacg tgcagatcgg cgatcatacc gccatggccg cttgcgtcgg catttccggc 780

agcgccaaga tcggcaggca ttgcatgctc gccggcggcg ttggcctggt cggccatatc 840

gagatctgcg acaacgtttt cgtgaccggc atgaccatgg tgacccgctc gatcaccgag 900

ccgggatcgt attcctccgg taccgccatg caaccggcgg ccgaatggaa aaaaagcgcc 960

gcgcgcattc gtcaactgga cgacatggcg cgtcgacttc agcagctgga aaaacgcctg 1020

gctgccgtga cctcaagcgg tgacgcttca tctgatgcgt aa 1062

<210> 12
<211> 1002
<212> DNA
<213> Artificial Sequence

<220>
<223> LpxD Comamonas testosterone (codon optimised)

<400> 12
atgagcgtgc tgctgggcca gatcctcgat gcgctgggcg gcgaactggt gggcggcgag 60

cgcgaaatgc ccatcagccg catcgcgccg ctggatagcg cgggccatgg cgatctgtcg 120

tttttaagca acccccgcta ccgccaacaa ctggccgcca gccaagccgc ttgtgtgatt 180

gtcgccccga ccatgcgcga cgaagccgcc cagcgcggcg cttgtatcgt gaccgccgat 240

ccgtacgcct atttcgcccg cgccacccag tggtggaaag cccaccagca aggcggccgc 300

ccgcgcggca tccacgccag cgccgtggtc gatgccaccg cccaagttca cgagagcgcg 360

tatgtcggcc cccagtgcgt cgtcgaggcc ggcgccgtga tcggcgcgga caccgtgctg 420

aagagccgcg tcaccatcag ccaaggttgc gttttaggcg agcggtgtat tttacatccg 480

ggcgtggtga tcggggccga tggcttcggc ttcgccccca gcgctggcca atgggaaaag 540

atcgagcagc tgggcgccgt gcgcatcggg aatgacgtgg agatcggcgc caacacgtgc 600

gtggaccgcg gcgcgctgga cgacacggtc atcgaggacg gcgtgaagat cgacaattta 660

gtgcagatcg cccacaacgt ccacatcggc gcgcacaccg tcatcgccgg caacaccggc 720

attgccggct cggcccgcat tggcaagcgc tgccagattg gcggggccgc caacatttta 780

ggccatttaa ccatcgccga tgggaccgtc atcagcccga cctcgatggt cacccgctcg 840

ctgcccaagg cgggcttcta caccggcatc ttcccgctcc aagaaaacga gcagtgggag 900

aagaatgcgg ccaccttccg ccagctgtac actttacgcg agcgcgtgaa gaaactggag 960

caagctctgg ccgaggggcg ccagagcaac aacggcaact ga 1002

<210> 13
<211> 1062
<212> DNA
<213> Artificial Sequence

<220>
<223> LpxD Pseudomonas aeruginosa (codon optimised)

<400> 13
atgatgagca ctttaagcta cactttaggc cagctggccg cccatgtggg cgccgaggtc 60

cgcggcgacg ccgatctgcc cattcaaggt ttagccacgc tgcaagaagc cggcccggcg 120

cagctgagct ttttagcgaa cccccagtac cgcaagtacc tcccggagtc gcgcgccggc 180

gccgtgctgc tgaccgccgc ggacgccgac ggcttcgccg gcaccgcttt agtcgtggcc 240

aatccctatt tagcgtacgc ctctttatcg cacctcttcg atcgcaaacc gaaggccgcc 300

gcgggcatcc acccgaccgc gatcgtggcc gccgatgccg aagtggaccc gagcgcgagc 360

gtgggggcct atgccgtcat cgagagcggc gcccgcatcg gcgccggcgt gagcatcggc 420

gcgcattgcg tgattggcgc gcgcagcgtc attggcgaag gcggctggct ggcgccgcgc 480

gtgactttat accatgacgt gaccatcggc gctcgcgtca gcattcagag cggcgccgtc 540

atcgggggcg agggctttgg cttcgccaac gagaagggcg tctggcagaa aatcgcccaa 600

atcggcggcg tcaccatcgg cgacgacgtg gagattggcg ccaacaccac catcgatcgc 660

ggcgcgctgt cggacaccct catcggcaat ggcgtcaagc tggacaacca gattatgatc 720

gcgcacaacg tgcagatcgg cgaccatacc gccatggccg cttgtgtggg cattagcggc 780

agcgccaaga tcggccgcca ctgcatgctg gccggcggcg tgggtttagt gggccatatc 840

gagatttgcg acaacgtgtt cgtcaccggc atgaccatgg tgacccggag catcacggag 900

ccgggctcgt acagcagcgg caccgcgatg caaccggccg ccgagtggaa gaagtcggcc 960

gcccgcatcc gccaactgga tgacatggcg cgccgtttac aacagctgga gaagcgcctc 1020

gccgccgtga ccagcagcgg cgatgccagc agcgacgcct ga 1062

<210> 14
<211> 93
<212> DNA
<213> Artificial Sequence

<220>

<223>  N-term insert for genomic integration into B. pertussis

<400>  14
accatctgca agtaccaggg ccgggccctg gtcgacggcc aactggtcgc cgaagccaag      60

ctgatgtgcg cgatccgtag cctggaagag taa                                   93


<210>  15
<211>  491
<212>  DNA
<213>  Artificial Sequence

<220>
<223>  C-term insert for genomic integration into B. pertussis

<400>  15
gcctgcgcat aggcatggtc gccggcgagc cttcgggcga cctgctggcc gggcgcatca      60

tcgccggcct gcaggcccgg gcacccggcg tgcactgcgc cggcataggc gggccccaga     120

tggcggcgcg cggcttcgag gcctggcacc ccatgcatgc gctcacggtg ttcggctaca     180

tcgatgcgtt caagcggatt cccagcctgc tgtcgaccta cggcgacgtc aagcggcgcc     240

tgctggccga gccgccgtcg gtgttcgtgg gcatcgacgc gccggatttc aacctgcgcc     300

tggagcacca gttgcgccag gccggtaccc ccaccgtgca tttcgtcggt ccctcgatct     360

gggcctggcg ctacgaacgc atcaacaaga tccgcgccgc cgtctcccac atgctggtgc     420

tgtttccgtt cgaggaagcg ctctatcgca aggaaggaat cccggtcacc tatgtcggcc     480

atccgctggc c                                                          491


<210>  16
<211>  537
<212>  DNA
<213>  Bordetella bronchiseptica

<400>  16
atgcaatttc tcaagaaaaa caagcccctg ttcggcatcg ttacactggc tctggcatgt      60

gccaccgccc aggcgcagcc cactcagggc ggggtcagcc tgcattacgg tattggcgac     120

cactatcagc gcgtcacgct gaactacgaa acccccacgc tctggagcca ccagttcggc     180

ggcaattggg gccgcctgga cctgaccccc gaactgggcg cgtcatactg gtgggccgac     240

ggctcgcgct cgcccggcca cgtgtggcag gccagcgcca ttccgatgtt ccgctggtgg     300

accggcgagc gcttttacat cgaggccggc atcggcgcca cggttttcag cagcaccagc     360

ttcgccgaca agcgcatcgg ttcggccttc cagtttggcg accatatcgg gctgggcttc     420

ctgctgacgc ccagcaaccg catcggcctg cgctattcgc acttctccaa cgccggcatc     480

aaggaaccga accccggcct cgatatcgtg cagctgacct atacgtacca gttctga        537


<210>  17
<211>  296

<212>  DNA
<213>  Artificial Sequence

<220>
<223>  Porin promoter (BP0840 promoter; BX640413.1:174494-174789)

<400>  17
tccttgagtg aactgggggg tcggcgattt ccagtttctc aaatccggtt cggatgaacc      60

atgcatacaa cctattgaat cttcacagtt agcccgcgcg cgattccgga ttaaggggac     120

aggatgttgc aacttaccaa caatggggcg ggaaaccggc ttttttctga gcctgaccat     180

agccagtcct gccgattttt gatgcaatag cgtcaacttc ccttcgcgga gttagggggg     240

gcggcaggcc ggtatgtgct gcattgctgc tcttgtcact caaatcaacg gagatt         296


<210>  18
<211>  1620
<212>  DNA
<213>  Bordetella pertussis

<400>  18
atgactctcg ctacccgatc catgcaaccc catgcggttt cccctggcca ggccaggtcc      60

tggcccctgc ccgcggcggg ctggctgctt ctcgcggtcg gcgtctggct ggccttcctg     120

tcctggatgc ggcccctggc gctgcccgac gaagggcgct acgccggcgt ggcctgggac     180

atgctgcgca acggctcgtt cgcggtgccg ctcatcgacg gcatgccgta tttccacaag     240

ccgccgctgt actactggct ggccgagctg tcgttccgcc tgttcggcgt caacgaatgg     300

gcggcgcgcc tgccctcggc gctggccgcc tgggccagcg ccgtcgcgct gtacctgttc     360

gtgcgccgcc atcgcgacgc cgcgtccgcg acgctctgcg tgctggtgct ggccacgctg     420

ccgctgtttt tcggcggcgc ccagtacgcc aacatggata tgctggtggc cggcatgatc     480

acgctgtgcg tgctggccgg cgccgacacg gcgctgcgtg tccgcggcgg gcaggcctgg     540

cgcgccatgg cgctggccac cggcgtgtgc gccgcgctgg ccatgctggc caagggcctg     600

atcgggctgg tgctgccggg cgcgatcctg ctggcctggc tggcgtggcg gcgcgactgg     660

cgcggcctgc gggccttgct gtggccgccc gccatcctgg cgttcgcggt ggtggccgtg     720

ccctggttct ggctgatgca ggtacgctac ccgggctttt tccagtactt cttcgtccac     780

cagcatttcg agcgtttcgc gcagaccggc ttcaacaatg tgcagccgtt ctggttctac     840

ctgcccgtca tcgcgggcct ggcattgccg tggtcgttgt gggccggcgg cttgctgcgc     900

aagcaattct gggccgccga cgccgacccg gacggcttgc gccgcctggc gctggtctgg     960

ctggcggtca tcgtggcgtt tttctccatg ccgcagtcca agctggtcgg ctacatcatg    1020

ccggtgctgc cgccgctggc cttcctgctg gccgaagtgg tgatgggcgc cttgcgcgac    1080

ccggcggtgg cgcgggccac gcgccgcatg gcgcgcgtca gcgccctggt ggcggtggcc    1140

atctgcgtga ccgcggtgtt cgtggcgtcg ttcaacgccc gcggcagttc gcgcgagctg    1200

gccctgtcgc tgcgcggcga gctgcggccg gacgacacgc tggtggccct gcatacctat 1260

ccgttcgacc tgcagctgta tgcccatgcg gcccggccga tgtgggtggt cgacgactgg 1320

agcaaccccg aaattcccaa gcgcgacaat tggcgcaggg agctctacga cgcggtccag 1380

ttcgagccgg ccctgggcga gcggctgctg gtctcggcag atactttcca gcagcgcctg 1440

tgccaggcgc cggagggcag ccgttactgg gtctggggca cggcggccga cgaagaggcg 1500

tacgcgccgc tgcgcggcca ggcggcgcgc tttgccgacg cgcggcgcag cctctggctg 1560

gtgctggtgg acgaagcctt caaggggcgt gtctgtgacg gaacgcccac aggcggctag 1620

<210> 19
<211> 4395
<212> DNA
<213> Bordetella pertussis

<400> 19
gtggataaag atgaatcggc attgcggcaa cttgtcgaca tggcgcttgt aggctacgac 60

ggcgtggtgg aagagttgct ggcgctgccc tcggaagagt ccggggatct tgcgggtgga 120

cgggccaaac gagagaaggc cgaattcgcg ctgtttagcg aagcgcccaa cggggatgaa 180

cccatcggcc aggatgctcg tacatggttc tattttccca agtaccgccc ggtggcggtt 240

tccaatttga agaaaatgca ggtggcgatt cgtgctcgtc tcgagcctga gtcattgatc 300

ctgcagtggt tgatcgcact cgatgtctat ctcggcgtgt tgatcgccgc cttgtcccgc 360

actgtgatca gcgacctggt attcgagtat gtcaaggcgc gctacgagat ctactaccta 420

ctgaatcgcg tgccgcatcc gctggcgacg gcgtacctca agcgccgccg ccagcgtccg 480

gtggatcgtt cgggacgtct gggctcggtg ttcgaacacc cgctatggtt cgcctacgac 540

gaattggccg gcaccgtcga tctggatgcc gacatctacg agcaggcgct cgccgaaagc 600

atcgaacgcc gcatggacgg agagccggac gacggcagtc tggataccgc cgaacacgac 660

gtatggcgtt tgtgccgcga cggcatcaat cggggcgagc aggcgatttt ccaggcatcc 720

ggcccatatg gcgtggtcgc ggatgcaggg tatatgcgca ccgttgccga tctggcgtat 780

gccgatgcat tggcggattg cctgcatgcc caactgcgca tccgggcgca aggctcggtc 840

gatagcccgg gagacgagat gccacgcaaa cttgatgcgt gggaaatcgc caagtttcat 900

ctggccgcga cccagcaggc gcgggttgat ctgctcgagg cggcgttcgc gctcgactac 960

gccgccttgc gcgatgtgcg cgtctacggc gattaccgca atgcgctggc tcttcggttc 1020

atcaagcgcg aggccttgcg gttgctgggg gcgcggcgcg gcaacgcctc tacgatgccg 1080

gcggttgcgg ctggcgagta cgacgaaatc gtggccagtg gagcagccaa tgacgcggct 1140

tatgtatcca tggccgccgc gttgatcgcg ggcgtgctct gcgatctcga gagcgcgcag 1200

cgcacgttgc ccgtcgtatt ggccaggttt cggccccttg gcgtgcttgc gcgattcaga 1260

aggctggagc aggaaaccgc gggcatgctg cttggcgacc aggagccgga gcctcggggc 1320

ttcatcagtt ttaccgattt tcgcgatagc gacgcgttcg ccagctacgc ggagtatgcg 1380

gcccagttca acgactatat cgatcaatac agcatactcg aggcgcagcg gctggcgcgg 1440

attctggccc tgggctcgcg gatgacggtc gatcaatggt gccttcccct gcagaaagta 1500

cggcactaca aggtgctgac atcgcagcca gggctgatcg cgcgtggaat cgaaaatcac 1560

aacaggggca ttgaatattg cctggggcgg ccgccgctga ccgatctgcc gggtcttttc 1620

accatgttcc agctccatga ttccagctgg ctgttggtat cgaacatcaa cggtgagctt 1680

tggtctgatg tccttgcgaa cgctgaggtg atgcagaatc ctacgctggc cgcccttgcc 1740

gagccgcaag gccgctttcg aaccggccgt cgaacgggcg ggtggtttct cggcggcccc 1800

gcgactgaag ggccaagtct gcgcgacaac tacctcctca aattgaggca gagcaatccg 1860

gggctggatg tcaagaaatg ctggtatttc gggtatcggc aagagtacag gctgcccgca 1920

ggcgcgctgg gtgtgccgct attcgctgtt tccgttgccc tgaggcacag tctggacgac 1980

ctcgcggcgc acgcgaagtc cgctttgtac aaacccagcg aatggcagaa gttcgccttc 2040

tggatcgtgc cgttctaccg ggaaatattc ttttcgaccc aggatcgttc ctatcgggtc 2100

gatgtgggga gcatcgtctt tgattcgatt tccttgcttg cctcggtgtt cagcatagga 2160

gggaagttgg gcagtttcac ccgtacccag tatggcaacc tgcgcaattt cgtggtgcgg 2220

caacgcatcg cggggttgag cgggcagcgc ttgtggcgtt cggtgctcaa ggagcttccg 2280

gcattgatcg gagccagcgg gttgcgcctg tcgcgttcgc tgctcgtcga tctgtatgag 2340

atcttcgagc ccgtgcctat ccgtcggctt gtcgcgggat tcgtgagcgc cactacggtc 2400

ggcgggcgta accaggcctt cctgcggcag gcattctccg ctgccagttc ctcggccggg 2460

cgcacggggg gccaattggc aagcgaatgg cggatggccg gcgtggacgc caccggtttg 2520

gtcgagtcca cgtcgggcgg caggtttgag ggcatctaca cgcgcggctt gggaccgttg 2580

agcgagtgca ccgagcactt catcgtcgaa tccgggaatg cctacagagt catctgggat 2640

gcatacacgc atggttggcg cgtggtcaat gggcgtttgc cgccgaggct gacctatacc 2700

gttccggttc ggctgaacgg gcaaggccac tgggagacgc atctggatgt ccctggccgg 2760

ggtggggcgc cggaaatctt cggacgcatc cgcacgcgta atctggtcgc ccttgccgcc 2820

gagcaggccg cgccgatgcg ccgcctgctc aaccaggcga ggcgtgtggc gctcaggcat 2880

atcgatacct gcaggagcag gcttgcgttg ccgcgcgctg aatccgatat ggacgcggcg 2940

atccggattt tcttcggaga gccggacgcc ggccttcgcc agcgcatcgg gcgacgcctg 3000

caggaggtca gggcctatat cggcgatctg agtccggtca atgacgtgct gtaccgggcg 3060

ggatatgacc tcgacgatgt cgcaacgctg tttaacgcag tggaccggaa cacgtcgctg 3120

ggcaggcagg ctcggatgga gttgtatctg gatgccattg tcgatttgca tgccaggctc 3180

ggctatgaaa atgcgcgttt tgtcgacctg atggcgttcc acctgctcag cctgggccat 3240

gccgcgacgg ccagtgaggt cgtggaggcc gtttcgcccc ggctgctggg caatgtgttc 3300

gatatctcga acgtcgccca gctcgaacgc ggtatcggga atcccgccag caccggtctt 3360

ttcgtcatgc tgggcgccta ttcggaatcg tcgcccgcga tattccagtc gttcgtgaac 3420

gatatatttc ccgcatggcg acaggcttcc ggcggggggc cgctggtatg gaacttcggt 3480

cctgccgcca tcagcccgac gcgcctggat tacgcaaata ccgatatcgg attgctcaac 3540

catggggata tttccccgct gcgcgccagg ccgccattgg gcggcaggcg cgacatcgat 3600

cttcctccgg ggctggatat ttcgttcgtg cgttacgacc gcccggtgcg catgtccgcg 3660

ccgcgtgcgc tcgacgccag cgtcttcagg ccggtcgacg ggcctgtcca tggctatatt 3720

caatcgtgga cgggggctga gatcgaatat gcgtacggcg cgcccgcagc ggcacgcgag 3780

gtcatgctga ccgacaatgt gcggatcatc agcatcgaga acggcgatga aggggcgatc 3840

ggggtgcgcg tgaggctcga cactgttccc gtcgcgacgc cgctcatcct gactggcggc 3900

tccttgagcg ggtgcacgac gatggttggg gtcaaggagg gctacctggc cttctaccac 3960

actggcaagt cgaccgaact cggggattgg gcgactgcgc gcgaaggcgt acaggcgctt 4020

taccaggccc atttggcgat ggggtacgcg ccgatctcaa tcccggcgcc gatgcgcaat 4080

gacgacttgg tcagcatcgc cgcgacctac gaccgggcgg tcattgccta tctgggcaag 4140

gatgtgccgg gaggcggcag tacgcgcata acgcgtcacg atgagggggc gggcagtgtg 4200

gtttcgttcg actacaacgc agcagtccag gcgtcggccg ttccccgcct cggccaggta 4260

tacgttctga tttccaacga cggacaaggg gcgcgagccg tcctgctggc ggaggatctg 4320

gcctgggcag gcagcggcag tgccttggat gtgttgaacg aacgattggt gacgttgttt 4380

ccggcgccgg tctga 4395

<210> 20
<211> 1427
<212> DNA
<213> Artificial Sequence

<220>
<223> Construct for ArnT KO

<400> 20
gtgtgccagc tgggaaatca tcgtcgtcga cgacggcagc accgacgata ccgccgagct 60

gatggcgcaa tggtccgcgg tcgagggcat ccgctacgtg cagctgtcgc gcaatttcgg 120

caaggaggcc gccctgacgg ccggcctgga agccgcggac ggcgacgccg tcatctgtct 180

ggacgccgac atgcagcatc cgcccgaact gataggcgac atgctggcgg cctggcgcaa 240

cggcgccgac atggtctacg cggtgcggcg ccagcgcgac gacgagccgt ggttcaagcg 300

tgtcggcgcc cgcgcgttct accgcctgct gagcacggcg cgcggcgtcg aggtgccgcc 360

gcacgcgggc gacttccgcc tgatggaccg ccgcgtggtc gaggccctgg tggcgctgcc 420

cgaacgcacc cgcttcatga aggggctgta tgcctgggtg ggcttcaagt cgcaggccgt 480

gccctacacc ccgcaagcgc gccgccatgg cgccagccac ttcagcgcct ggaagctgtt 540

tcgcctggcc tgcgacggcc tgaccgcctt caccacctgg ccgctgcggc tggtcagcct 600

catcggcgtg ctgttcgcgc tgctgtcgct gtcctacggc ggttacctgg tggccgacta 660

tctcatcagc ggcaacgcgg tgtccggctg gaccaccatc gtcaccgcgc tgctgttctt 720

tgccggcatc aacctgattt cgctgggcgt ggtgggcgag tacgtggccc ggatcttcga 780

cgaagtgaaa ggccggcccc tgttcatcgc ccgtcaacgc cgcgggcgcg ccaagcgcgc 840

ggccaaggcg cgttcccaat gactctcgct acccgatcca tgcaacccca tgcggtttcc 900

cctggccagg ccaggtcctg gcccctgccc gcggcgggct ggctgcttct cgcggtcgga 960

tagctcgagt gacggaacgc ccacaggcgg ctagcgacga atgtcaggaa ggccagcccg 1020

atcaacacca gggccagcag cacgtcatag ggcagcgacg tggcgtgcag cagccaggca 1080

tatgacaact cattgatggc gaaacccagg gctgacacaa ggaagaaacg ccgggcggct 1140

atggtccagg atgccgcaag gtggcggaaa gtgagtctga aatgaccagt gaacgatacg 1200

acgaaggcca acagccagcc caccacgttg gcccccagcg ggggcaggcc ggcccattcc 1260

acgcaggcga ccgcgacggc ccaatgggtg gcggccgcgg cgcatccgac ggcaatgaac 1320

caggcaatct gtcgcagcag gccgcgcatg tcgaagaggt gtctcccttg aggaaccagg 1380

ccggcgatgt ccgttgccgg cgcatcgcgg tgtgcggcga tgatttt 1427

<210> 21
<211> 2705
<212> DNA
<213> Artificial Sequence

<220>
<223> Construct for replacing DNT with LpxD P. aeruginosa

<400> 21
cgccgccagc atttccgagg cgctcggcta cgagcccggc gtcaacggta tccacgcgcc 60

agcggctgcc tgagtccggc ctggcctggc gataaggcgg tgcgtgcagg cgtggctgcg 120

atcgcaccgt aaccgttggt gcggcgtcct gatctccgga gcgttcggct gggagatggc 180

tgcgcatccg atgggccggt tcttgtgtgt ttgctagcct gactaacagg cgacctgggg 240

tcgtttccgg tttccggtca ggggcaaaca aacgtggata aagatgaatc ggcattgcgg 300

caacttgtcg acatggcgct tgtaggctac gacggcgtgg tggaagagtt gctggcgctg 360

ccctcggaag agtccgggga tcttgcgggt ggacggccca aacgagagaa ggccgagttc 420

gcgctgttta gcgaagcgcc caacggggat gaacccatcg gccaggactg agagctctcc 480

ttgagtgaac tggggggtcg gcgatttcca gtttctcaaa tccggttcgg atgaaccatg 540

catacaacct attgaatctt cacagttagc ccgcgcgcga ttccggatta aggggacagg 600

atgttgcaac ttaccaacaa tggggcggga aaccggcttt tttctgagcc tgaccatagc 660

cagtcctgcc gatttttgat gcaatagcgt caacttccct tcgcggagtt agggggggcg 720

gcaggccggt atgtgctgca ttgctgctct tgtcactcaa atcaacggag attggtacca 780

tgatgagcac tttaagctac actttaggcc agctggccgc ccatgtgggc gccgaggtcc 840

gcggcgacgc cgatctgccc attcaaggtt tagccacgct gcaagaagcc ggcccggcgc 900

agctgagctt tttagcgaac ccccagtacc gcaagtacct cccggagtcg cgcgccggcg 960

ccgtgctgct gaccgccgcg gacgccgacg gcttcgccgg caccgcttta gtcgtggcca 1020

atccctattt agcgtacgcc tctttatcgc acctcttcga tcgcaaaccg aaggccgccg 1080

cgggcatcca cccgaccgcg atcgtggccg ccgatgccga agtggacccg agcgcgagcg 1140

tgggggccta tgccgtcatc gagagcggcg cccgcatcgg cgccggcgtg agcatcggcg 1200

cgcattgcgt gattggcgcg cgcagcgtca ttggcgaagg cggctggctg gcgccgcgcg 1260

tgactttata ccatgacgtg accatcggcg ctcgcgtcag cattcagagc ggcgccgtca 1320

tcgggggcga gggctttggc ttcgccaacg agaagggcgt ctggcagaaa atcgcccaaa 1380

tcggcggcgt caccatcggc gacgacgtgg agattggcgc caacaccacc atcgatcgcg 1440

gcgcgctgtc ggacaccctc atcggcaatg gcgtcaagct ggacaaccag attatgatcg 1500

cgcacaacgt gcagatcggc gaccataccg ccatggccgc ttgtgtgggc attagcggca 1560

gcgccaagat cggccgccac tgcatgctgg ccggcggcgt gggtttagtg ggccatatcg 1620

agatttgcga caacgtgttc gtcaccggca tgaccatggt gacccggagc atcacggagc 1680

cgggctcgta cagcagcggc accgcgatgc aaccggccgc cgagtggaag aagtcggccg 1740

cccgcatccg ccaactggat gacatggcgc gccgtttaca acagctggag aagcgcctcg 1800

ccgccgtgac cagcagcggc gatgccagca gcgacgcctg aaagcttttg aacgaacgat 1860

tggtgacgtt gtttccggcg ccggtctgaa tcgcattccg cgaaacggcg ggcgcgcagg 1920

cctcgcccga tcggccggcc gggccgcgcg atgcgcggcc cggccgcatt gggacgcggc 1980

ctttattgct tttccacgcc cgccttcttg aacagctcgg cccattgcgg cacctgctgc 2040

ttgaccttgg cttgcagcgc ctcgggatta gcctcgttgg tcagcacctc ggcgcccagc 2100

tgcttcatgc gctcctggaa cttgggatcg gccaggccgg cctgcaggct cttgaccagc 2160

ttgtcgacca ccggcttggg cgtgcccttg ggggcccaca tgccgtgcca gatacccact 2220

tcgaaaccct tgtagccgga ctcgtccatg gtgggcaggt cgggcagggt gggcacgcgc 2280

ttgaggctgg tgaccgcgta ggccttcacc ttgccgctgg tgatctgctg cgtggtattg 2340

gtggtctggt cgcacatcag gtcgacctgc ttgcccagca gatcgttcat ggccggcgcc 2400

gtgcccttgt agggaatggt cagcaggttg acgcccagcg cctcgaccag catggtgccg 2460

cacaagtggc tggccgcgcc gatgccggca ttggccagtg aaatcttgtc ggcgttcttc 2520

ttgacgtact cggccagttc cttgatgttg ttgggcggga aatcgccgcg ggcaatgatg 2580

gtcatcggca cgtccaccac caggccgatg ggctcgaagc tggtgtaggg ctcgtagccg 2640

gggttcttgt acagcgaggg cgcggtcgag aagccggcgt gcatgagcag gatcgagtag 2700

ccgtc 2705

## Description

### TECHNICAL FIELD

The present invention relates to the field of vaccines, in particular to the prevention or treatment of infections caused by bacteria of the *Bordetella* genus, particularly *Bordetella pertussis.*

### BACKGROUND TO THE INVENTION

Pertussis, caused by *Bordetella pertussis,* is a highly contagious airway infection. Acute infection can cause severe illness characterized by respiratory failure, pulmonary hypertension, leucocytosis, and death. Pertussis is a vaccine-preventable disease, with either acellular pertussis (aP) or whole-cell pertussis (wP) vaccines being used worldwide. However, the disease has persisted in vaccinated populations, and epidemiological data has reported a worldwide increase in pertussis incidence in recent years.

Several hypotheses have been postulated, including the evolution of pertussis strains and the decreased duration of protection from aP vaccines compared to wP vaccines. One of the fields of research aimed at controlling the re-emergence of pertussis is directed towards the development of new vaccines.

Vesicles derived from pathogens have been used in the development of immunogenic compositions such as vaccines [1]. The use of outer membrane vesicles (OMVs) or their derivatives could potentially deliver a broad spectrum of antigens in their native form.

OMVs comprise the bacterial lipopolysaccharide (LPS), which is composed of a highly variable O antigen, a less variable core oligosaccharide and a highly conserved lipid moiety designated lipid A. Bacterial lipooligosaccharides (LOS) share similar lipid A structures with an identical array of functional activities as LPSs, but they lack O-antigen units.

However, the presence of LPS in OMVs may present issues from a clinical and regulatory point of view. For example, when LPS is degraded in the human body, the production of excessive proinflammatory cytokines can be elicited. Through their interaction with human TLR4 they can potentially cause a number of adverse effects (reactogenicity) such as fever, chills, shock, and a variety of other symptoms depending on the particular organism and the condition of the patient.

The endotoxic activity of the LPS is mainly determined by the composition of its lipid A moiety, which consists of a glucosamine disaccharide substituted with one or two phosphate groups and a variable number of acyl chains [2].

WO2018/167061 [3] describes the use of LpxA variants derived from either *Pseudomonas aeruginosa* (LpxAₚₐ) or *Neisseria meningitidis* (LpxA_{Nm}) to reduce the length of the C3' acyl chain in *Bordetella pertussis.* The exogenous LpxA genes were expressed from a plasmid, whilst the genomic, endogenous LpxA gene was knocked out. Episomal expression of LpxApₐ had the effect of reducing the length of some acyl chains and the authors noted that TLR4 stimulation was reduced. However, the strains exhibited strong growth defects. In addition, expression of LpxA_{Nm} was lethal. Similar strong growth defects were observed when an LpxD variant derived from *Pseudomonas aeruginosa* was episomally expressed in *B. pertussis.* Whilst the results confirm that reactogenicity can be modified by engineering of lipid A, the growth defect of the strains is a major impediment to the use of such strains in vaccine manufacturing.

Thus, there remains a need for improvements suitable for the manufacture of immunogenic compositions such as vaccines.

### SUMMARY OF THE INVENTION

The Applicant has developed novel recombinant *Bordetella pertussis* bacterial strains that overcome the issues observed in the art and are particularly suitable for the preparation of whole-cell antigens and/or outer membrane vesicle components. These components may be used in immunogenic compositions such as vaccines.

In a first aspect, the invention relates to a recombinant *Bordetella pertussis* bacterium which comprises: at least one genomic LpxA gene encoding an LpxA protein, wherein the LpxA protein comprises a mutation at position 170 and/or a mutation at position 229 relative to SEQ ID NO: 1; and/or at least one genomic insertion of a heterologous LpxD gene.

The amino acid sequence of the wild-type LpxA protein from *Bordetella pertussis* (LpxA_{Bpe}) is provided as SEQ ID NO: 1. The amino acid sequence of the LpxA protein from *Bordetella parapertussis* (LpxA_{Bpa}) is provided as SEQ ID NO: 2.

The term "mutation" as used herein, refers to deletion, addition, or substitution of amino acid residues in the amino acid sequence of a protein or polypeptide as compared to the amino acid sequence of a reference protein or polypeptide, particularly a wild-type protein or polypeptide.

The term "substitution" when referring to an amino acid sequence, refers to a change in an amino acid for a different amino acid or amino-acid moiety. For example, when referring to LpxA, a substitution refers to a change in an amino acid relative to SEQ ID NO: 1. Substitution of an amino acid at one particular location in the protein sequence is referred to using the following annotation "(amino acid residue in wild type protein)(amino acid position)(amino acid residue in mutated protein)". For example, G229A refers to a substitution of a Glycine (G) residue at the 229th position of the amino acid sequence of the reference protein (here SEQ ID NO:1) by an Alanine (A) residue (in the mutant).

Particularly, the mutation at position 170 is a substitution relative to SEQ ID NO:1. Yet more particularly, the mutation at position 170 is a substitution of Glycine. Still yet more particularly, the mutation at position 170 is a substitution of Glycine with Serine (G170S).

Particularly, the mutation at position 229 is a substitution relative to SEQ ID NO:1. Yet more particularly, the mutation at position 229 is a substitution of Glycine. Still yet more particularly, the mutation at position 229 is a substitution of Glycine with Alanine (G229A).

Particularly the LpxA protein comprises a mutation at position 170 and a mutation at position 229 relative to SEQ ID NO:1. Yet more particularly, the mutation at position 170 is a substitution of Glycine and the mutation at position 229 is a substitution of Glycine. Still yet more particularly, the mutation at position 170 is a substitution of Glycine with Serine (G170S) and the mutation at position 229 is a substitution of Glycine with Alanine (G229A). Still yet even more particularly, the LpxA protein comprises a Serine residue at position 170, an Alanine residue at position 229. Particularly, and in addition to the mutations at positions 170 and/or 229, the LpxA protein has at least 85%, at least 86%, at least 87%, at least 88%, at least 89%, at least 90%, at least 91%, at least 92%, at least 93%, at least 94%, at least 95%, at least 96%, at least 97%, at least 98% or at least 99% amino acid sequence identity to either SEQ ID NO: 1 or SEQ ID NO: 2. In some embodiments, the LpxA protein has 90% sequence identity to SEQ ID NO: 2. In some embodiments, the LpxA protein has the amino acid sequence of SEQ ID NO: 2 (i.e. it has 100% sequence identity).

The amino acid sequence of an LpxD protein from *C. testosteroni* (LpxD_{Ct}) is provided as SEQ ID NO: 3. The amino acid sequence of an LpxD protein from *P. aeruginosa* (LpxD_{Pa}) is provided as SEQ ID NO: 4.

Particularly, the heterologous LpxD gene encodes an LpxD protein having at least 85%, at least 86%, at least 87%, at least 88%, at least 89%, at least 90%, at least 91%, at least 92%, at least 93%, at least 94%, at least 95%, at least 96%, at least 97%, at least 98% or at least 99% amino acid sequence identity with either SEQ ID NO:2 or SEQ ID NO: 3. More particularly, the heterologous LpxD gene encodes an LpxD protein having at least 95% amino acid sequence identity with either SEQ ID NO:2 or SEQ ID NO: 3. Yet more particularly, the heterologous LpxD gene encodes an LpxD protein having 100% sequence identity with an amino acid sequence selected from the group consisting of SEQ ID NO:2 or SEQ ID NO: 3. In some embodiments, the heterologous LpxD gene encodes an LpxD protein having the amino acid sequence of SEQ ID NO:2. In some embodiments, the heterologous LpxD gene encodes an LpxD protein having the amino acid sequence of SEQ ID NO: 3.

The term "heterologous LpxD gene" refers to an LpxD gene or nucleotide sequence from a different species than the species of the host organism it is introduced into, in the context of the present invention, the heterologous LpxD gene or nucleotide sequence is not normally found in wild-type strains of *Bordetella pertussis.* In certain embodiments, the nucleotide sequence of the heterologous LpxD gene may be codon optimised for expression in *Bordetella pertussis.*

The term "genomic" refers to the bacterial cell genome, i.e. chromosomal DNA, whilst "genomic insertion" is used to refer to stable integration into the bacterial cell genome thereby excluding transient or episomal expression, such as expression of genes in a plasmid.

In some embodiments of the invention, expression or activity of the endogenous LpxA gene in the recombinant *Bordetella pertussis* bacterium is modified, reduced, suppressed or inactivated relative to a wild-type *Bordetella pertussis* bacterium. In some embodiments of the invention, expression or activity of the endogenous LpxD gene in the recombinant *Bordetella pertussis* bacterium may be modified, reduced, suppressed or inactivated relative to expression or activity seen in a wild-type *Bordetella pertussis* bacterium. In some embodiments of the invention, expression or activity of both the endogenous LpxA gene and endogenous LpxD gene in the recombinant *Bordetella pertussis* bacterium is modified, reduced, suppressed or inactivated relative to a wild-type *Bordetella pertussis* bacterium.

In some embodiments, the expression or activity of the endogenous LpxA gene is reduced, supressed or inactivated by knocking out the endogenous LpxA gene. In some embodiments, the expression or activity of the endogenous LpxA gene is modified, reduced, suppressed or inactivated by replacing the endogenous LpxA gene via, for example, a knock-in. In some embodiments, the expression or activity of the endogenous LpxA gene is modified by mutating the endogenous LpxA gene, particularly by mutation at positions 170 and/or 229. In some embodiments, the expression or activity of the endogenous LpxD gene is reduced, supressed or inactivated by knocking out the endogenous LpxD gene. In some embodiments, the expression or activity of the endogenous LpxD gene is modified, reduced, suppressed or inactivated by replacing the endogenous LpxD gene via, for example, a knock-in. By way of non-limiting example, a knock-out may be achieved by removing, for example, deleting, part or all of the endogenous gene or by inactivating the endogenous promoter which activates expression of the respective endogenous gene.

Surprisingly, recombinant *Bordetella pertussis* bacterial strains of the invention do not exhibit growth abnormalities observed in the art.

Particularly, the growth profile of a population of recombinant *Bordetella pertussis* bacteria of the invention is comparable to that of a population of wild type, parental strain of *Bordetella pertussis* bacteria. More particularly, the growth curve of a population of the recombinant *Bordetella pertussis* bacteria is comparable to that of a population of wild type, parental strain of *Bordetella pertussis* bacteria. For example, the values of the elapsed time and growth rate in standard fermentation processes of the population of recombinant *Bordetella pertussis* bacteria and a population of the wild type gram-negative bacteria are comparable. More particularly, the values of elapsed time and growth rate for the population of recombinant *Bordetella pertussis* bacteria and the population of wild type *Bordetella pertussis* bacteria vary by less than 20%, for example, less than 15%, less than 10% or less than 5%. Yet more particularly, the values of elapsed time and growth rate for the population of recombinant *Bordetella pertussis* bacteria and a population of wild-type *Bordetella pertussis* bacteria are substantially similar.

The recombinant *Bordetella pertussis* bacterial strains of the present invention express Lipid A that exhibits reduced endotoxic activity when compared to Lipid A expressed by the wild-type *Bordetella pertussis* bacterium, particularly when measured using TLR4 stimulation assays, particularly human TLR4 stimulation assays, for example, through NF-kB induction of a recombinant HEK-TLR4 reporter cell line. One skilled in the art will be aware of other suitable assays.

Particularly, recombinant *Bordetella pertussis* bacterial strains according to the present invention are derived from the Tohama I parental strain, and more particularly a Tohama I PTg strain, a strain of Tohama I that expresses genetically detoxified pertussis toxoid. As used herein, the term "parental strain" takes its' general meaning in the art to refer to the strain of *Bordetella pertussis* used as the starting microorganism to construct the recombinant strain. The parental strain is generally the wild-type or comparator strain against which characteristics of the recombinant strains of the invention are compared. Recombinant *Bordetella pertussis* bacteria of the invention will generally be isogenic with its parent strain, except for the genetic modification or modifications of the invention described herein.

In a Second Aspect of the invention, there is provided an outer membrane vesicle (OMV), particularly a population of outer membrane vesicles, derived from the recombinant *Bordetella pertussis* bacterium of the First Aspect. There is also provided a whole-cell antigen derived from the recombinant *Bordetella pertussis* bacterium of the First Aspect.

In a Third Aspect of the invention, there is provided an immunogenic composition comprising at least one OMV or a population of OMVs according to the Second Aspect and a pharmaceutically acceptable excipient. Particularly, immunogenic compositions may also comprise an adjuvant. In some embodiments, immunogenic compositions of the invention may comprise at least one antigen in addition to those present in the OMV or OMVs. In preferred embodiments of the invention, the at least one additional antigen is selected from the group consisting of (1) pertussis toxoid (PT), (2) FHA, (3) pertactin (PRN), (4) FIM2/FIM3, (5) adenylate cyclase, (6) diphtheria toxoid (DT), (7) tetanus toxoid (TT), (8) inactivated polio virus (IPV), (9) hepatitis B surface antigen and (10) Hib PRP.

In a Fourth Aspect of the invention, there is provided the recombinant *Bordetella pertussis* bacterium according to the First Aspect, at least one OMV or population of OMVs of the Second Aspect or the immunogenic composition of the Third Aspect for use in inducing an immune response in a suitable mammal.

Particularly, there is provided the recombinant Bordetella pertussis bacterium according to the First Aspect, at least one OMV or population of OMVs of the Second Aspect or the immunogenic composition of the Third Aspect for use in therapy and/or prophylaxis, for example, for use as a vaccine.

Particularly, there is provided a method for inducing an immune response in a suitable mammal comprising administering to the suitable mammal the recombinant Bordetella pertussis bacterium according to the First Aspect, at least one OMV or population of OMVs of the Second Aspect or the immunogenic composition of the Third Aspect.

Particularly, there is provided the recombinant Bordetella pertussis bacterium according to the First Aspect, at least one OMV or population of OMVs of the Second Aspect or the immunogenic composition of the Third Aspect for use in the manufacture of a medicament, for example, for therapy and/or prophylaxis, for example, for use as a vaccine.

In a Fifth Aspect of the invention, there is provided a method of modulating the reactogenicity of the Lipid A of a Bordetella pertussis bacterium, comprising stably integrating into the genome of the bacterium at least one gene selected from the group consisting of LpxA and LpxD, wherein:
(i) the LpxA gene encodes an LpxA protein having at least 80% sequence identity with SEQ ID NO: 1 or 2 and wherein the protein comprises Serine at position 170 (S170) and/or Alanine at position 229 (A229) when numbered in accordance with SEQ ID NO: 1 or 2; and/or
(ii) the LpxD gene encodes an LpxD protein having at least 90% amino acid sequence identity with SEQ ID NO:3 or SEQ ID NO: 4..

### BRIEF DESCRIPTION OF THE DRAWINGS

**Figure 1****.** Structure of lipid A from *Bordetella pertussis* (A) and *Bordetella parapertussis* (B).
**Figure 2****.** Alignment of the LpxA amino acid sequences from the *Bordetella* genus.
**Figure 3****.** Fermentation profile of *B. pertussis* Tomaha I PTg. Curve 1: pH regulated at 7.2 by addition of acetic acid (Curve 3) to avoid the basification of cell broth resulting in stopping the culture; Curve 2: dissolved oxygen concentration; Curve 4: stirring speed manipulated to keep dissolved oxygen concentration (DO) at 25% to avoid oxygen limitation (DO/speed regulation); Curve 5: temperature regulated at 35°C.

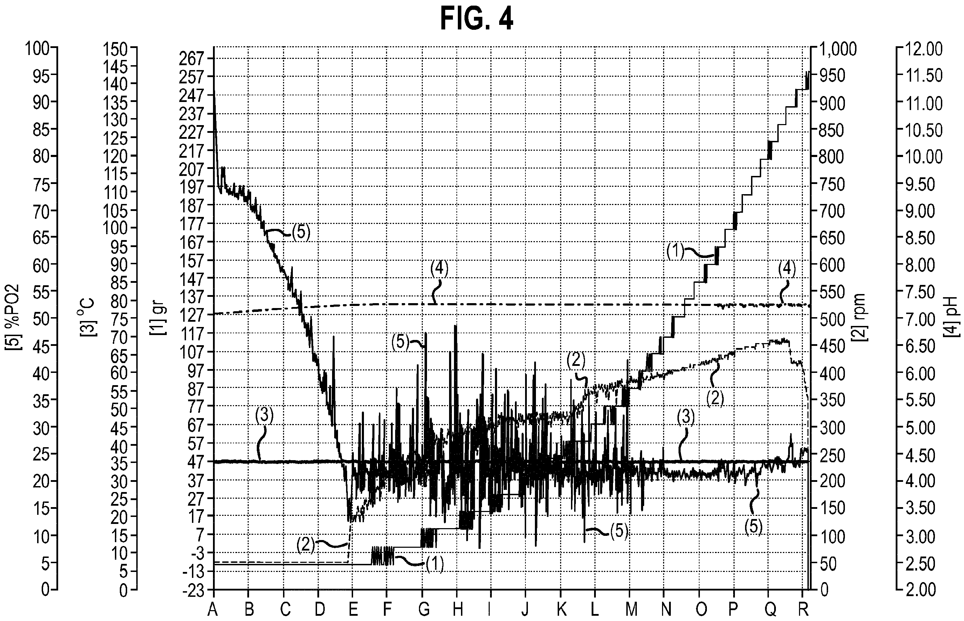

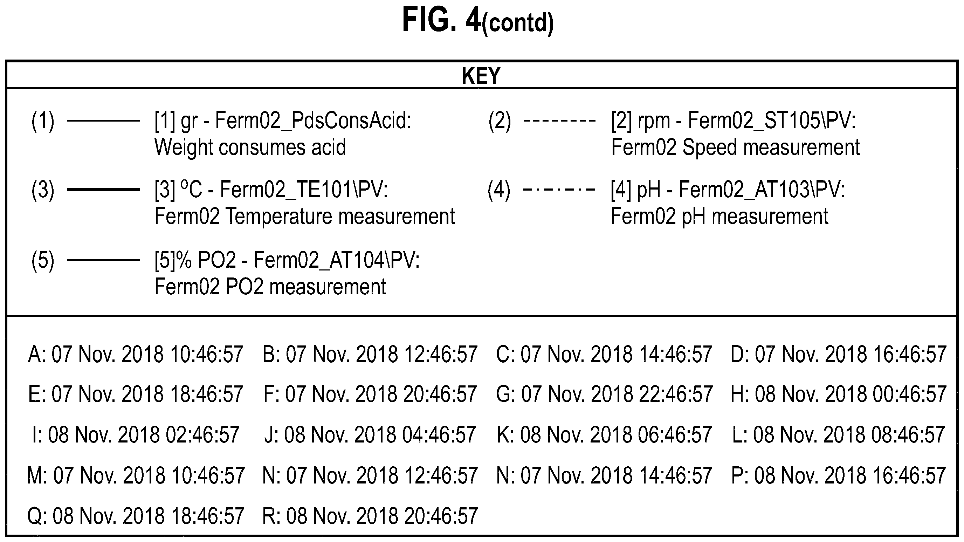

**Figure 4****.** Fermentation profile of a recombinant *B. pertussis* strain of the invention expressing LpxA from *B. parapertussis* (ΔLpxA_{Bpe}/LpxA_{Bpa}). Curve 2: stirring speed manipulated to keep dissolved oxygen concentration (DO) at 25% to avoid oxygen limitation (DO/speed regulation); Curve 3: temperature regulated at 35°C; Curve 4: pH regulated at 7.2 by addition of acetic acid (Curve 1) to avoid the basification of cell broth resulting in stopping the culture; Curve 5: dissolved oxygen concentration. NB: Due to clogging of the exhaust filter (black arrow), the DO and stirring speed dropped to 0 during the filter replacement process for about 20min. This did not affect the results of the fermentation.
**Figure 5****.** Stirring speed fermentation profiles of ΔLpxA_{Bpe}/LpxA_{Bpa} (grey) and the parental strain, Tohama I PTg (black).
**Figure 6****.** Structural analysis of LOS by LC-MS - OMVs from *B*. *pertussis* of ΔLpxA_{Bpe}/LpxA_{Bpa}, expressing genomically integrated LpxA_{Bpa}.
**Figure 7****.** Structural analysis of LOS by LC-MS - OMVs from *B. pertussis,* episomally expressing LpxA_{Bpa}.
**Figure 8****.** Structural analysis of LOS by LC-MS - OMVs from *B. pertussis* ΔArnT/ΔLpXA_{Bpe}/LpXA_{Bpa}, an ArnT and LpxA_{Bpe} double knock-out variant expressing genomically integrated LpxA_{Bpa}.
**Figure 9****.** Structural analysis of LOS by LC-MS - OMVs from *B. pertussis* ΔLpXA_{Bpe}/LpXA_{Bpa}/ΔDNT/LpxD_{Pa}, a LpxA_{Bpe} and DNT double knock-out variant expressing genomically integrated LpxA_{Bpa} and LpxD_{Pa}.
**Figure 10****.** Structural analysis of LOS by LC-MS - OMVs from *B. pertussis* ΔLpxA_{Bpe}/LpxA_{Bpa}/ΔDNT/LpxD_{Pa}/ΔLpxD_{Bpe}, a LpxA_{Bpe}, DNT and LpxD_{Bpe} triple knock-out variant expressing genomically integrated LpxA_{Bpa} and LpxD_{Pa}.
**Figure 11****.** Structural analysis of LOS by LC-MS - OMVs from *B. pertussis* LpxD_{Pa}, episomally expressing LpxD_{Pa}.
**Figure 12****.** Structural analysis of LOS by LC-MS - OMVs from *B. pertussis* ΔArnT/PagL_{Bbr}, an ArnT knock-out variant expressing PagL_{Bbr}.
**Figure 13****.** Structural analysis of LOS by LC-MS - OMVs from wild type *B. pertussis* Tomaha I Ptg.

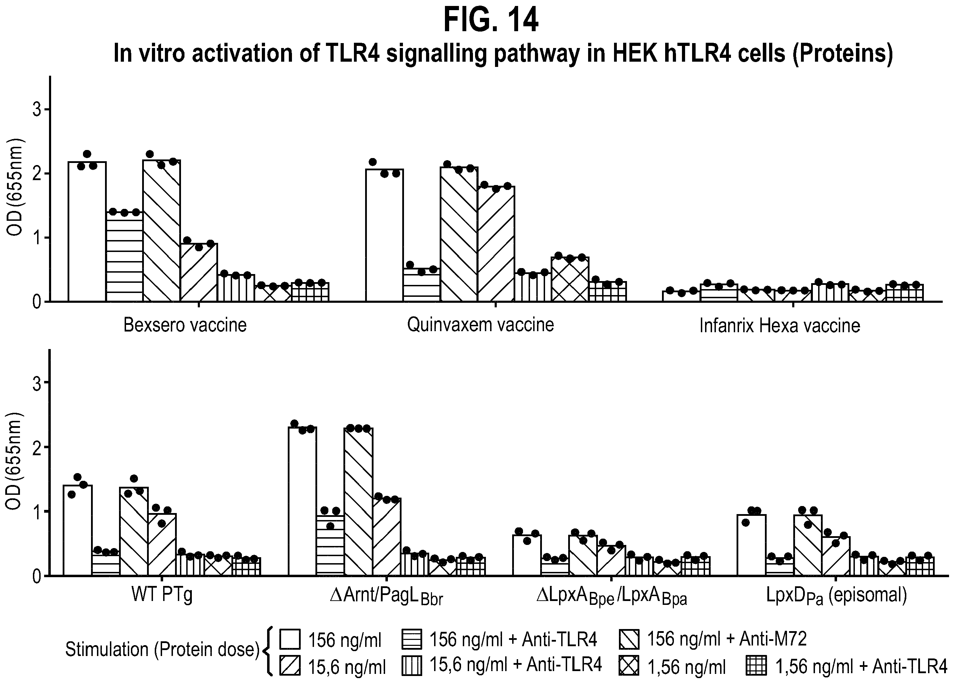

**Figure 14****.** *In vitro* activation of TLR4 signalling in HEK-hTLR4 cells. HEK-TLR4 cells were stimulated based on protein content with OMV from recombinant *B. pertussis* strains; OMV from *B. pertussis* Tohama I PTg strain (wild type control); or based on equivalent fraction of human dose (vaccine controls) with BEXSERO, QUINVAXEM or INFANRIX HEXA vaccines. Non-stimulated cells were included as a negative control (data not shown).

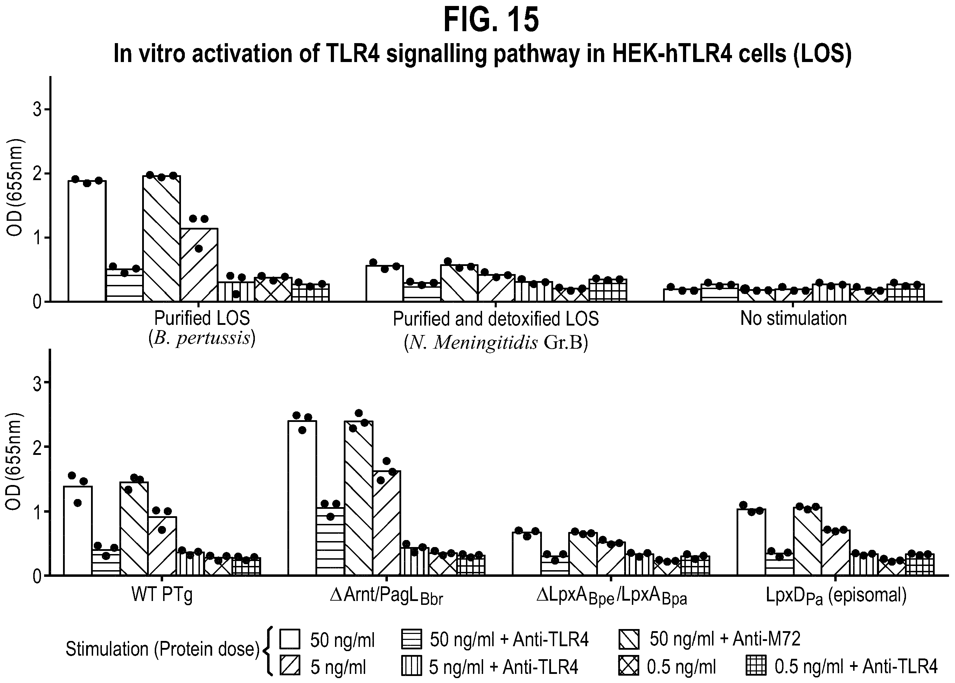

**Figure 15** *In vitro* activation of TLR4 signalling in HEK-hTLR4 cells. HEK-TLR4 cells were stimulated based on LOS content with OMV from recombinant *B. pertussis* strains; OMV from *B. pertussis* Tohama I PTg strain (wild type control); purified LPS from *B. pertussis* Tomaha I PTg; or a detoxified purified LPS from *Neisseria meningitis* B ΔMsbB strain (ΔMsbB). Non-stimulated cells were included as a negative control.

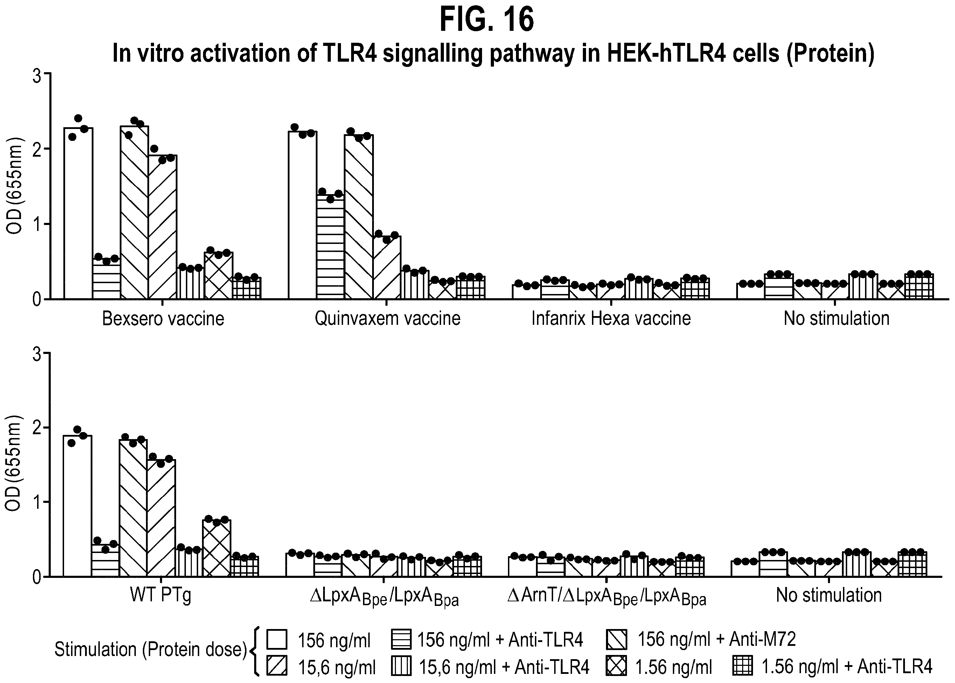

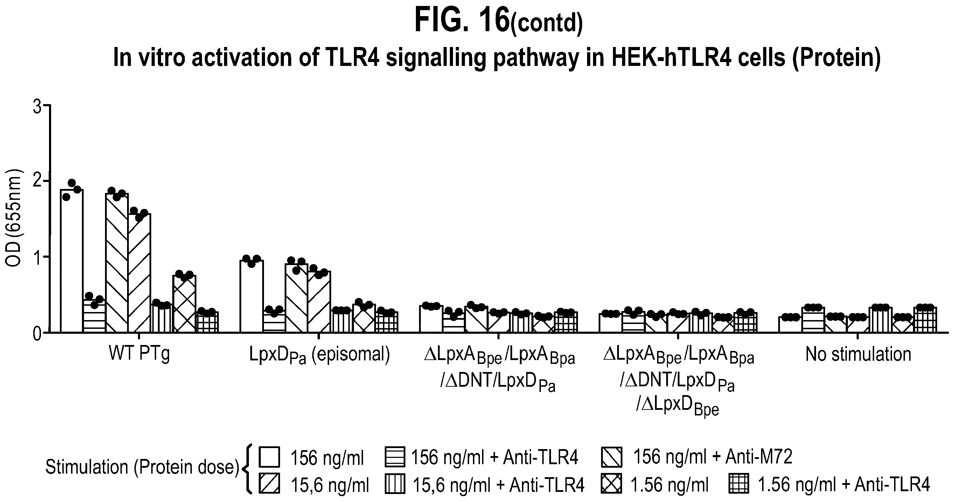

**Figure 16****.** *In vitro* activation of TLR4 signalling in HEK-hTLR4 cells. HEK-TLR4 cells were stimulated based on protein content with OMV from recombinant *B. pertussis* strains; OMV from *B. pertussis* Tohama I PTg strain (wild type control); OR BEXSERO, QUINVAXEM and INFANRIX HEXA vaccines. Non-stimulated cells were included as a negative control.

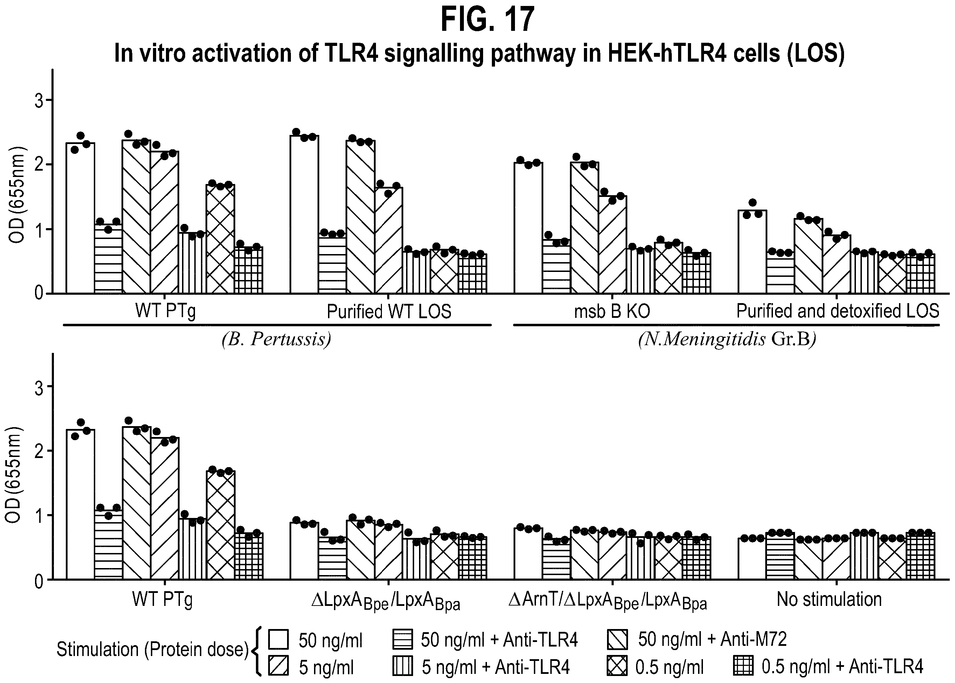

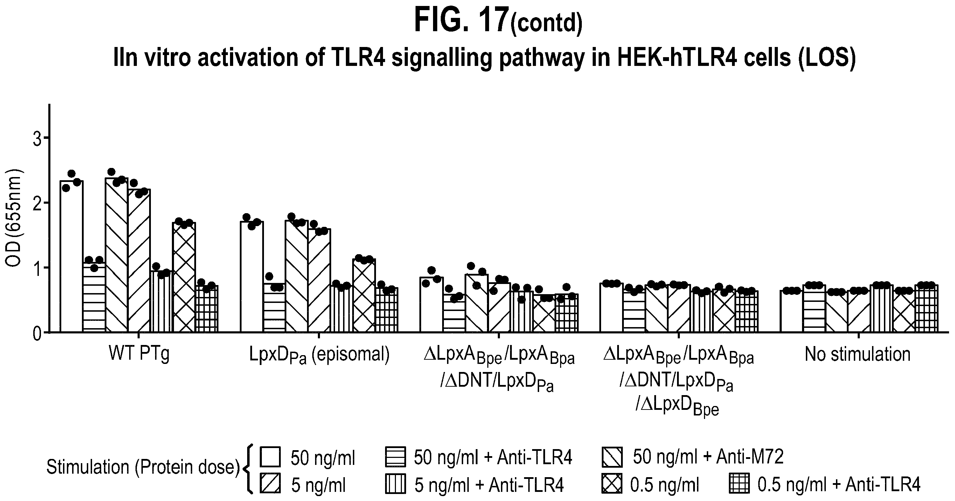

**Figure 17****.** *In vitro* activation of TLR4 signalling in HEK-hTLR4 cells. HEK-TLR4 cells were stimulated based on LOS content with OMV from recombinant *B. pertussis* strains; OMV from *B. pertussis* Tohama I PTg strain (wild type control); purified LPS from *B. pertussis;* or OMV from a detoxified *Neisseria meningitis* B ΔMsbB strain (ΔMsbB). Stimulations were normalized by LOS content. Non-stimulated cells were included as a negative control.

### DETAILED DESCRIPTION OF THE INVENTION

The invention relates to recombinant Bordetella pertussis strains comprising LPS that exhibits reduced endotoxicity when compared to LPS of wild type *Bordetella pertussis* bacterium.

Surprisingly, the present inventors have discovered that the bacterium of the invention also exhibits a bacterial growth profile comparable to that of wild-type, non-recombinant, *Bordetella pertussis* bacterium.

Use of the term "wild-type" in relation to a bacterium refers to the typical strain as it occurs in nature. Such wild-type strains lack the modifications of the invention described herein. Thus, the term "wild type" is used to refer to microorganisms of the same strain as the microorganism of the invention but which lack the genetic modifications of the invention, for example the LpxA and/or LpxD genetic modifications described herein. The wild-type strain may also be referred to as the parental strain. As used herein, "endogenous" gene or protein is used to refer to the unmodified gene (nucleic acid sequence) or protein (amino acid sequence) found in the wild-type, parental, strain of *Bordetella pertussis* bacterium from which the recombinant bacterium was originally derived.

On the other hand, the term "exogenous" is used to refer to genes (nucleic acid sequences) or proteins (amino acid sequences) that are not naturally found in the wild-type *Bordetella pertussis* bacterial strain from which the recombinant bacterium is derived. The term "exogenous" may be used interchangeably with the term "heterologous".

The present invention relates to recombinant *Bordetella pertussis* strains which comprise: at least one genomic LpxA gene encoding an LpxA protein, wherein the LpxA protein comprises a mutation at position 170 and/or a mutation at position 229 relative to SEQ ID NO: 1; and/or at least one genomic insertion of a heterologous LpxD gene.

### The bacterium

The recombinant *Bordetella pertussis* bacteria of the invention can be derived from various parental strains of *Bordetella pertussis* including, by way of non-limiting example: Tohama I, for example *Bordetella pertussis* Moreno-Lopez (ATCC: BAA-589), BP165, BPW28, 10536, 18323, 1135, BAA-2706, BAA-2705 or PT/28G [W28] (ATCC: 53894). Preferably, the recombinant *Bordetella pertussis* bacterium of the present invention is derived from a strain that expresses a genetically detoxified pertussis toxoid, particularly the genetically detoxified pertussis toxoid referred to as PT-9K/129G. Recombinant *Bordetella pertussis* strains expressing the PT-9K/129G genetically detoxified pertussis toxoid comprise two amino acid substitutions within the S1 subunit of PT, specifically R9K and E129G (see for example EP0396964). Particularly, the strain of *Bordetella pertussis* from which the recombinant bacterium is derived is Tohama I. More particularly the recombinant *Bordetella pertussis* bacterium is derived from a strain of Tohama I that expresses a genetically detoxified pertussis toxoid. Yet more particularly, the recombinant *Bordetella pertussis* bacterium is derived from a strain of Tohama I that expresses the genetically detoxified pertussis toxoid PT-9K/129G.

Still yet more particularly, the recombinant *Bordetella pertussis* bacterium comprises an S1 gene that has been modified to include the mutations R9K and E129G and expresses the genetically detoxified pertussis toxoid PT-9K/129G.

Whilst pertussis toxin is secreted by *Bordetella pertussis,* it may still be present in isolated whole-cells and outer membrane vesicles. Therefore, the use of strains that express genetically detoxified PT is advantageous. Preferably, the recombinant *Bordetella pertussis* bacterium of the invention does not express the dermonecrotic toxin (DNT) gene.

### Bacterial lipopolysaccharide and lipid A

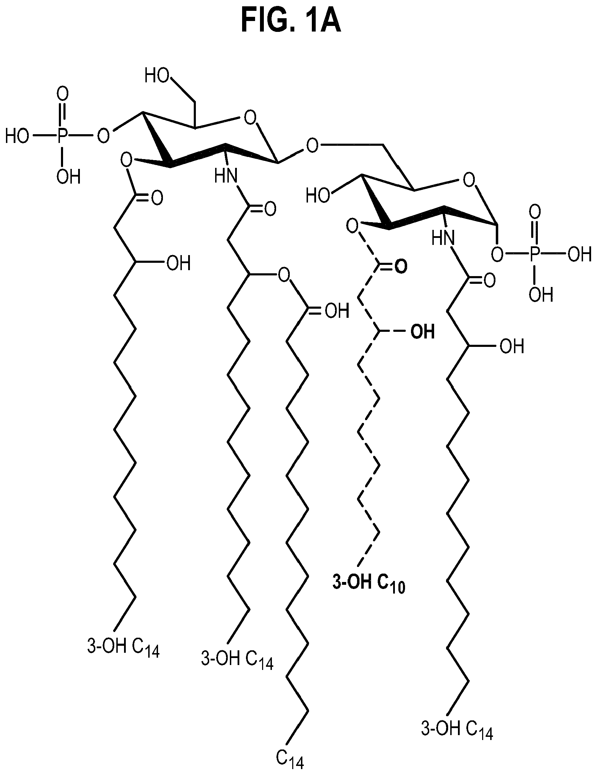

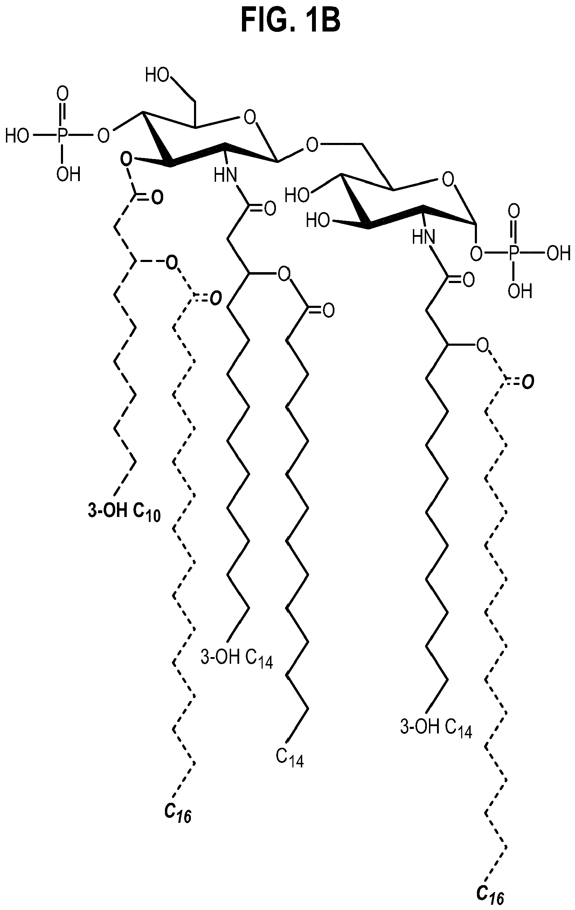

Bacterial lipopolysaccharide (LPS) is composed of a highly variable O antigen, a less variable core oligosaccharide and a highly conserved lipid moiety designated lipid A. Lipid A consists of a glucosamine disaccharide substituted with one or two phosphate groups and a number of acyl chains of varying length (Figure 1). The endotoxic activity of the LPS is mainly determined by the composition of the lipid A moiety.

The present invention is based, in part, on the modification of the length of the C2, C2' and/or C3' acyl chains of lipid A in LPS of Bordetella pertussis (Figure 1). To this end, the lipid A biosynthetic pathway of Bordetella pertussis has been engineered.

### LpxA acyltransferases

Acyltransferase LpxA (referred to as 'LpxA') catalyses the transfer of ester-linked primary β-hydroxy-myristate to a monosaccharide precursor (UDP-glucosamine) at lipid A 3 and 3' positions, determining the length of the acyl chains incorporated at both positions, which varies among gram-negative bacteria [4, 5, 6].

The endotoxic activity of the LPS is mainly determined by the composition of its lipid A moiety. Whilst variation in the number of acyl chains in lipid A can impact signalling through TLR4, the mechanism by which changes in these chains differentially affects TLR4 signalling has not been fully elucidated [2].

Thus, expression of heterologous LpxA genes might reduce the endotoxicity of LPS

LpxA from *Pseudomonas aeruginosa* (LpxA_{Pa}) and *Neisseria meningitidis* (LpxA_{Nm}) catalyse the transfer of acyl chains with a length of 10 (C₁₀) or 12 (C₁₂) carbon atoms, respectively. However, in previous studies, such as those described in WO2018/167061, LpxA*_{Pa}* or LpxA_{Nm} genes were expressed episomally in *B. pertussis,* and the endogenous LpxA gene was inactivated. Episomal expression of LpxA_{Pa} reduced the length of the C3' acyl chain from C₁₄ to C₁₀ but the resulting recombinant strains exhibited strong growth defects making them unsuitable for large-scale fermentation and use. On the other hand, episomal expression of LpxA_{Nm} in *Bordetella pertussis* was lethal.

Surprisingly, the Inventors have found that, in *Bordetella pertussis,* genomic expression of LpxA from *B. parapertussis* (*LpxA_{Bpa}*) overcomes the fitness/growth issues observed in the art whilst also reducing endotoxicity of LPS.

The amino acid sequence of LpxA from *Bordetella parapertussis* is provided as SEQ ID NO: 2. The amino acid sequence of LpxA from *Bordetella pertussis* is provided as SEQ ID NO: 1.

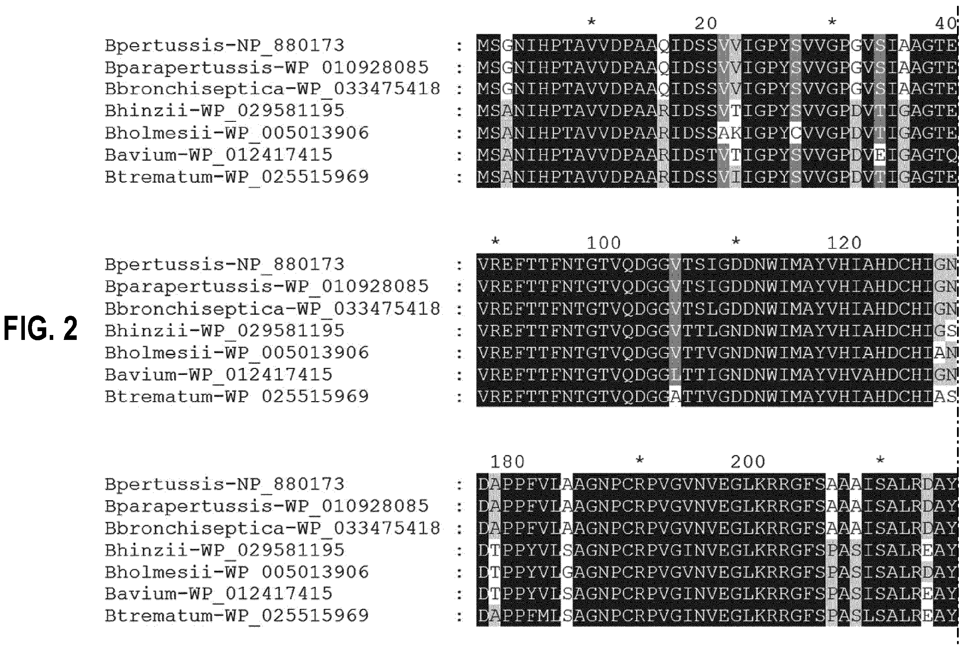

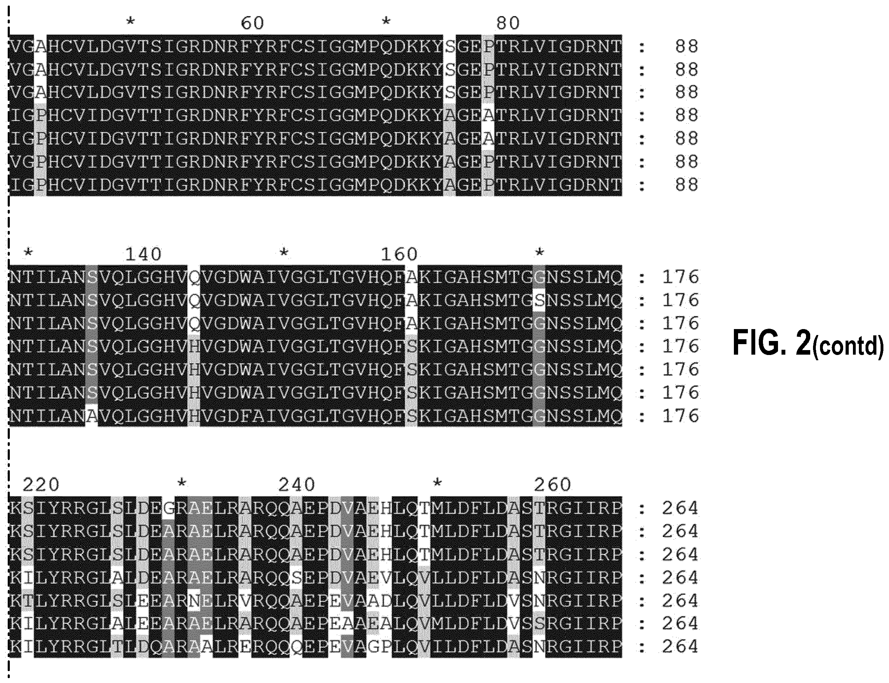

The amino acid sequence of LpxA from *B. pertussis* differs from that of *B. parapertussis* in two residues, corresponding to amino acids at positions 170 and 229 of SEQ ID NOs: 1 and 2 (Figure 2). The presence of particular substitutions at amino acid position 170 and/or position 229 is believed to alter the specificity of LpxA to selectively incorporate C₁₀ acyl chains at position C3' of Lipid A. Thus, recombinant *Bordetella pertussis* strains of the invention may express an LpxA gene which encodes an LpxA amino acid sequence in which one or both of the amino acids at positions 170 and 229 (when numbered by reference to SEQ ID NO:1) have been substituted by an amino acid residue selected from the group consisting of Alanine (A), Serine (S), Threonine (T) and Cysteine (C). Particularly, the recombinant *Bordetella pertussis* strain may express an LpxA gene that encodes an LpxA amino acid sequence in which G170 has been substituted by Serine (G170S) and/or in which G229 has been substituted by Alanine (G229A). Particularly, the recombinant *Bordetella pertussis* strain may express an LpxA gene that encodes an LpxA amino acid sequence that comprises a Serine residue at position 170 and/or an Alanine residue at position 229 (when numbered by reference to SEQ ID NO:1).

Amino acid substitutions or mutations may be introduced by standard molecular biology techniques such as random mutagenesis, site-directed mutagenesis, directed evolution, gene replacement and the like.

Preferably, the LpxA gene, in addition to the mutation(s) described above, encodes an amino acid sequence having at least 80% sequence identity with SEQ ID NO: 2, for example, 81%, at least 82%, at least 83%, at least 84%, at least 8%, at least 86%, at least 87%, at least 88%, at least 89% or 90%, at least 91%, at least 92%, at least 93%, at least 94%, at least 95%, at least 96%, at least 97%, at least 98%, at least 99% or 100% sequence identity.

Particularly, sequence identity of two sequences may be determined using the GENEPAST algorithm provided by GenomeQuest, Inc. The algorithm was formerly called "Kerr" and performs an arithmetical determination of percent sequence identity without any biological weighing (Dufresne et al, Nature Biotechnology 20, 1269-1271 (2002), or Andree et al, World Patent Information, 2008, vol. 30, issue 4, pages 300-308). Generally, sequence identity is calculated with respect to and along the entire (i.e. full) length of the longest of the sequences being compared.

### LpxD acyltransferase

LpxD acyltransferase (LpxD) catalyses the transfer of amide-linked β-hydroxy-myristate at the 2 and 2' positions of lipid A, determining the length of the acyl chains incorporated at both positions, which again varies among gram-negative bacteria [6].

The expression of exogenous LpxD variants, may improve the endotoxic profile of the bacterial LPS. Thus, recombinant *Bordetella pertussis* strains of the invention may comprise at least one heterologous LpxD gene. Particularly, the heterologous LpxD gene is integrated into the bacterial genome.

A suitable amino acid sequence of LpxD from *C. testosteroni* (LpxD_{Ct}) is provided as SEQ ID NO: 3. A suitable amino acid sequence of LpxD from *P. aeruginosa* (LpxD_{Pa}) is provided as SEQ ID NO: 4.

Thus, in some embodiments the at least one heterologous LpxD gene is an LpxD gene from *P. aeruginosa.* In some embodiments the at least one heterologous LpxD gene is an LpxD gene from *C. testosteroni.* In some embodiments, the heterologous LpxD gene replaces the locus of the dermonecrotic toxin. In some embodiments, the heterologous LpxD gene replaces the endogenous LpxD gene. In some embodiments, the heterologous LpxD gene is under the control of the BP0840 promoter (outer membrane porin promoter). Even more particularly, the wild-type copy of the LpxD gene (LpxD_{Bp}) is knocked-out.

In some embodiments the amino acid sequence encoded by the at least one heterologous LpxD gene has at least 90% identity with SEQ ID NO:3.

Thus, in some embodiments, the amino acid sequence encoded by the at least one LpxD gene has at least 90%, at least 95% or 100% identity with SEQ ID NO:3. More particularly, the LpxD amino acid sequence has at least 90%, at least 91%, at least 92%, at least 93%, at least 94%, at least 95%, at least 96%, at least 97%, at least 98%, at least 99% or 100% identity with SEQ ID NO:3.

In some embodiments the amino acid sequence encoded by the at least one heterologous LpxD gene has at least 90% identity with SEQ ID NO:4.

Thus, in some embodiments, the amino acid sequence encoded by the at least one LpxD gene has at least 90%, at least 95% or 100% identity with SEQ ID NO:4. More particularly, the LpxD amino acid sequence has at least 90%, at least 91%, at least 92%, at least 93%, at least 94%, at least 95%, at least 96%, at least 97%, at least 98%, at least 99% or 100% identity with SEQ ID NO:4.

Particularly, the recombinant *Bordetella pertussis* bacterium of the invention expresses both a heterologous LpxD gene and an LpxA gene that encodes an LpxA amino acid sequence comprising a Serine residue at position 170 and an Alanine residue at position 229. More particularly, the *B. pertussis* bacterium expresses LpxA_{Bpa} and LpxD_{Pa}.

In some embodiments, the at least one LpxA gene and the at least one LpxD genes are integrated into the bacterial genome. In other embodiments, the LpxA gene is integrated into the bacterial genome whereas the LpxD gene may be expressed episomally.

The endogenous LpxA and/or LpxD genes of the recombinant bacterium may be inactivated by means well known to the person skilled in the art. For example, the genes may be knocked out.

Generally, the endogenous LpxA gene is mutated but it may be inactivated or replaced, for example with SEQ ID NO: 2. Generally, the endogenous LpxD gene is inactivated. In some embodiments, both the endogenous LpxA and endogenous LpxD genes are inactivated.

In some embodiments, the recombinant *Bordetella pertussis* bacterium of the invention comprises additional genetic modifications, such as expression of other exogenous genes and/or inactivation of other endogenous genes. By way of non-limiting example, (1) the endogenous ArnT gene (SEQ ID NO: 18) may be inactivated, for example, deleted or knocked-out; (2) the endogenous dermonecrotic toxin (DNT) gene (SEQ ID NO: 19) is inactivated, for example, deleted or knocked-out; (3) the bacterium may express PagL (SEQ ID NO: 16) from *B. bronchiseptica*; (4) the bacterium may express an LpxE protein from a heterologous gram-negative bacterium (SEQ ID NO: 6 or 7); and/or (5) the bacterium may express an LpxF protein from a heterologous gram-negative bacterium (SEQ ID NO: 8).

### Lipid A acyl chains

Generally, Lipid A of gram-negative bacteria consists of a β-(1→6)-linked disaccharide acylated with up to eight fatty acids of different lengths and complexities as well as charged substituents such as phosphate groups, phosphoethanolamine residues, or positively charged sugar residues and there are many variations within lipid A of different bacteria species. The acylation at positions 2 and 2' of the disaccharide (reducing and terminal sugar residue, respectively) leads to amide-linked lipids, which is also the case for the 3- and 3'-positions when the 3-amino-3-deoxy-d-glucosamine is acylated. The hydroxyl groups at positions 3 and 3' as well as the 3-hydroxyl group of the fatty acyl chains are also acylated, resulting in ester-linked substituents. The extent of acylation with 4-8 acyl groups attached to the β-(1→6)-linked disaccharide varies between species and more than a single form is often present in the lipid A from a certain bacterial species.

Generally, the length of the acyl chains at positions C2, C2' and C3' of Lipid A in wild-type *Bordetella pertussis* is C₁₄. As used herein, 'C' followed by an integer in subscript denotes a molecule based upon a carbon chain of a length denoted by the integer. Thus, C₁₄ and C₁₀ would denote molecules having a 14-carbon atom chain and a 10-carbon atom chain respectively.

The recombinant bacterium of the invention produces lipid A in which the length of the acyl chain at position C2 and/or C2' and/or C3' is reduced in comparison to the length of the corresponding acyl chains of lipid A produced by the wild-type gram-negative bacterium.

Particularly, the length of the acyl chain at position C2 is less than C₁₄, such as C₆ to C₁₂, C₈ to C₁₂ or C₁₀ to C₁₂, particularly C₁₀ or C₁₂, for example, C₆, C₈, C₁₀ or C₁₂. Particularly, the length of the acyl chain at position C2' is less than C₁₄, such as C₆ to C₁₂, C₈ to C₁₂ or C₁₀ to C₁₂, particularly C₁₀ or C₁₂, for example, C₆, C₈, C₁₀ or C₁₂. Particularly, the length of the acyl chain at position C3' is less than C₁₄, such as C₆ to C₁₂, C₈ to C₁₂ or C₁₀ to C₁₂, particularly C₁₀ or C₁₂, for example, C₆, C₈, C₁₀ or C₁₂.

In some embodiments, the length of the acyl chains at both position C2 and position C2' are less than C₁₄, such as C₆ to C₁₂, C₈ to C₁₂ or C₁₀ to C₁₂, particularly C₁₀ or C₁₂, for example, C₆, C₈, C₁₀ or C₁₂. In some embodiments, the length of the acyl chains at both position C2 and position C3' are less than C₁₄, such as C₆ to C₁₂, C₈ to C₁₂ or C₁₀ to C₁₂, particularly C₁₀ or C₁₂, for example, C₆, C₈, C₁₀ or C₁₂. In some embodiments, the length of the acyl chains at both position C2' and position C3' are less than C₁₄, such as C₆ to C12, C₈ to C₁₂ or C₁₀ to C₁₂, particularly C₁₀ or C₁₂, for example, C₆, C₈, C₁₀ or C₁₂. In some embodiments, the length of the acyl chains at position C2, position C2' and position C3' are less than C₁₄, such as C₆ to C₁₂, C₈ to C₁₂ or C₁₀ to C₁₂, particularly C₁₀ or C₁₂, for example, C₆, C₈, C₁₀ or C₁₂.

Lipid A produced by the recombinant bacterium of the invention may exist as a mixture of species having acyl chains of differing lengths.

Thus, considering the total population of lipid A in the cell, at least 10% of the acyl chains at the C2 position are C₁₀ or C₁₂ and/or at least 10% of the acyl chains at the C2' position are C₁₀ or C₁₂ and/or at least 10% of the acyl chains at the C3' position are C₁₀ or C₁₂. Particularly, at least 10% may be at least 12%, at least 15%, at least 20%, at least 24%, at least 25%, at least 30%, at least 35%, at least 40%, at least 50%, at least 60%, at least 70%, at least 80%, at least 85%, at least 86%, at least 87%, at least 88%, at least 89%, at least 90%, at least 95%, at least 96%, at least 97%, at least 98%, at least 99% or 100%. Preferably at least 10% is at least 80%, more preferably at least 90%. Yet more particularly about 100%

### Bacterial growth

Recombinant strains of the invention are stable whilst also being suitable for growth at large scale, for example, in manufacturing. Surprisingly, the growth profile of recombinant *Bordetella pertussis* bacteria of the invention is comparable to that of the wild-type, parental, strain indicating that neither the LpxA mutation(s) nor the LpxD mutation(s) exemplified are detrimental to culture growth.

The recombinant bacterial strains of the invention are thus particularly suitable, for example, for the preparation of whole-cell bacterial antigens and/or OMVs, which may then be used as components of immunogenic compositions such as vaccines.

Thus, the growth rate of a culture of the recombinant gram-negative bacterium of the invention is comparable to that of a culture of the wild type, parental, strain. Particularly, in standard fermentation processes, for example in 10 litre fermentation volumes, the values of the elapsed time and growth rate are substantially similar, for example, vary by less than 20%, less than 15%, less than 10% or less than 5%.

### Outer Membrane Vesicles (OMVs)

In a second aspect, the present invention relates to an outer membrane vesicle or population of outer membrane vesicles derived from the recombinant *Bordetella pertussis* bacterium of the first aspect.

OMVs are lipid bilayer nanoscale spherical particles (10-500 nm in diameter) naturally and constitutively released by Gram negative bacteria during growth. OMVs are generated through a "budding out" of the bacterial outer membrane and, consistent with this, they have a composition similar to that of the bacterial outer membrane, including lipopolysaccharide (LPS), glycerophospholipids, outer membrane proteins, and periplasmic components.

Typically, outer membrane vesicles are prepared artificially from bacteria, and may be prepared using detergent treatment (e.g. with deoxycholate), or by non-detergent means.

Techniques for forming OMVs include treating bacteria with a bile acid salt detergent (*e.g.* salts of lithocholic acid, chenodeoxycholic acid, ursodeoxycholic acid, deoxycholic acid, cholic acid, ursocholic acid, *etc*.) or with sodium deoxycholate [7 and 8]. Other techniques may be performed substantially in the absence of detergent [9 and 10] using techniques such as sonication, homogenisation, microfluidisation, cavitation, osmotic shock, grinding, French press, blending, *etc.* OMVs used with the invention may be prepared using an OMV extraction buffer having about 0.5% deoxycholate or lower, for example, about 0.4%, about 0.3%, about 0.2%, about 0.1%, <0.05% or even zero.

OMVs may also form spontaneously during bacterial growth and are released into culture medium. Thus, OMVs can be obtained by culturing bacteria of the invention in culture medium, separating whole cells from the smaller OMVs in the culture medium. Bacterial vesicles can conveniently be separated from whole bacteria by filtration *e.g.* through a 0.22µm filter. Bacterial filtrates may be clarified by centrifugation, for example high speed centrifugation (e.g. 20,000 x g for about 2 hours). Another useful process for OMV preparation is described in [11] and involves ultrafiltration of crude OMVs, instead of high speed centrifugation. The process may involve a step of ultracentrifugation after the ultrafiltration takes place. A simple process for purifying bacterial vesicles is described in [12], comprising: (i) a first filtration step in which the vesicles are separated from the bacteria based on their different sizes, with the vesicles passing into the filtrate *e.g.* using a 0.22µm microfiltration; and (ii) a second filtration step in which the vesicles are retained in the retentate e.g. using a 0.1µm microfiltration. The two steps can both use tangential flow filtration.

OMVs of the invention will include LOS (also known as LPS). The pyrogenic effect of LOS in OMVs may be lower than that seen with the same amount of purified LOS. Therefore, preferably when looking at pyrogenicity, generally OMVs from bacteria of the invention will be compared with OMVs derived from the parental strain. LOS levels are expressed in International Units (IU) of endotoxin and can be tested by the LAL assay (limulus amebocyte lysate). LOS may be present at less than 2000 IU per µg of OMV protein.

In some embodiments of the invention, the OMV is a detergent extracted OMV (dOMV). The term "dOMV" encompasses a variety of proteoliposomic vesicles obtained by disruption of the outer membrane of a Gram-negative bacterium typically by a detergent extraction process to form vesicles therefrom. Particularly, OMVs of the invention are dOMVs prepared using deoxycholate. More particularly about 0.5% deoxycholate or lower, for example, about 0.4%, about 0.3%, about 0.2%, about 0.1% or about 0.05%.

dOMVs of the invention may have a size distribution of between from about 20 to about 500 nm as measured by Dynamic Light Scattering DLS technique. Particularly, dOMVs of the invention may have a size distribution of between from about 50 to about 500 nm. More particularly, the dOMVs may have a size distribution of between from about 75 to about 250 nm, for example, from about 75 to about 150 nm. Even more particularly, the dOMVs of the invention may have an average diameter of about 20, 25, 30, 35, 40, 45, 50, 55, 60, 65, 70, 75, 80, 85, 90, 95, 100, 105, 110, 115, 120, 125, 130, 135, 140, 145 or 150 nm.

In some embodiments, bacteria of the invention can be modified to have up-regulated antigens or expression of heterologous antigens (*i.e.* antigens not native to the particular bacterial strain). As a result of this modification, vesicles prepared from such modified bacteria contain higher levels of the up-regulated or heterologous antigen(s). The increase in expression in the vesicles (measured relative to a corresponding wild-type strain) of the up-regulated antigen is usefully at least 10%, measured in mass of the relevant antigen per unit mass of vesicle, and is more usefully at least 20%, 30%, 40%, 50%, 75%, 100% or more.

### Whole-cell pertussis antigens

Recombinant *Bordetella pertussis* bacteria of the invention may be used as whole-cell pertussis (wP) antigens, in the form of inactivated *B.pertussis* cells. Preparation of cellular pertussis antigens is well documented in the art and may be obtained by heat inactivation of phase I culture of *B.pertussis* cells. Quantities of wP antigens can be expressed in international units (IU). For example, the NIBSC supplies the *'International Standard For Pertussis Vaccine'* (NIBSC code: 94/532), which contains 40 IU per ampoule.

When used in immunogenic compositions of the invention, the cellular pertussis antigen is typically present in an amount that is capable of eliciting an immune response when administered. Ideally, the cellular pertussis antigen can elicit a protective immune response. The amount of wP antigen in immunogenic compositions of the invention is typically at least 4 IU/dose. The wP antigen may be adsorbed onto or mixed with an aluminium adjuvant, for example, an aluminium phosphate adjuvant.

### TLR4 signalling cascade

The bacterium of the invention produces lipid A with reduced endotoxic activity when compared to that of lipid A from the wild-type strain, particularly when measured using a TLR4 stimulation assay.

TLR4 stimulation assays are well known to the skilled person. The stimulation of the TLR4 signalling cascade can lead to the activation of NF-κB and/or activator protein 1 (AP-1) transcription factors that are contributing to the expression of several pro-inflammatory cytokines, e.g. IL-1, IL-6, IL-8 and TNF-α and thus play a key role in inflammation. Determination of transcription activity in certain lines, for example HEK-Blue™-hTLR4 cells (Invivogen), can be used to assess stimulation of the TLR4 signalling cascade. More specifically, the inducible reporter gene secreting embryonic alkaline phosphatase (SEAP) of the HEK-Blue™-hTLR4 cells is under control of the IL-12 p40 minimal promoter fused to five NF-κB and AP-1 binding sites. Upon stimulation with a TLR4 ligand, activation of NF-kB and AP-1 induces the secretion of alkaline phosphatase that can be quantified in the supernatant by measuring optical density at 655 nm (OD₆₅₅).

Endotoxic activity of bacteria of the invention, for example whole-cell antigens or OMVs derived from bacteria of the invention may be determined using a TLR4 stimulation assay, particularly a human TLR4 stimulation assay, more particularly a human TLR4 stimulation assay with optical density (OD) measured at 655 nm. Particularly, the endotoxic activity of whole-cells or OMVs of the invention is compared to that of the wild-type parental strain. The capacity to activate the TLR4 signalling by OMVs derived from the bacterium of the invention may be compared to that of OMVs derived from wild type strains as well as to those from other bacteria or commercial immunogenic compositions, for example, BEXSERO (Meningitis B vaccine containing OMVs from Neisseria) or QUINVAXEM (whole-cell (wP) pertussis vaccine).

More particularly, endotoxic activity of OMVs of the invention exhibit a reduced or decreased activation of the TLR4 signalling cascade compared to the endotoxic activity of OMVs derived from the wild-type bacterium. Specifically, the OD₆₅₅ values obtained using the TLR4 stimulation assay when testing the LpxA and/or LpxD mutants should be similar, for example about ± 0.5 OD₆₅₅, to the OD₆₅₅ values obtained using the non-stimulated cells and/or to the OD₆₅₅ values observed with cells stimulated with an acellular pertussis (aP) vaccine.

In some embodiments, the TLR4 stimulation assays show a partial decrease in activation of the TLR4 signalling cascade for the OMVs of the invention in comparison to OMVs derived from wild-type bacteria. Specifically, the OD₆₅₅ values observed with the LpxA and/or LpxD bacteria or OMVs of the invention may be higher (≥0.5 OD₆₅₅) than the OD₆₅₅ values observed in the non-stimulated cells and/or to the OD₆₅₅ values observed with cells stimulated with aP vaccine, but lower than the OD₆₅₅ values observed with the cells stimulated with OMV derived from wild type bacterium and/or with a wP vaccine and/or the OMV-containing MenB vaccine.

### Immunogenic compositions

In a third aspect, the invention relates to immunogenic compositions comprising at least one outer membrane vesicle derived from the recombinant Bordetella pertussis bacterium of the invention and a pharmaceutically acceptable excipient.

OMVs of the invention are useful as active ingredients in immunogenic compositions. Similarly, whole-cell antigens comprising whole recombinant *Bordetella pertussis* cells of the invention which have been killed and deactivated (e.g. by treatment with formalin and/or heat), are also useful as active ingredients in immunogenic compositions.

The term "immunogenic composition" broadly refers to any composition that may be administered to elicit an immune response, such as an antibody or cellular immune response, against an antigen or antigens present in the composition. Thus, compositions of the invention are immunogenic.

When the immunogenic compositions prevent, ameliorate, palliate or eliminate disease from the subject, then such compositions may be referred to as a vaccine. Vaccines according to the invention may either be prophylactic (i.e. to prevent infection) or therapeutic (i.e. to treat infection), but will typically be prophylactic. In certain embodiments, the immunogenic composition is a vaccine. Prophylactic vaccines do not guarantee complete protection from disease because even if the patient develops antibodies, there may be a lag or delay before the immune system is able to fight off the infection. Therefore, and for the avoidance of doubt, the term prophylactic vaccine may also refer to vaccines that ameliorate the effects of a future infection, for example by reducing the severity or duration of such an infection.

The terms "protection against infection" and/or "provide protective immunity" means that the immune system of a subject has been primed (e.g by vaccination) to trigger an immune response and repel infection. Particularly, the immune response triggered is capable of repelling infection against *Bordetella pertussis.* A vaccinated subject may thus get infected, but is better able to repel the infection than a control subject. Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. Commonly, the desired result is the production of an antigen (e.g., pathogen)-specific immune response that is capable of or contributes to protecting the subject against the pathogen. This amount varies depending upon the health and physical condition of the individual to be treated, age, the taxonomic group of individual to be treated (*e.g.* non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

The term "antigen" refers to a substance that, when administered to a subject, elicits an immune response directed against the substance. In the context of the present invention, OMVs of the invention are antigens. Particularly, when administered to a subject the immunogenic composition will elicit an immune response directed against *Bordetella,* for example, *Bordetella pertussis.* Particularly the immune response directed against *Bordetella* is protective, that is, it can prevent or reduce infection or colonisation caused by *Bordetella,* particularly *Bordetella pertussis.* Compositions may thus be pharmaceutically acceptable. Generally, immunogenic compositions include components in addition to the antigens *e.g.* they typically include one or more pharmaceutical carrier(s) and/or excipient(s). A thorough discussion of such components is available in reference [13].

A "pharmaceutically acceptable carrier" is a carrier that does not itself induce the production of antibodies. Such carriers are well known to those of ordinary skill in the art and include, by way of non-limiting example, polysaccharides, polylactic acids, polyglycolic acids, amino acid copolymers, sucrose, trehalose, lactose, and lipid aggregates (such as oil droplets or liposomes). Immunogenic compositions may also contain diluents, such as water, saline, glycerol, and the like. Sterile pyrogen-free, phosphate-buffered physiologic saline is a typical diluent. Such compositions may also include, auxiliary substances, such as wetting or emulsifying agents, pH buffering substances, etc.

Compositions will generally be administered to a subject, for example a patient, particularly a suitable mammal, such as a human, in aqueous form. Prior to administration, however, the composition may have been in a non-aqueous form. For instance, although some vaccines are manufactured in aqueous form, then filled and distributed and administered also in aqueous form, other vaccines are lyophilised during manufacture and are reconstituted into an aqueous form at the time of use. Thus, a composition of the invention may be dried, such as a lyophilised formulation.

The composition may include a preservative such as thiomersal or 2-phenoxyethanol. It is preferred, however, that the vaccine should be substantially free from (*i.e.* less than 5µg/ml) mercurial material *e.g.* thiomersal-free. Vaccines containing no mercury are more preferred. Thiomersal-free vaccines are particularly preferred.

To control tonicity, a physiological salt, such as a sodium salt may be included. Sodium chloride (NaCl) may be used, which may be present at between 1 and 20 mg/ml *e.g.* about 10±2mg/ml NaCl.

Compositions will generally have an osmolality of between 200 mOsm/kg and 400 mOsm/kg, particularly between 240-360 mOsm/kg, and more particularly within the range of 290-310 mOsm/kg.

Compositions may include one or more buffers. Typical buffers include: a phosphate buffer; a Tris buffer; a borate buffer; a succinate buffer; a histidine buffer; or a citrate buffer. Buffers will typically be included in the 5-20mM range.

The pH of a composition will generally be between 5.0 and 8.1, and more typically between 6.0 and 8.0 *e.g.* 6.5 and 7.5, or between 7.0 and 7.8.

The composition is preferably sterile. Particularly the composition is non-pyrogenic *e.g.* containing <1 EU (endotoxin unit, a standard measure) per dose, for example, <0.1 EU per dose. The composition may be gluten free.

The composition may include material for a single immunisation, or may include material for multiple immunisations (*i.e.* a 'multidose' kit). The inclusion of a preservative is preferred in multidose arrangements. As an alternative (or in addition) to including a preservative in multidose compositions, the compositions may be contained in a container having an aseptic adaptor for removal of material.

Immunogenic compositions, for example human vaccines, are typically administered in a dosage volume of about 0.5ml, although a half dose (*i.e.* about 0.25ml) may be administered, for example, to children.

Immunogenic compositions of the invention may also comprise one or more immunoregulatory agents. Preferably, one or more of the immunoregulatory agents include one or more adjuvants. As used herein, "adjuvant" means a compound or substance (or combination of compounds or substances) that, when administered to a subject in conjunction with an antigen or antigens, for example as part of an immunogenic composition or vaccine, increases or enhances the subject's immune response to the administered antigen or antigens (compared to the immune response obtained in the absence of adjuvant). The adjuvants may include a TH1 adjuvant and/or a TH2 adjuvant, further discussed below.

Adjuvants which may be used in compositions of the invention include, mineral containing compositions such as aluminium salts and calcium salts. The compositions of the invention may include mineral salts such as hydroxides (*e.g.* oxyhydroxides), phosphates (*e.g.* hydroxyphosphates, orthophosphates), sulphates, *etc.* [*e.g.* see chapters 8 & 9 of ref.14], or mixtures of different mineral compounds, with the compounds taking any suitable form (*e.g.* gel, crystalline, amorphous, *etc*.), and with adsorption being preferred. The mineral containing compositions may also be formulated as a particle of metal salt.

The adjuvants known as "aluminium hydroxide" are typically aluminium oxyhydroxide salts, which are usually at least partially crystalline. Aluminium oxyhydroxide, which can be represented by the formula AIO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ [chapter 9 of ref. **Error! Bookmark not defined.**4]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (e.g. as seen in transmission electron micrographs) is typical for aluminium hydroxide adjuvants. The pl of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminium phosphate" are typically aluminium hydroxyphosphates, often also containing a small amount of sulfate (*i.e.* aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (e.g. when heated to 200°C) indicates the presence of structural hydroxyls [chapter 9 of ref. **Error! Bookmark not defined.**4].

The PO₄/Al³⁺ molar ratio of an aluminium phosphate adjuvant may be between 0.3 and 1.2, particularly between 0.8 and 1.2, and more particularly 0.95±0.1. The aluminium phosphate may be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g.* plate-like morphology as seen in transmission electron micrographs). Typical diameters of the particles are in the range 0.5-20µm (*e.g.* about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention may have a PZC of between 4.0 and 7.0, more particularly between 5.0 and 6.5 *e.g.* about 5.7.

Suspensions of aluminium salts used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e.g.* present at a concentration between 1.0 and 20 mM, between 5 and 15 mM, particularly about 10 mM. The suspensions may also comprise sodium chloride.

In some embodiments, an adjuvant component includes a mixture of both an aluminium hydroxide and an aluminium phosphate. In this case there may be more aluminium phosphate than hydroxide *e.g.* a weight ratio of at least 2:1 *e.g.* ≥5:1, ≥6:1, ≥7:1, ≥8:1, ≥9:1, *etc.* In some embodiments, an adjuvant component includes aluminium phosphate. In some embodiments, an adjuvant component includes aluminium hydroxyphosphate sulfate.

The concentration of Al⁺⁺⁺ in a composition for administration to a patient may be between from 10mg/ml to 0.01 mg/ml, for example, less than 5 mg/ml, less than 4 mg/ml, less than 3 mg/ml, less than 2 mg/ml, less than 1 mg/ml, for example, about 5mg/ml, about 4mg/ml, about 3mg/ml, about 2mg/ml, about 1mg/ml, about 0.3mg/ml, about 0.05mg/ml or about 0.01mg/ml. A particular range is between from about 0.3mg/ml to about 1mg/ml. In some embodiments, a maximum of 0.85mg/dose is preferred, for example about 0.5mg/l or about 0.3mg/ml.

Oil emulsion compositions suitable for use as adjuvants in the invention include squalene-water emulsions, such as MF59 [Chapter 10 of ref. **Error! Bookmark not defined.**4; see also ref. 15] (5% Squalene, 0.5% Tween 80, and 0.5% Span 85, formulated into submicron particles using a microfluidizer). Complete Freund's adjuvant (CFA) and incomplete Freund's adjuvant (IFA) may also be used.

Saponin formulations may also be used as adjuvants in the invention. Saponins are a heterogeneous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

AS01 is an Adjuvant System containing MPL (3-O-desacyl-4'- monophosphoryl lipid A), QS21 ((Quillaja saponaria Molina, fraction 21) Antigenics, New York, NY, USA) and liposomes. AS01B is an Adjuvant System containing MPL, QS21 and liposomes (50 µg MPL and 50 µg QS21). AS01E is an Adjuvant System containing MPL, QS21 and liposomes (25 µg MPL and 25 µg QS21). In one embodiment, the immunogenic composition or vaccine comprises AS01. In another embodiment, the immunogenic composition or vaccine comprises AS01B or AS01E. In a particular embodiment, the immunogenic composition or vaccine comprises AS01E. AS02 is an Adjuvant system containing MPL and QS21 in an oil/water emulsion. AS02V is an Adjuvant System containing MPL and QS21 in an oil/water emulsion (50 µg MPL and 50 µg QS21). AS03 is an Adjuvant System containing α-Tocopherol and squalene in an oil/water (o/w) emulsion. AS03A is an Adjuvant System containing α-Tocopherol and squalene in an o/w emulsion (11.86 mg tocopherol). AS03B is an Adjuvant System containing α-Tocopherol and squalene in an o/w emulsion (5.93 mg tocopherol). AS03C is an Adjuvant System containing α-Tocopherol and squalene in an o/w emulsion (2.97 mg tocopherol). In one embodiment, the immunogenic composition or vaccine comprises AS03. AS04 is an Adjuvant System containing MPL (50 µg MPL) adsorbed on an aluminium salt (500 µg AI3+). In one embodiment, the immunogenic composition or vaccine comprises AS04. A system involving the use of QS21 and 3D-MPL is disclosed in WO 94/00153. A composition wherein the QS21 is quenched with cholesterol is disclosed in WO 96/33739. An additional adjuvant formulation involving QS21, 3D-MPL and tocopherol in an oil in water emulsion is described in WO 95/17210. In one embodiment the immunogenic composition additionally comprises a saponin, which may be QS21. The formulation may also comprise an oil in water emulsion and tocopherol (WO 95/17210). Unmethylated CpG containing oligonucleotides (WO 96/02555) and other immunomodulatory oligonucleotides (WO 0226757 and WO 03507822) are also preferential inducers of a TH1 response and are suitable for use in the present invention.

Additional adjuvants include Toll like receptor agonists, (in particular Toll like receptor 2 agonist, Toll like receptor 3 agonist, Toll like receptor 4 agonist, Toll like receptor 7 agonist, Toll like receptor 8 agonist and Toll like receptor 9 agonist). Compositions of the invention may include one or more small molecule immunopotentiators. For example, the composition may include a TLR2 agonist (e.g. Pam3CSK4), a TLR4 agonist (e.g. an aminoalkyl glucosaminide phosphate, such as E6020), a TLR7 agonist (e.g. imiquimod), a TLR8 agonist (e.g. resiquimod (also a TLR7 agonist)) and/or a TLR9 agonist (e.g. IC31). Any such agonist ideally has a molecular weight of <2000Da.

TLR7 agonists used with the invention ideally includes at least one adsorptive moiety. The inclusion of such moieties in TLR agonists allows them to adsorb to insoluble aluminium salts (e.g. by ligand exchange or any other suitable mechanism) and improves their immunological behaviour. Phosphorus-containing adsorptive moieties are particularly useful, and so an adsorptive moiety may comprise a phosphate, a phosphonate, a phosphinate, a phosphonite, a phosphinite, etc.The TLR agonist may include at least one phosphonate group. In particular embodiments, a composition of the invention may include a TLR7 agonist which includes a phosphonate group. This phosphonate group can allow adsorption of the agonist to an insoluble aluminium salt. In some embodiments, the TLR agonist is 3-(5-amino-2-(2-methyl-4-(2-(2-(2-phosphonoethoxy)ethoxy)ethoxy)phenethyl)benzo [f]-[1,7]naphthyridin-8-yl)propanoic acid, as shown below.

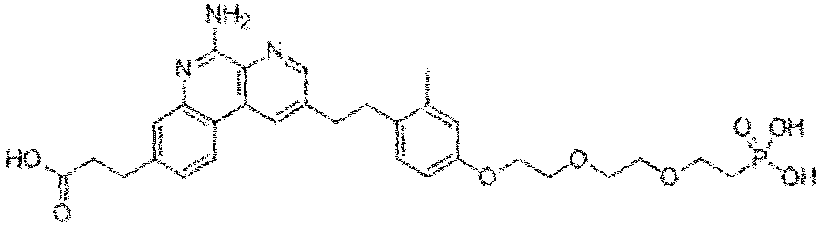

Preferred TLR agonists are water-soluble. Thus, they can form a homogenous solution when mixed in an aqueous buffer with water at pH 7 at 25°C and 1 atmosphere pressure to give a solution which has a concentration of at least 50 µg/ml. The term "water-soluble" thus excludes substances that are only sparingly soluble under these conditions.

Compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g.* a lyophilised composition or a spray-freeze dried composition). The composition may be prepared for topical administration *e.g.* as an ointment, cream or powder. The composition may be prepared for pulmonary administration *e.g.* as an inhaler, using a fine powder or a spray. The composition may be prepared for nasal, aural or ocular administration e.g. as drops. The composition may be in kit form, designed such that a combined composition is reconstituted just prior to administration to a patient. Such kits may comprise one or more antigens in liquid form and one or more lyophilised antigens.

Where a composition is to be prepared extemporaneously prior to use (*e.g.* where a component is presented in lyophilised form) and is presented as a kit, the kit may comprise two vials, or it may comprise one ready-filled syringe and one vial, with the contents of the syringe being used to reactivate the contents of the vial prior to injection.

### Combination Vaccines

Immunogenic compositions of the invention will generally be combination vaccines and, in addition to OMVs of the invention, may include further protective antigen(s) from *Bordetella pertussis* and at least one pathogen other than *Bordetella pertussis.*

The additional protective antigen(s) can be viral and/or bacterial. In addition to *Bordetella pertussis,* typical bacterial pathogens include, but are not limited to: *Corynebacterium diphtheriae; Clostridium tetani*; *Haemophilus influenzae* type b. Typical viral pathogens include, but are not limited to: poliovirus and hepatitis B virus. For the avoidance of doubt, reference to additional antigens is intended to refer to further antigenic components included in the immunogenic compositions of the invention beyond the constituents of the OMVs. For example, small amounts of pertussis toxoid may be found as a constituent of OMVs but reference to pertussis toxoid as a further antigen refers to an amount of pertussis toxoid that is specifically added over and above the OMV constituent, for example, as an isolated protein antigen.

### Acellular pertussis antigens

In addition to OMVs of the invention, immunogenic compositions of the invention may further comprise one or more acellular pertussis (aP) antigens, particularly selected from the following well-known and well-characterized *B.pertussis* antigens: (1) detoxified pertussis toxin (pertussis toxoid, or 'PT'); (2) filamentous hemagglutinin ('FHA'); (3) pertactin (also known as 'PRN' or the '69 kiloDalton outer membrane protein'); (4) fimbriae type 2 ('FIM2'); (5) fimbriae type 3 ('FIM 3'). It is most preferred that both detoxified PT and FHA are used. In some embodiments PT, FHA and PRN are used. These antigens are preferably prepared by isolation from *B.pertussis* culture grown in modified Stainer-Scholte liquid medium. PT and FHA can be isolated from the fermentation broth (*e.g.* by adsorption on hydroxyapatite gel), whereas pertactin can be extracted from the cells by heat treatment and flocculation (*e.g.* using barium chloride). The antigens can be purified in successive chromatographic and/or precipitation steps. PT and FHA can be purified by hydrophobic chromatography, affinity chromatography and size exclusion chromatography. Pertactin can be purified by ion exchange chromatography, hydrophobic chromatography and size exclusion chromatography.

FHA and pertactin may be treated with formaldehyde prior to use according to the invention. PT may be detoxified by treatment with formaldehyde and/or glutaraldehyde. As an alternative to this chemical detoxification procedure preferably the PT may be a mutant PT in which enzymatic activity has been reduced by mutagenesis, for example, genetically detoxified PT, preferably the PT-9K/129G.

In some embodiments, immunogenic compositions of the invention further comprise the fimbrial antigens FIM2 and FIM3.

The aP antigen(s) may be used in an unadsorbed state, but may be adsorbed onto one or more aluminium salt adjuvant(s) before being used. Typically, the aP antigens are substantially free from mercurial preservatives such as thimerosal.

The acellular pertussis antigens may be present in the immunogenic compositions of the invention in an amount that is capable of eliciting an immune response when administered. Ideally, the acellular pertussis antigens can elicit a protective immune response. Quantities of acellular pertussis antigens are typically expressed in micrograms. The concentration of PT in a vaccine is usually between 5 and 50µg/ml. Typical PT concentrations are 5µg/ml, 16µg/ml, 20µg/ml or 50µg/ml, for example, about 20µg per dose or about 25µg per dose. The concentration of FHA in a vaccine is usually between 10 and 50µg/ml. Typical FHA concentrations are 10µg/ml, 16µg/ml or 50µg/ml, for example, about 20µg per dose or about 25µg per dose. The concentration of pertactin in a vaccine is usually between 5 and 16µg/ml. Typical pertactin concentrations are 5µg/ml, 6µg/ml or 16µg/ml, for example, about 3µg per dose or about 8µg per dose. FIM2 and FIM3 may be present at a concentration 5 and 16µg/ml. Typical concentrations of FIM2 and FIM3 are 5µg/ml, 10µg/ml or 20µg/ml, for example, for example, about 5µg per dose or about 10µg per dose. Booster vaccines for adolescents and adults typically contain 2.5 to 8 µg PT, between 4 and 8 µg FHA and between 2.5 and 8 µg pertactin per 0.5 ml dose. Typically, a booster vaccine comprises 4 µg PT, 4 µg FHA and 8 µg pertactin, more preferably 5 µg PT, 2.5 µg FHA and 2.5 µg pertactin, per 0.5 ml dose. A paediatric vaccine usually comprises 7 µg PT, 10 µg FHA and 10 µg pertactin, per 0.5 ml dose.

Where the aqueous component includes each of PT, FHA and pertactin, their weight ratios can vary, but may be *e.g.* about 16:16:5, about 5:10:6, about 20:20:3, about 25:25:8, or about 10:5:3 (PT:FHA:PRN).

### Diphtheria

*Corynebacterium diphtheriae* causes diphtheria. Diphtheria toxin can be treated (*e.g.* using formalin or formaldehyde) to remove toxicity while retaining the ability to induce specific anti-toxin antibodies after injection. The diphtheria toxoids used in diphtheria vaccines are well known in the art. Preferred diphtheria toxoids are those prepared by formaldehyde treatment. The diphtheria toxoid can be obtained by growing *C. diphtheriae* in growth medium (*e.g.* Fenton medium, or Linggoud & Fenton medium), which may be supplemented with bovine extract, followed by formaldehyde treatment, ultrafiltration and precipitation. Particularly the growth medium for growing *C. diphtheriae* is free from animal-derived components. The toxoided material may then be treated by a process comprising sterile filtration and/or dialysis. Alternatively, genetically detoxified diphtheria toxin (*e.g.,* CRM197) may be used which may be formaldehyde-treated to maintain long-term stability during storage.

The diphtheria toxoid may be adsorbed onto an adjuvant such as an aluminium salt adjuvant.

Quantities of diphtheria toxin and/or toxoid in a composition are generally measured in the 'Lf' unit ("flocculating units", or the "limes flocculating dose", or the "limit of flocculation"), defined as the amount of toxin/toxoid which, when mixed with one International Unit of antitoxin, produces an optimally flocculating mixture [16,17]. For example, the NIBSC supplies 'Diphtheria Toxoid, Plain' [18], which contains 300 LF per ampoule, and also supplies 'The 2nd International Reference Reagent For Diphtheria Toxoid For Flocculation Test' (NIBSC Code: 02/176) which contains 900 Lf per ampoule. The concentration of diphtheria toxin or toxoid in a composition can readily be determined using a flocculation assay by comparison with a reference material calibrated against such reference reagents.

The immunizing potency of diphtheria toxoid in a composition is generally expressed in international units (IU). The potency can be assessed by comparing the protection afforded by a composition in laboratory animals (typically guinea pigs) with a reference vaccine that has been calibrated in IUs. NIBSC supplies the '4th WHO International Standard for Diphtheria Toxoid (Adsorbed)' (NIBSC code: 07/216) which contains 213 IU per ampoule, and is suitable for calibrating such assays.

A three-dilution assay can be used to determine the potency of the compositions of the invention. After immunization, guinea-pigs are bled or challenged either by the subcutaneous or by the intradermal route. In an alternative embodiment, mice are used in place of guinea pigs. When guinea pigs or mice are bled, the antitoxin levels of the individual animals are titrated by means of toxin neutralization tests performed using *in vivo* or *in vitro* serological methods that have been validated on vaccines of the types being tested. In one embodiment, diphtheria toxoids produced in fermentation medium comprising animal-derived components are used for validation. The potency of the composition of the invention is calculated using appropriate statistical methods. For three-dilution assays, the limits of the 95% confidence intervals of the estimate of potency is within 50-200% of the estimated potency unless the lower limit of the 95% confidence interval of the estimated potency is greater than 30 IU per single human dose. When one-dilution tests are performed, the potency of the test vaccine is demonstrated to be significantly greater than 30 IU per human dose.

By IU measurements, compositions generally include at least 30 IU/dose. Compositions typically include between 20 and 80 Lf/ml of diphtheria toxoid, typically about 50 Lf/ml. Booster vaccines for adolescents and adults typically contain between 4 Lf/ml and 8 Lf/ml of diphtheria toxoid, e.g., 2.5 Lf, preferably 4 Lf, per 0.5 ml dose. Paediatric vaccines typically contain between 20 and 50 Lf/ml of diphtheria toxoid, e.g. 10 Lf or 25 Lf per 0.5 ml dose.

Purity of a protein preparation can be expressed by the ratio of specific protein to total protein. The purity of diphtheria toxoid in a composition is generally expressed in units of Lf diphtheria toxoid per unit mass of protein (nondialysable) nitrogen. For instance, a very pure toxin/toxoid might have a purity of more than 1700 Lf/mg N, indicating that most or all of the protein in the composition is diphtheria toxin/toxoid [19].

### Tetanus

*Clostridium tetani* causes tetanus. Tetanus toxin can be treated to give a protective toxoid. The toxoids are used in tetanus vaccines and are well known in the art. Thus, a combination vaccine of the invention can include a tetanus toxoid. Preferred tetanus toxoids are those prepared by formaldehyde treatment. The tetanus toxoid can be obtained by growing *C.tetani* in growth medium (*e.g.* a Latham medium derived from bovine casein), followed by formaldehyde treatment, ultrafiltration and precipitation. The growth medium for growing *C.tetani* may be free from animal-derived components. The material may then be treated by a process comprising sterile filtration and/or dialysis.

The tetanus toxoid may be adsorbed onto an adjuvant, for example, an aluminium salt adjuvant.

Quantities of tetanus toxoid can be expressed in 'Lf' units (see below), defined as the amount of toxoid which, when mixed with one International Unit of antitoxin, produces an optimally flocculating mixture [16]. The NIBSC supplies 'The 2nd International Reference Reagent for Tetanus Toxoid For Flocculation Test' (NIBSC Code: 04/150) which contains 690 LF per ampoule, by which measurements can be calibrated.

Booster vaccines for adolescents and adults typically contain 5 Lf of tetanus toxoid per 0.5 ml dose. Paediatric vaccines typically contain between 5 and 10 Lf of tetanus toxoid per 0.5 ml dose.

The immunizing potency of tetanus toxoid is measured in international units (IU), assessed by comparing the protection afforded by a composition in laboratory animals (typically guinea pigs) with a reference vaccine e.g. using NIBSC's 'Tetanus Toxoid Adsorbed Third International Standard 2000' [20,21], which contains 469 IU per ampoule. The potency of tetanus toxoid in a composition of the invention should be at least 35 IU per dose *e.g.* at least 70 IU/ml. More particularly, the potency of tetanus toxoid in a composition of the invention is at least 40 IU per dose. However, in booster vaccines for adults and adolescents, a reduced potency of 20 IU/dose may be acceptable because of the reduced antigen content in comparison to paediatric vaccine intended for primary immunization.

A multiple dilution assay can be used to determine the potency of the compositions of the invention. After immunization, guinea-pigs are bled or challenged either by the subcutaneous or by the intradermal route. As an alternative, mice may be used in place of guinea pigs. When guinea pigs or mice are bled, the antitoxin levels of the individual animals are titrated by means of toxin neutralization tests performed using *in vivo* or *in vitro* serological methods that have been validated on vaccines of the types being tested. The potency of the composition of the invention is calculated using appropriate statistical methods. Where multiple dilution assays are used, the lower and upper limits of 95% confidence interval should be within 50-200% of the estimated potency respectively. The lower 95% confidence limit of the estimated potency of a tetanus vaccine used for the primary immunization of children should not be less than 40 IU per single human dose.

The purity of tetanus toxoid in a composition is generally expressed in units of Lf tetanus toxoid per unit mass of protein (non-dialyzable) nitrogen. The tetanus toxoid should have a purity of at least 1000 Lf/mg N.

### Hib

*Haemophilus influenzae* type b ('Hib') causes bacterial meningitis. Hib vaccines are typically based on the capsular saccharide antigen the preparation of which is well documented in the art. The *H.influenzae* bacteria can be cultured in the absence of animal-derived components. The Hib saccharide is conjugated to a carrier protein in order to enhance its immunogenicity, especially in children. Typical carrier proteins in these conjugates are tetanus toxoid, diphtheria toxoid, the CRM197 derivative of diphtheria toxin, or an outer membrane protein complex (OMPC) from serogroup B meningococcus. Thus, a combination vaccine of the invention can include a Hib capsular saccharide conjugated to a carrier protein.

Any suitable activation chemistry and/or linker chemistry can be used in the conjugation of Hib saccharides. The saccharide will typically be activated or functionalised prior to conjugation. Activation may involve, for example, cyanylating reagents such as CDAP (*e.g.* 1-cyano-4-dimethylamino pyridinium tetrafluoroborate [30, 31]). Other suitable techniques use carbodiimides, hydrazides, active esters, norborane, p-nitrobenzoic acid, N-hydroxysuccinimide, S-NHS, EDC, TSTU; see also the introduction to reference 32).

Linkages via a linker group may be made using any known procedure, for example, the procedures described in references 33 and 34. One type of linkage involves reductive amination of the polysaccharide, coupling the resulting amino group with one end of an adipic acid linker group, and then coupling a protein to the other end of the adipic acid linker group [35, 36, 37]. Other linkers include B-propionamido [38], nitrophenyl-ethylamine [39], haloacyl halides [40], glycosidic linkages [41, 42, 43], 6-aminocaproic acid [44], ADH [45], C₄ to C₁₂ moieties [46] *etc.* As an alternative to using a linker, direct linkage can be used. Direct linkages to the protein may comprise oxidation of the polysaccharide followed by reductive amination with the protein, as described in, for example, references 42 and 47.

Tetanus toxoid may be used in the conjugate commonly referred to as 'PRP-T'. PRP-T can be made by activating a Hib capsular polysaccharide using cyanogen bromide, coupling the activated saccharide to an adipic acid linker (such as (1-ethyl-3-(3-dimethylaminopropyl) carbodiimide), typically the hydrochloride salt), and then reacting the linker-saccharide entity with a tetanus toxoid carrier protein.

The CRM197 diphtheria toxoid is another preferred Hib carrier protein [48,49,50]. A preferred conjugate comprises the Hib saccharide covalently linked to CRM197 via adipic acid succinic diester [51,52].

The saccharide moiety of the conjugate may comprise full-length polyribosylribitol phosphate (PRP) as prepared from Hib bacteria, and/or fragments of full-length PRP. Conjugates with a saccharide:protein ratio (w/w) of between 1:5 (*i.e.* excess protein) and 5:1 (*i.e.* excess saccharide) may be used *e.g.* ratios between 1:2 and 5:1 and ratios between 1:1.25 and 1:2.5. In preferred vaccines, however, the weight ratio of saccharide to carrier protein is between 1:2.5 and 1:3.5. In vaccines where tetanus toxoid is present both as an antigen and as a carrier protein then the weight ratio of saccharide to carrier protein in the conjugate may be between 1:0.3 and 1:2 [53].

Quantities of Hib antigens are typically expressed in µg of saccharide. The concentration of saccharide in a vaccine is typically between from 3 to 30µg/ml *e.g.* 20µg/ml. Administration of the Hib conjugate preferably results in an anti-PRP antibody concentration of ≥0.15µg/ml, and more preferably ≥1µg/ml, and these are the standard response thresholds. In some embodiments of the invention, the Hib polyribosylribitol phosphate is synthetic, for example, as described in [54 or 55].

### Hepatitis B virus

Hepatitis B virus (HBV) is a cause of viral hepatitis. The HBV virion consists of an inner core surrounded by an outer protein coat or capsid, and the core contains the viral DNA genome. The major component of the capsid is a protein known as HBV surface antigen or, more commonly, 'HBsAg', which is typically a 226-amino acid polypeptide with a molecular weight of -24 kDa. All existing hepatitis B vaccines contain HBsAg, and when this antigen is administered to a normal patient, it stimulates the production of anti-HBsAg antibodies which protect against HBV infection. Thus, immunogenic compositions of the invention can include HBsAg.

For vaccine manufacture, HBsAg can be made in a number of ways. For example, by expressing the protein by recombinant DNA methods. HBsAg for use with the method of the invention should be recombinantly expressed, *e.g.* in yeast cells. Suitable yeasts include *Saccharomyces* (such as *S.cerevisiae*), *Hanensula* (such as *H.polymorpha*) or *Pichia* hosts. The yeasts can be cultured in the absence of animal-derived components. Yeast-expressed HBsAg is generally non-glycosylated, and this is the most preferred form of HBsAg for use in immunogenic compositions of the invention. Yeast-expressed HBsAg is highly immunogenic and can be prepared without the risk of blood product contamination. Many methods for purifying HBsAg from recombinant yeast are known in the art.

The HBsAg will generally be in the form of substantially-spherical particles (average diameter of about 20nm), including a lipid matrix comprising phospholipids. Yeast-expressed HBsAg particles may include phosphatidylinositol, which is not found in natural HBV virions. The particles may also include a non-toxic amount of LPS in order to stimulate the immune system [56]. The particles may retain non-ionic surfactant (*e.g.* polysorbate 20) if this was used during disruption of yeast [57].

The HBsAg is preferably from HBV subtype adw2.

A preferred method for HBsAg purification involves, after cell disruption: ultrafiltration; size exclusion chromatography; anion exchange chromatography; ultracentrifugation; desalting; and sterile filtration. Lysates may be precipitated after cell disruption (*e.g.* using a polyethylene glycol), leaving HBsAg in solution, ready for ultrafiltration.

After purification HBsAg may be subjected to dialysis (*e.g.* with cysteine), which can be used to remove any mercurial preservatives such as thimerosal that may have been used during HBsAg preparation [58].

Quantities of HBsAg are typically expressed in micrograms. Combination vaccines containing HBsAg usually include between 5 and 60 µg/ml. The concentration of HBsAg in a composition of the invention is preferably less than 60 µg/ml *e.g.* ≤55 µg/ml, ≤50 µg/ml, ≤45 µg/ml, ≤40 µg/ml, *etc.* A concentration of about 20 µg/ml is typical e.g. 10µg per dose. In some embodiments of the invention, a composition includes a 'low dose' of HBsAg. This means that the concentration of HBsAg in the composition is ≤5 µg/ml e.g. <4, <3, <2.5, <2, <1, *etc.* In a typical 0.5ml unit dose volume, therefore, the amount of HBsAg is less than 25µg *e.g.* <2, <1.5, <1, <0.5, *etc.*

### Poliovirus

Poliovirus causes poliomyelitis. Inactivated polio virus vaccine (IPV), as disclosed in more detail in chapter 24 of reference **Error! Bookmark not defined.,** has been known for many years. Thus, a combination vaccine of the invention can include an inactivated poliovirus antigen.

Polioviruses may be grown in cell culture, and a preferred culture uses a Vero cell line, derived from monkey kidney. Vero cells can conveniently be cultured microcarriers. After growth, virions may be purified using techniques such as ultrafiltration, diafiltration, and chromatography. Where animal (and particularly bovine) materials are used in the culture of cells, they should be obtained from sources that are free from transmissible spongiform encephalopathies (TSEs), and in particular free from bovine spongiform encephalopathy (BSE). Preferably, polioviruses are grown in cells cultured in medium free of animal-derived components.

Prior to administration to patients, polioviruses must be inactivated, and this can be achieved by treatment with formaldehyde (or, preferably, a non-aldehyde agent). Poliomyelitis can be caused by one of three types of poliovirus. The three types are similar and cause identical symptoms, but they are antigenically very different and infection by one type does not protect against infection by others. It is therefore preferred to use three poliovirus antigens with the invention: poliovirus Type 1 (*e.g.* Mahoney strain), poliovirus Type 2 (*e.g.* MEF-1 strain), and poliovirus Type 3 (*e.g.* Saukett strain). Other strains of poliovirus Type 1, Type 2 and Type 3 are known in the art and may also be used. The viruses are preferably grown, purified and inactivated individually, and are then combined to give a bulk trivalent mixture for use with the invention.

Quantities of IPV are typically expressed in the 'DU' unit (the "D-antigen unit" [59]). Combination vaccine usually comprise between 1-100 DU per polioviral type per dose e.g., about 40 DU of type 1 poliovirus, about 8 DU of type 2 poliovirus, and about 32 DU of type 3 poliovirus, but it is possible to use lower doses than these [60,61] *e.g.* 10-20 DU for type 1, 2-4 DU for type 2, and 8-20 DU for type 3. A combination vaccine of the invention can include a 'low dose' of a poliovirus. For a Type 1 poliovirus this means that the concentration of the virus in the composition is ≤20 DU/ml e.g. <18, <16, <14, <12, <10, *etc.* For a Type 2 poliovirus this means that the concentration of the virus in the composition is ≤4 DU/ml e.g. <3, <2, <1, <0.5, *etc.* For a Type 3 poliovirus this means that the concentration of the virus in the composition is ≤16 DU/ml *e.g.* <14, <12, <10, <8, <6, *etc.* Where all three of Types 1, 2 and 3 polio virus are present the three antigens can be present at a DU ratio of 5:1:4 respectively, or at any other suitable ratio *e.g.* a ratio of 15:32:45 when using Sabin strains [62]. A low dose of antigen from Sabin strains is particularly useful, with ≤10 DU type 1, ≤20 DU type 2, and ≤30 DU type 3 (per unit dose, typically 0.5 ml).

Where an IPV component is used, and the polioviruses were grown on Vero cells, a vaccine composition preferably contains less than 10ng/ml, preferably ≤1ng/ml *e.g.* ≤500pg/ml or ≤50 pg/ml of Vero cell DNA *e.g*. less than 10ng/ml of Vero cell DNA that is ≥50 base pairs long.

### Preparing a combination vaccine

Antigenic components from these pathogens for use in vaccines are commonly referred to by abbreviated names: 'D' for diphtheria toxoid; 'T' for tetanus toxoid; 'P' for pertussis antigens, with 'aP' being acellular (*e.g.* including at least OMVs of the invention, PT and FHA and optionally pertactin and/or FIM2/FIM3); HBsAg for hepatitis B surface antigen; 'Hib' for conjugated *H.influenzae* b capsular saccharide; and 'IPV' for 3-valent inactivated poliovirus.

Embodiments of the invention include, but are not limited to combination vaccines comprising the following components:
- D, T, aP
- D, T, aP, IPV
- D, T, aP, HBsAg
- D, T, aP, Hib
- D, T, aP, Hib, IPV
- D, T, aP, HBsAg, Hib
- D, T, aP, HBsAg, IPV
- D, T, aP, HBsAg, IPV, Hib

These combination vaccines may consist essentially of only the antigens listed, or may further include antigens from additional pathogens. Particularly, these combination vaccines contain only the antigens listed as active ingredients but may further comprise excipients such as adjuvants, buffers and the like. In some embodiments the aP component consists of OMVs of the invention, PT (preferably genetically detoxified PT) and FHA. In some embodiments the aP component consists of OMVs of the invention, PT (preferably genetically detoxified PT), FHA and PRN. In some embodiments the aP component consists of OMVs of the invention, PT (preferably genetically detoxified PT), FHA and FIM2/FIM3. In some embodiments the aP component consists of OMVs of the invention, PT (preferably genetically detoxified PT), FHA, PRN and FIM2/FIM3.

For paediatric combination vaccines, the ratio of D:T is typically greater than 1 *(i.e.* paediatric vaccines usually have excess D in Lf units) and generally between 2:1 and 3:1 (measured in Lf units), *e.g.* 2.5:1. In contrast, for booster vaccine that are administered to adolescents or adults (who usually have received at least one paediatric combination vaccine comprising D and T), the ratio of T:D is typically greater than 1 (*i.e.* booster vaccines usually have excess T in Lf units) and generally between 1.5:1 and 2.5:1, *e.g.* 2:1.

One useful vaccine includes OMVs of the invention and, per unit dose, 2Lf D, 5Lf T, 4µg PT-9K/129G, 4µg FHA and 8µg pertactin. Another useful vaccine includes OMVs of the invention and, per unit dose, 25Lf D, 10Lf T, 25µg PT-9K/129G, 25µg FHA and 8µg pertactin.

When combining antigenic components to prepare a multivalent composition, the antigens can be added individually, or they can be pre-mixed. Where a combination vaccine comprises D and T antigens and additional antigens, a pre-mixed D-T component can be used in the preparation of the combination vaccine. This bivalent component can be combined with further antigens. Where a D-T mixture is used for preparing a combination vaccine, the ratio of diphtheria toxoid to tetanus toxoid in the mixture can be between 2:1 and 3:1 (measured in Lf units), preferably between 2.4:1 and 2.6:1, *e.g.* preferably 2.5:1.

### Methods of treatment

In a fourth aspect of the invention, the OMVs or immunogenic composition may be used to induce an immune response in a mammal, particularly a suitable mammal. The suitable mammal is preferably a human; the immune response is preferably protective and preferably involves antibodies.

Immunogenic and pharmaceutical compositions of the invention are for *in vivo* use for eliciting an immune response against an immunogen of interest. As disclosed herein, methods for raising an immune response in a mammal comprising the step of administering an effective amount of an immunogenic composition or pharmaceutical composition of the invention are provided. The immune response is preferably protective and preferably involves antibodies and/or cell mediated immunity. The method may raise a booster response.

The invention also provides an immunogenic composition or pharmaceutical composition of the invention for use in a method for raising an immune response in a mammal.

The invention also provides the use of an OMV or population of OMVs of the invention in the manufacture of a medicament for raising an immune response in a mammal.

By raising an immune response in the mammal by these uses and methods, the mammal can be protected against various diseases and/or infections e.g. against bacterial and/or viral diseases as discussed above. The OMVs and compositions are immunogenic, and are more preferably vaccine compositions. Vaccines according to the invention may either be prophylactic (i.e. to prevent infection) or therapeutic (i.e. to treat infection), but will typically be prophylactic.

The mammal, particularly a suitable mammal may be a human or a large veterinary mammal (e.g. horses, cattle, deer, goats, and pigs). Where the vaccine is for prophylactic use, the human is preferably a child (e.g. a toddler or infant) or a teenager; where the vaccine is for therapeutic use, the human is preferably a teenager or an adult. A vaccine intended for children may also be administered to adults e.g. to assess safety, dosage, immunogenicity, etc.

Vaccines prepared according to the invention may be used to treat both children and adults. Thus, a human patient may be less than 1 year old, less than 5 years old, 1-5 years old, 5-15 years old, 15-55 years old, or at least 55 years old. In some embodiments, the child is an individual under one year of age, for example, less than one day old, about 1 week old, about 2 weeks old, about 3 weeks old, about 4 weeks old, about 2 months old, about 3 months, about 4 months, about 5 months, about 6 months, about 7 months, about 8 months old, about 9 months old, about 10 months old, about 11 months old, less than about 12 months old).

Particular patient groups for receiving the vaccines are the elderly (e.g. 20 ∼50 years old, ∼60 years old, and preferably ∼65 years), the young (e.g. ∼5 years old or infants less than about 12 months old), hospitalised patients, healthcare workers, armed service and military personnel, pregnant women, the chronically ill, or immunodeficient patients. The vaccines are not suitable solely for these groups, however, and may be used more generally in a population.

Compositions of the invention will generally be administered directly to a patient. Direct delivery may be accomplished by parenteral injection (e.g. subcutaneously, intraperitoneally, intravenously, intramuscularly, intradermally, or to the interstitial space of a tissue. Alternative delivery routes include rectal, oral (e.g. tablet, spray), buccal, sublingual, vaginal, topical, transdermal or transcutaneous, intranasal, ocular, aural, pulmonary or other mucosal administration. Intradermal and intramuscular administration are two preferred routes. Injection may be via a needle (e.g. a hypodermic needle), but needle-free injection may alternatively be used. A typical intramuscular dose is 0.5 mL.

The invention may be used to elicit systemic and/or mucosal immunity, preferably to elicit an enhanced systemic and/or mucosal immunity.

These uses and methods are preferably for the prevention and/or treatment of a disease caused by *Bordetella pertussis* and optionally one or more of *Corynebacterium diphtheriae, Clostridium tetani, haemophilus influenzae* serotype b, polio virus and hepatitis B virus.

The subject in which disease is prevented may not be the same as the subject that receives the immunogenic composition of the invention. For instance, an immunogenic composition may be administered to a female (before or during pregnancy) in order to protect offspring (so-called 'maternal immunization'. The immunization of the pregnant female provides antibody-mediated immunity to the infant through passive maternal immunity. The passive immunity occurs naturally when maternal antibodies are transferred to the foetus through the placenta. Passive immunity is especially important to infants because they are born without any actively acquired immunity. Administration of compositions of the invention to a pregnant female enhances immunity in the female, and antibodies are passed to the new-born through the placenta, conferring passive maternal immunity on the infant. However, passive immunity in infants is only temporary and starts to decrease after the first few weeks, or months of life. As passive immunity is only temporary, it may be important for the infant to receive administration of a composition of the invention, to induce active immunity in the infant, before the passive immunity diminishes. Administration of a second immunogenic composition to the infant after birth induces active immunity in the infant, and extends the immunity passed on from the mother during pregnancy.

Dosage can be by a single dose schedule or a multiple dose schedule. Multiple doses may be used in a primary immunisation schedule and/or in a booster immunisation schedule. In a multiple dose schedule the various doses may be given by the same or different routes e.g. a parenteral prime and mucosal boost, a mucosal prime and parenteral boost, etc. Multiple doses will typically be administered at least 1 week apart (e.g. about 2 weeks, about 3 weeks, about 4 weeks, about 6 weeks, about 8 weeks, about 10 weeks, about 12 weeks, about 16 weeks, etc.). In one embodiment, multiple doses may be administered approximately 6 weeks, 10 weeks and 14 weeks after birth, e.g. at an age of 6 weeks, 10 weeks and 14 weeks, as often used in the World Health Organisation's Expanded Program on Immunisation ("EPI"). In an alternative embodiment, two primary doses are administered about two months apart, e.g. about 7, 8 or 9 weeks apart, followed by one or more booster doses about 6 months to 1 year after the second primary dose, e.g. about 6, 8, 10 or 12 months after the second primary dose. In a further embodiment, three primary doses are administered about two months apart, e.g. about 7, 8 or 9 weeks apart, followed by one or more booster doses about 6 months to 1 year after the third primary dose, e.g. about 6, 8, 10, or 12 months after the third primary dose.

### Methods to modulate reactogenicity of lipid A

In a fifth aspect, the invention relates to a method of modulating the reactogenicity of the Lipid A of a Bordetella pertussis bacterium, comprising stably integrating into the genome of the bacterium at least one gene selected from the group consisting of LpxA and LpxD, wherein:
(i) the LpxA gene encodes an LpxA protein having at least 80% sequence identity with SEQ ID NO: 1 or 2 and wherein the protein comprises Serine at position 170 (S170) and/or Alanine at position 229 (A229) when numbered in accordance with SEQ ID NO: 1 or 2; and/or
(ii) the LpxD gene encodes an LpxD protein having at least 90% amino acid sequence identity with SEQ ID NO:3 or SEQ ID NO: 4. As described above, the reactogenicity of lipid A of a gram-negative bacterium, particularly a *B.pertussis* bacterium, can be modulated by expression of exogenous enzymes from the lipid A biosynthetic pathway, for example acetyltransferases LpxA and LpxD.

Genomic integration of the LpxA and LpxD genes allows for the stable expression of the exogenous DNA without the need for antibiotic selection. In addition, surprisingly the Inventors discovered that such strains do not suffer growth defects seen in the art.

### Definitions

As used in the present disclosure and claims, the singular forms "a," "an," and "the" include plural forms unless the context clearly dictates otherwise; i.e., "a" means "one or more" unless indicated otherwise.

The term "and/or" as used in a phrase such as "A and/or B" is intended to include "A and B," "A or B," "A," and "B." Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

The term "comprising" encompasses "including" e.g. a composition "comprising" X may include something additional e.g. X + Y. The word "substantially" does not exclude "completely" e.g. a composition which is "substantially free" from Y may be completely free from Y. In some implementations, the term "comprising" refers to the inclusion of the indicated active agent, such as recited polypeptides, as well as inclusion of other active agents, and pharmaceutically acceptable carriers, excipients, emollients, stabilizers, etc., as are known in the pharmaceutical industry. In some implementations, the term "consisting essentially of" refers to a composition, whose only active ingredient is the indicated active ingredient(s), for example antigens, however, other compounds may be included which are for stabilizing, preserving, etc. the formulation, but are not involved directly in the therapeutic effect of the indicated active ingredient. Use of the transitional phrase "consisting essentially" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461,463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means "limited to" unless expressly specified otherwise. In certain territories, the term "comprising an active ingredient consisting of" may be used in place of "consisting essentially".
The term "about" in relation to a numerical value x means within an acceptable contextual error range for the particular value as determined by one of ordinary skill in the art, which will depend in part on how the value is measured or determined, i.e. the limitations of the measurement system or the degree of precision required for a particular purpose, for example, x±10%, x±5%, x±4%, x±3%, x±2%, x±1%.
The word "substantially" does not exclude "completely" e.g. a composition which is "substantially free" from Y may be completely free from Y but may encompass conditions that function in all important aspects as free conditions but where the numerical values indicate the presence of some impurities or substances. Where necessary, the word "substantially" may be omitted from the definition of the invention. Where particular values are used in the specification and in the claims, unless otherwise stated, the term "substantially" means with an acceptable error range for the particular value.

Where methods refer to steps of administration, for example as (a), (b), (c), etc., these are intended to be sequential, i.e., step (c) follows step (b) which is preceded by step (a). Antibodies will generally be specific for their target, i.e., they will have a higher affinity for the target than for an irrelevant control protein, such as bovine serum albumin.

All references or patent applications cited within this patent specification are incorporated by reference herein.

### SPECIFIC EMBODIMENTS OF THE INVENTION

Embodiment 1. A recombinant *Bordetella pertussis* bacterium which comprises:
   (i) at least one genomic LpxA gene encoding an LpxA protein, wherein the LpxA protein comprises a mutation at position 170 and/or a mutation at position 229 relative to SEQ ID NO: 1; and/or
   (ii) at least one genomic insertion of a heterologous LpxD gene.
Embodiment 2. The recombinant *Bordetella pertussis* bacterium of Embodiment 1 which comprises at least one genomic LpxA gene encoding an LpxA protein, wherein the LpxA protein comprises a substitution at position 170 and/or a substitution at position 229 relative to SEQ ID NO: 1.
Embodiment 3. The recombinant *Bordetella pertussis* bacterium of Embodiment 1 or 2 which comprises a Serine residue at position 170 and/or an Alanine residue at position 229 relative to SEQ ID NO: 1.
Embodiment 4. The recombinant *Bordetella pertussis* bacterium of Embodiment 3, which comprises (i) a Serine residue at position 170 and (ii) an Alanine residue at position 229 when numbered in accordance with SEQ ID NO:1.
Embodiment 5. The recombinant *Bordetella pertussis* bacterium of Embodiment 4 which comprises an LpxA gene that encodes an LpxA protein having at least 80% amino acid sequence identity to SEQ ID NO: 1 or 2.
Embodiment 6. The recombinant *Bordetella pertussis* bacterium of any preceding Embodiment, wherein the heterologous LpxD gene encodes an LpxD protein having at least 90% amino acid sequence identity with SEQ ID NO:3 or SEQ ID NO: 4.
Embodiment 7. The recombinant *Bordetella pertussis* bacterium of Embodiment 6, wherein the heterologous LpxD gene encodes an LpxD protein having at least 95% amino acid sequence identity with SEQ ID NO:3 or SEQ ID NO: 4.
Embodiment 8. The recombinant *Bordetella pertussis* bacterium of Embodiment 7, wherein the heterologous LpxD gene encodes an LpxD protein having an amino acid sequence selected from the group consisting of SEQ ID NO:3 or SEQ ID NO: 4.
Embodiment 9. The recombinant *Bordetella pertussis* bacterium of any preceding Embodiment, wherein the endogenous LpxA gene is inactivated and/or the endogenous LpxD gene is inactivated.
Embodiment 10. The recombinant *Bordetella pertussis* bacterium of any one of the preceding Embodiments which produces lipid A wherein (i) at least 30% of the C3' acyl chains are from about C10 to about C12 in length; and/or (ii) at least 30% of the C2' acyl chains are from about C10 to about C12 in length; and/or (iii) at least 30% of the C2 acyl chains are from about C10 to about C12 in length.
Embodiment 11. The recombinant *Bordetella pertussis* bacterium of any one of the preceding Embodiments that produces Lipid A with reduced endotoxic activity compared to that of the Lipid A produced by the parental strain, particularly when measured using TLR4 stimulation assays, particularly human TLR4 stimulation assays.
Embodiment 12. The recombinant *Bordetella pertussis* bacterium of any one of the preceding Embodiments, wherein the growth curve of a bacterial population of said bacteria is comparable to the growth curve of a population of the parental strain.
Embodiment 13. An isolated outer membrane vesicle (OMV) derived from the recombinant Bordetella pertussis bacterium of any one of Embodiments 1 to 12 which comprises modified Lipid A incorporated into the membrane wherein substantially all of C3' acyl chains of the Lipid A have a length of C10 and/or wherein substantially all of C2 and C2' acyl chains of the Lipid A have a length of C12.
Embodiment 14. The isolated outer membrane vesicle according to Embodiment 13 which is substantially free of dermonecrotic toxin and/or which contains endogenous genetically detoxified pertussis toxoid.
Embodiment 15. An immunogenic composition comprising at least one isolated OMV according to Embodiment 13 or 14 and a pharmaceutically acceptable excipient.
Embodiment 16. The immunogenic composition of Embodiment 15, further comprising at least one additional antigen selected from the group consisting of (1) pertussis toxoid (PT), (2) FHA, (3) pertactin (PRN), (4) FIM2/FIM3, (5) adenylate cyclase, (6) diphtheria toxoid (DT), (7) tetanus toxoid (TT), (8) inactivated polio virus (IPV), (9) hepatitis B surface antigen and (10) Hib PRP.
Embodiment 17. The OMV of Embodiment 13 or 14 or the immunogenic composition of Embodiment 15 or 16 for use in inducing an immune response in a suitable mammal, for example a human.
Embodiment 18. The OMV of Embodiment 13 or 14 or the immunogenic composition of Embodiment 15 or 16 for use in prophylaxis or as a vaccine.
Embodiment 19. A method of modulating the reactogenicity of the Lipid A of a *Bordetella pertussis* bacterium, comprising stably integrating into the genome of the bacterium at least one gene selected from the group consisting of LpxA and LpxD, wherein:
   (i) the LpxA gene encodes an LpxA protein having at least 80% sequence identity with SEQ ID NO: 1 or 2 and wherein the protein comprises Serine at position 170 (S170) and/or Alanine at position 229 (A229) when numbered in accordance with SEQ ID NO: 1 or 2; and/or
   (ii) the LpxD gene encodes an LpxD protein having at least 90% amino acid sequence identity with SEQ ID NO:3 or SEQ ID NO: 4.
Embodiment 20. A recombinant *Bordetella pertussis* bacterium which comprises at least one genomic LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2.
Embodiment 21. A recombinant *Bordetella pertussis* bacterium which comprises at least one episomal LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2.
Embodiment 23. A recombinant *Bordetella pertussis* bacterium which comprises (i) at least one genomic LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and (ii) at least one genomic insertion of a heterologous LpxD gene wherein the heterologous LpxD gene encodes an amino acid sequence having at least 95% sequence identity with SEQ ID NO:3 or SEQ ID NO: 4.
Embodiment 24. A recombinant *Bordetella pertussis* bacterium which comprises at least one episomal LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and (ii) at least one genomic insertion of a heterologous LpxD gene wherein the heterologous LpxD gene encodes an amino acid sequence having at least 95% sequence identity with SEQ ID NO:3 or SEQ ID NO: 4.
Embodiment 25. A recombinant *Bordetella pertussis* bacterium which comprises at least one genomic insertion of a heterologous LpxD gene wherein the heterologous LpxD gene encodes an amino acid sequence having at least 95% sequence identity with SEQ ID NO:3 or SEQ ID NO:4.
Embodiment 26. A recombinant *Bordetella pertussis* bacterium which comprises at least one genomic LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and wherein the bacterium is derived from the Tohama strain and expresses the genetically detoxified pertussis toxoid PT-9K/129G.
Embodiment 27. A recombinant *Bordetella pertussis* bacterium which comprises at least one episomal LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and wherein the bacterium is derived from the Tohama strain and expresses the genetically detoxified pertussis toxoid PT-9K/129G.
Embodiment 28. A recombinant *Bordetella pertussis* bacterium which comprises (i) at least one genomic LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and (ii) at least one genomic insertion of a heterologous LpxD gene wherein the heterologous LpxD gene encodes an amino acid sequence having at least 95% sequence identity with SEQ ID NO:3 or SEQ ID NO: 4 and wherein the bacterium is derived from the Tohama strain and expresses the genetically detoxified pertussis toxoid PT-9K/129G.
Embodiment 29. A recombinant *Bordetella pertussis* bacterium which comprises at least one episomal LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and (ii) at least one genomic insertion of a heterologous LpxD gene wherein the heterologous LpxD gene encodes an amino acid sequence having at least 95% sequence identity with SEQ ID NO:3 or SEQ ID NO: 4 and wherein the bacterium is derived from the Tohama strain and expresses the genetically detoxified pertussis toxoid PT-9K/129G.
Embodiment 30. A recombinant *Bordetella pertussis* bacterium which comprises at least one genomic insertion of a heterologous LpxD gene wherein the heterologous LpxD gene encodes an amino acid sequence having at least 95% sequence identity with SEQ ID NO:3 or SEQ ID NO: 4 and wherein the bacterium is derived from the Tohama strain and expresses the genetically detoxified pertussis toxoid PT-9K/129G.
Embodiment 31. A recombinant *Bordetella pertussis* bacterium which comprises at least one genomic LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and wherein the bacterium is derived from the Tohama strain, expresses the genetically detoxified pertussis toxoid PT-9K/129G and does not express the dermonectrotic toxin (DNT) gene (SEQ ID NO: 19).
Embodiment 32. A recombinant *Bordetella pertussis* bacterium which comprises at least one episomal LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and wherein the bacterium is derived from the Tohama strain, expresses the genetically detoxified pertussis toxoid PT-9K/129G and does not express the dermonectrotic toxin (DNT) gene (SEQ ID NO: 19).
Embodiment 33. A recombinant *Bordetella pertussis* bacterium which comprises (i) at least one genomic LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and (ii) at least one genomic insertion of a heterologous LpxD gene wherein the heterologous LpxD gene encodes an amino acid sequence having at least 95% sequence identity with SEQ ID NO:3 or SEQ ID NO: 4 and wherein the bacterium is derived from the Tohama strain, expresses the genetically detoxified pertussis toxoid PT-9K/129G and does not express the dermonectrotic toxin (DNT) gene (SEQ ID NO: 19).
Embodiment 34. A recombinant *Bordetella pertussis* bacterium which comprises at least one episomal LpxA gene encoding an LpxA protein, wherein the LpxA protein comprises a Serine residue at position 170 and an Alanine residue at position 229 (relative to SEQ ID NO: 1) and wherein the LpxA gene encodes an amino acid sequence having at least 80% identity to SEQ ID NO: 1 or 2 and (ii) at least one genomic insertion of a heterologous LpxD gene wherein the heterologous LpxD gene encodes an amino acid sequence having at least 95% sequence identity with SEQ ID NO:3 or SEQ ID NO: 4 and wherein the bacterium is derived from the Tohama strain, expresses the genetically detoxified pertussis toxoid PT-9K/129G and does not express the dermonectrotic toxin (DNT) gene (SEQ ID NO: 19).
Embodiment 35. A recombinant *Bordetella pertussis* bacterium which comprises at least one genomic insertion of a heterologous LpxD gene wherein the heterologous LpxD gene encodes an amino acid sequence having at least 95% sequence identity with SEQ ID NO:3 or SEQ ID NO: 4 and wherein the bacterium is derived from the Tohama strain, expresses the genetically detoxified pertussis toxoid PT-9K/129G and does not express the dermonectrotic toxin (DNT) gene (SEQ ID NO: 19).
Embodiment 36. An isolated outer membrane vesicle (OMV) derived from the recombinant Bordetella pertussis bacterium of any one of Embodiments 20 to 35 which comprises modified Lipid A incorporated into the membrane wherein substantially all of C3' acyl chains of the Lipid A have a length of C10 and/or wherein substantially all of C2 and C2' acyl chains of the Lipid A have a length of C12.
Embodiment 37. An immunogenic composition, particularly a pharmaceutical composition such as a vaccine, comprising an immunologically effective amount of isolated OMVs according to Embodiment 36 and a pharmaceutically acceptable carrier.

### MODES FOR CARRYING OUT THE INVENTION

### Bacterial strains and gene sequences

Tables 1 to 3 list the key bacterial strains, DNA and amino acid sequences used:

| **Table 1. Bacterial strains and description** | |
|---|---|
| **Strain** | Description |
| **Tohama I PTg** | Wild type *B*. *pertussis* Tomaha I which expresses the genetically detoxified pertussis toxoid PT-9K/129G (parental strain) |
| Δ**LpxA_{Bpe}/LpxA_{Bpa}** | Tohama I PTg with genomic integration of LpxA_{Bpa} and knock-out of endogenous LpxA_{Bpe} genes |
| Δ**ArnT/**Δ**LpxA_{Bpe}/LpxA_{Bpa}** | Tohama I PTg with genomic integration of LpxA_{Bpa} and knock-out of endogenous LpxA_{Bpe} and ArnT |
| Δ**LpxA_{Bpe}/LpxA_{Bpa}/**Δ**DNT/LpxD_{Pa}** | Tohama I PTg with genomic integration of LpxA_{Bpa} and LpxD_{Pa} and knock-out endogenous LpxA_{Bpe} and DNT genes |
| **LpxA_{Bpa/}**Δ**LpxA_{Bpe}/**Δ**DNT/LpxD_{Pa}/**Δ**LpxD_{Bpe}** | Tohama I PTg with genomic integration of LpxA_{Bpa} and LpxD_{Pa} and knock-out LpxA_{Bpe}, LpxD_{Bpe} and DNT genes |
| Δ**ArnT/PagL_{Bbr}** | Tohama I PTg with genomic integration of PagL and knock-out of endogenous ArnT gene |
| **LpxD_{Pa}** | Tohama I PTg with episomal expression of LpxD from *P. aeruginosa* |
| **LpxA_{Bpa}** | Tohama I PTg with episomal expression of LpxA from *B. parapertussis* |

| **Table 2. Key amino acid sequences** | | |
|---|---|---|
| **Sequence ID** | **Amino acid sequence** | **Ref.: ID** |
| 1 | LpxA *Bordetella pertussis* | UniProt: Q7VYB8 |
| 2 | LpxA *Bordetella parapertussis* | GenBank: BBH97344 |
| 3 | LpxD *Comamonas testosteroni* | GenBank: WP 012839123 |
| 4 | LpxD *Pseudomonas aeruginosa* | GenBank: WP 003098585 |
| 5 | PagL *Bordetella bronchiseptica* | GenBank: CAE35745 |
| 6 | LpxE *Francisella novicida* | UniProt: Q66RM6 |
| 7 | *LpxE Rhizobium leguminosarum* | UniProt: A0A2K9Z9T7 |
| 8 | *LpxF Francisella novicida* | UniProt: A0Q4N6 |

| **Table 3. Key DNA sequences** | | |
|---|---|---|
| **Sequence ID** | **Nucleic acid sequence** | **GenBank ID** |
| 9 | LpxA *Bordetella parapertussis* | NC_018828.1:2648346-2649140 |
| 10 | LpxD *Comamonas testosteroni* | BBQP01000023.1:16517588 |
| 11 | LpxD *Pseudomonas aeruginosa* | LR590473.1:19088931909954 |
| 12 | LpxD *Comamonas testosteroni* (Codon optimised for expression in *B. pertussis*) | N/A |
| 13 | LpxD *Pseudomonas aeruginosa* (Codon optimised for expression in *B. pertussis*) | N/A |
| 14 | N-term insert for genomic integration into *B. pertussis* | N/A |
| 15 | C-term insert for genomic integration into *B*. *pertussis* | N/A |
| 16 | PagL *Bordetella bronchiseptica* | BX640448:164131-163595 |
| 17 | Porin promoter (BP0840 promoter) | BX640413.1:174494-174789 |
| 18 | ArnT *Bordetella pertussis* | |
| 19 | DNT *Bordetella pertussis* | |
| 20 | Construct for ArnT KO | N/A |
| 21 | Construct for replacing DNT with LpxD *P. aeruginosa* | N/A |

### Production and culture of recombinant bacterial strains

The heterologous Lpx genes were expressed in *Bordetella pertussis* either by genomic integration or by episomal (non-genomic) expression.

### Genomic integration

Synthetic sequences containing the sequence of interest (SEQ ID NO: 9) and upstream and downstream homology arms to allow for homologous recombination (SEQ ID NO: 14 and 15) were cloned into vector pSORTP1 [63] using flanking EcoRI and HindIII restriction sites. Vector pSORTP1 contains an origin of conjugative transfer, gentamycin and ampicillin selection markers and a copy of the rpsL gene that confers sensitivity to streptomycin.

The pSORTP1 vectors were transformed into *E. coli* SM10 *λpir* and recombinant clones were co-cultivated with a nalidixic acid/streptomycin-resistant *B. pertussis* strain Tohama I to allow conjugative transfer of the vector. The streptomycin resistance of this strain is conferred by a mutation in the rpsL gene. The plasmid cannot replicate in *B. pertussis* and needs to be integrated into the bacterial genome by homologous recombination in order to confer gentamycin resistance. Recombinant *B. pertussis* were obtained by concurrent selection on gentamycin (to remove wild-type *B. pertussis*) and nalidixic acid (to remove E. coli SM10 donor strain). Genomic introduction of the pSORTP1 vector containing a functional copy of the rpsL gene sensitises *B. pertussis* to streptomycin. Selection on streptomycin forces removal of the vector backbone by a second homologous recombination event on the other side of the transgene to achieve a markerless mutation. Using this approach, we obtained a recombinant *B. pertussis* Tohama I strain expressing the LpxA sequence from *B. parapertussis.* A number of additional strains were constructed as follows:
In strain ΔArnT/PagL_{Bbr} the ArnT locus in the *B. pertussis* genome was replaced with a copy of the *B. bronchiseptica* PagL gene under the control of the promoter region *of B. pertussis* BP0840 (outer membrane porin). A recombinant sequence was constructed with ArnT upstream and downstream regions containing the *B. bronchiseptica* PagL sequence. The recombinant sequence was cloned into vector pSORTP1 using flanking EcoRI and HindIII restriction sites. Following transformation, as described above, the resulting strain combines the knock-out of the ArnT gene and the expression of the PagL gene.

In strain ΔArnT/ΔLpxA_{Bpe}/LpxA_{Bpa} the ArnT locus (SEQ ID NO: 20) in the *B. pertussis* genome is knocked-out.

In strain ΔLpxA_{Bpe}/LpXA_{Bpa}/ΔDNT/LpXD_{Pa}/ΔLpXD_{Bpe}, a copy of LpxD_{Pa} was integrated into the genome (SEQ ID NO: 21), replacing the dermonecrotic toxin (DNT) gene (SEQ ID NO: 19). The wild-type LpxD_{Bp} was subsequently knocked out.

### Episomal expression

Synthetic sequences were cloned into vector pMMB67EH (ATCC Ref.: 37622) using flanking Acc65l and Hindlll sites. Sequences encoding LpxA_{Bpa}, LpxD_{Ct} and LpxD_{Pa} were codon optimized for expression in *B. pertussis* (PriorityGENE service, GENEWIZ).

The pMMB67EH vectors containing the heterologous LpxD genes were transformed into *E*. *coli* SM10 *λpir* and recombinant clones were co-cultivated with a nalidixic acid/streptomycin-resistant *B. pertussis* strain Tohama I PTg to allow conjugative transfer of the vector. Recombinant *B. pertussis* containing the pMMB67EH plasmid was then selected on ampicillin and nalidixic acid. Stable transformation of *B. pertussis* Tohama I PTg with the respective pMMB67EH constructs was confirmed by PCR detection of the heterologous Lpx sequence.

The *B. pertussis* recombinant strains were grown in commercially available media (see 64) in standard conditions; the medium was supplemented with 1 mM IPTG for episomal expression.

### Growth evaluation of recombinant B. pertussis ΔLpxA_{Bpe}/LpxA_{Bpa}

In order to assess any potential impact on bacterial growth, recombinant strain ΔLpxA_{Bpe}/LpxA_{Bpa} was tested in a 10L fermentation.

Briefly, two full Bordet-Gengou agar plates were used to inoculate two 30mL, 24h pre-cultures. The 30 mL pre-cultures were then inoculated into two additional 24h pre-cultures of 1L; these two second pre-cultures (approx. 1.5L) were then used to inoculate the 10L fermenter at OD₆₅₀ₙₘ of 1.

A drop in the concentration of dissolved oxygen (DO) was observed at approximately 9 hours; this drop is associated with an increase in the oxygen consumption due to the exponential growth rate of the culture. The DO was then controlled by agitation speed.

The end of the fermentation process after 37.5 hours was characterized by a decrease of the stirring speed caused by a diminution of oxygen consumption, evidence of the depletion of the main carbon source (Na-L-glutamate). The optical density (OD₆₅₀ₙₘ) at the time of harvest was 10.1, with a cell viability of 6.3 10¹⁰ (CFU/ml).

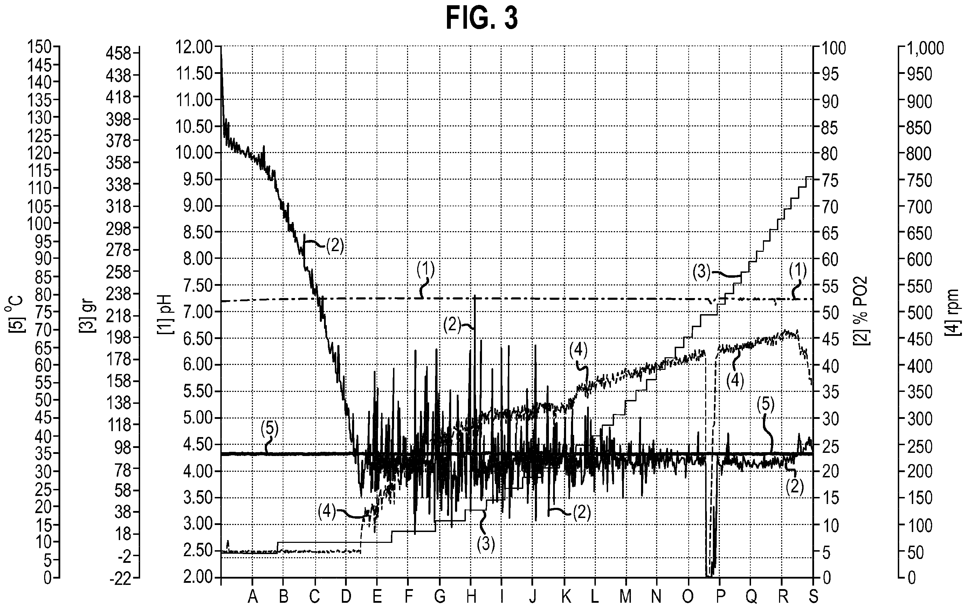

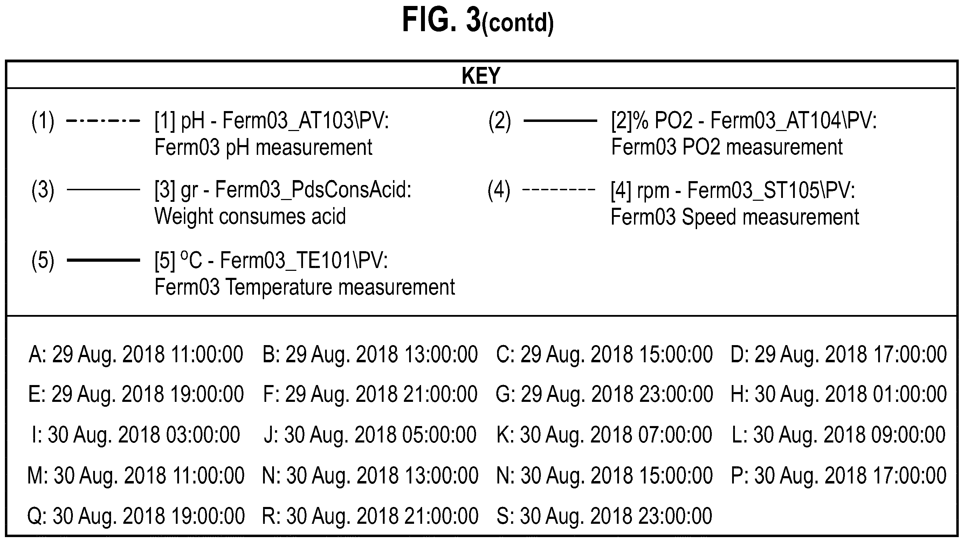

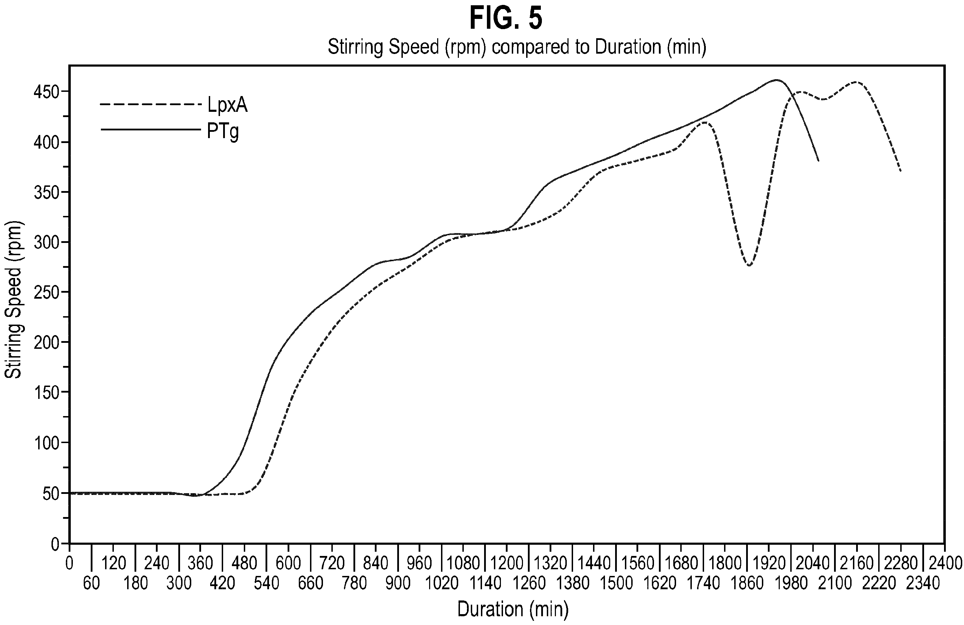

Overall, the fermentation profile of ΔLpxA_{Bpe}/LpxA_{Bpa} was comparable to that of the parental Tohama I PTg strain, with similar fermentation times and elapsed times to DO (Table 5, Figures 3 to 5).

**Table 5. Fermentation parameters for B. pertussis ΔLpxA_{Bpe}/LpxA_{Bpa} and Tohama I PTg.**

| | Δ**LpxA_{Bpe}/LpxA_{Bpa}** | **Mean values for Tohama I PTg (n=3)** |
|---|---|---|
| **Time elapsed to DO regulation (h)** | 9 | 9.1 +/-2.1 |
| **Fermentation duration (h)** | 37.5 | 36.7 +/- 3.7 |
| **Optical density (OD₆₅₀ₙₘ)** | 10.1 | 9.2 +/- 0.3 |
| **Viability (CFU/ml)** | 6.310⁺¹⁰ | 3.6 10⁺¹⁰ +/- 1.1 10⁺¹⁰ |

### OMV production

Outer membrane vesicles (OMV) were produced from bacterial cultures for mass spectrometric analysis of LPS structure as well as *in vitro* stimulation of the human TLR4 receptor. OMV were produced by detergent extraction from bacterial pellets after 24h culture.

Briefly, bacterial pellets were resuspended in 20mM Tris-HCl, 2mM EDTA and 50 U/mL benzonase, pH 8.6 and incubated for 30 min at room temperature. For detergent extraction of OMV, 0.1% DOC was added and the suspension was incubated for 30 min at 40°C. Cellular debris was removed by centrifugation at 20,000g for 30 min (4°C) and supernatant was sterile filtered (0.22µm). Sterile supernatant was then ultracentrifuged (145,000g, 2h, 4°C), resuspended in 1x PBS (with 5mM EDTA), ultracentrifuged a second time (same conditions), resuspended in 1x PBS (without EDTA) and sterile filtered (0.22µm).

### Characterization of LPS structure by mass spectrometry

The OMV preparations were analysed by LC-MS to determine the impact of the genomic mutation of LpxA and/or LpxD and the episomal expression of heterologous LpxA and/or LpxD genes on the structure of the LPS. OMV preparations from the wild-type parental strain Tohama I PTg were used as a control.

Briefly, OMV preparations were precipitated with 95% ethanol, pellets were dried and solubilized in 50% methanol and sonicated. Sonicated material was centrifuged and 5µL of supernatant was injected on the HPLC column for MS analysis of LOS structure.

### LpxA recombinant strains

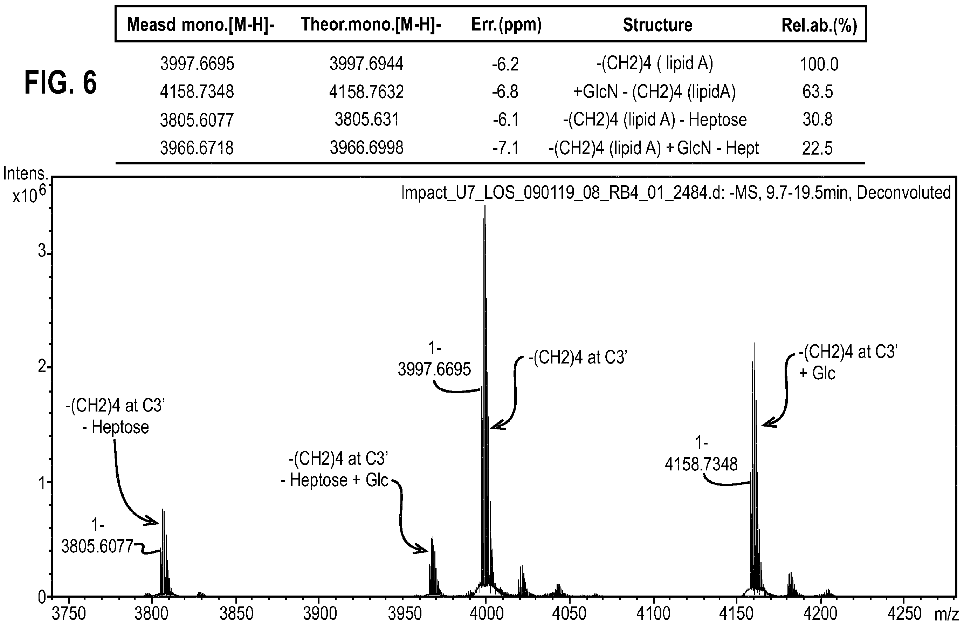

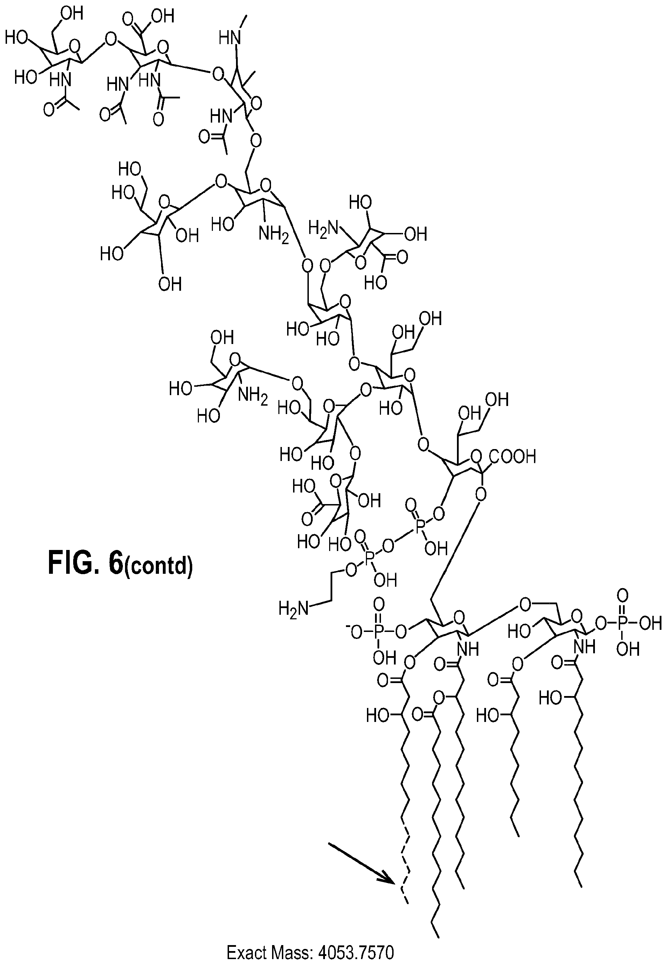

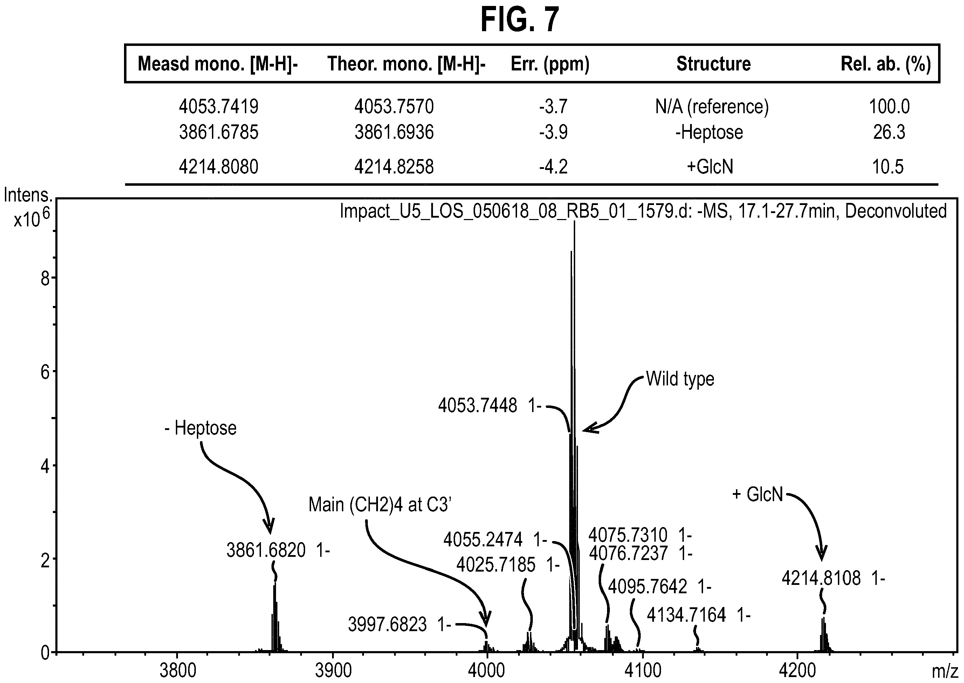

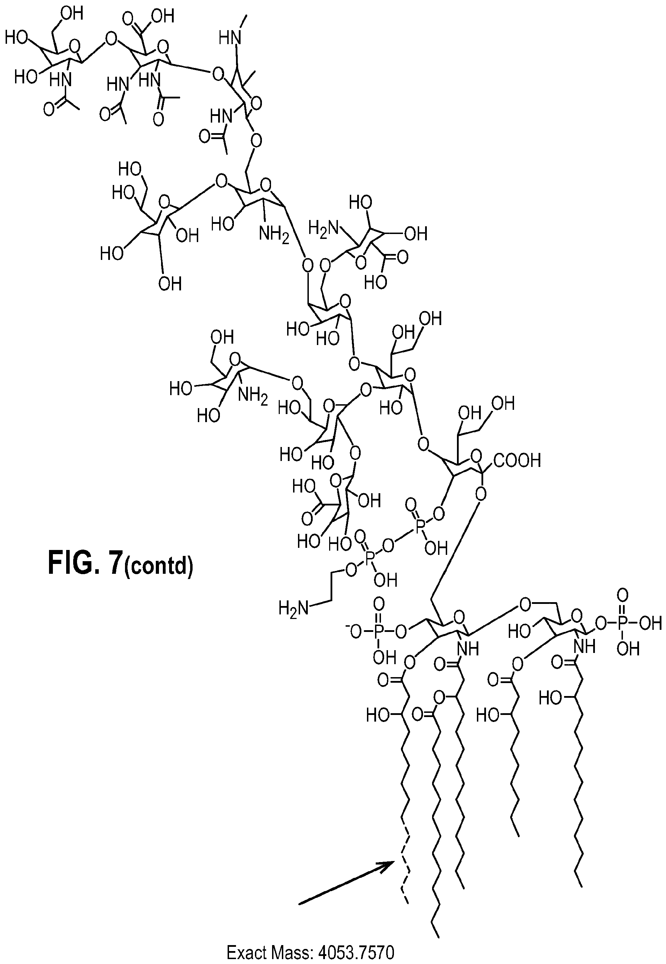

Genomic integration and expression of LpxA_{Bpa} had a significant impact on the LPS structure of the OMV from recombinant *B. pertussis* ΔLpxA_{Bpe}/LpxA_{Bpa}, with 100% of acyl chains at position C3' displaying a length reduction from C₁₄ to C₁₀ (Figure 6). In comparison, expression of episomal LpxA_{Bpa} in *B. pertussis* Tomaha I had a very low, unquantifiable impact (strain LpxA_{Bpa}; Figure 7).

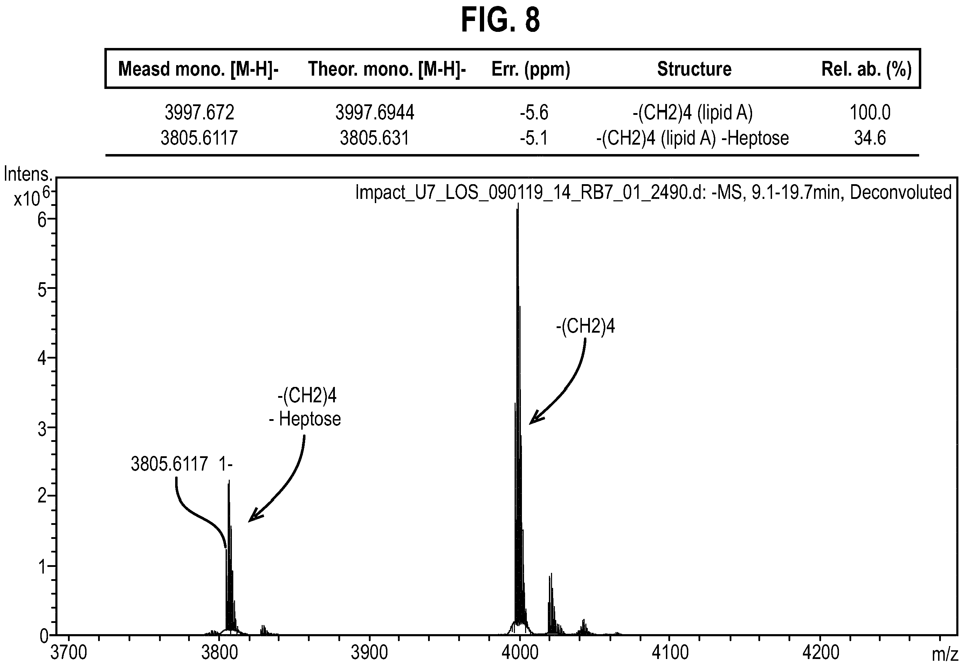

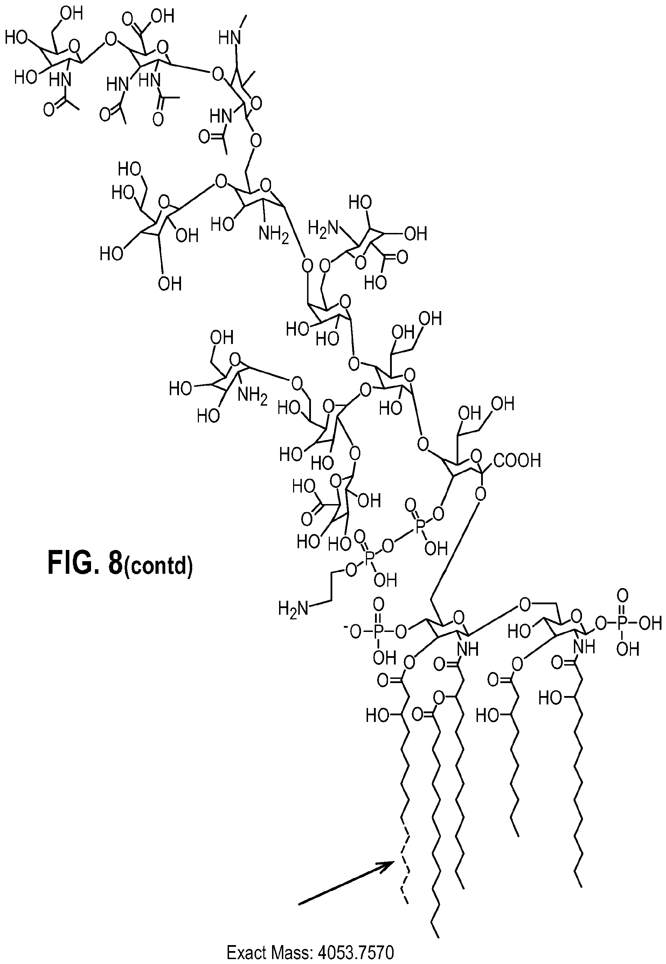

Genomic integration and expression of LpxA_{Bpa} had a similar effect on the LPS structure when combined with ΔArnT, with 100% of acyl chains at position C3' displaying a length reduction from C₁₄ to C₁₀ (Figure 8).

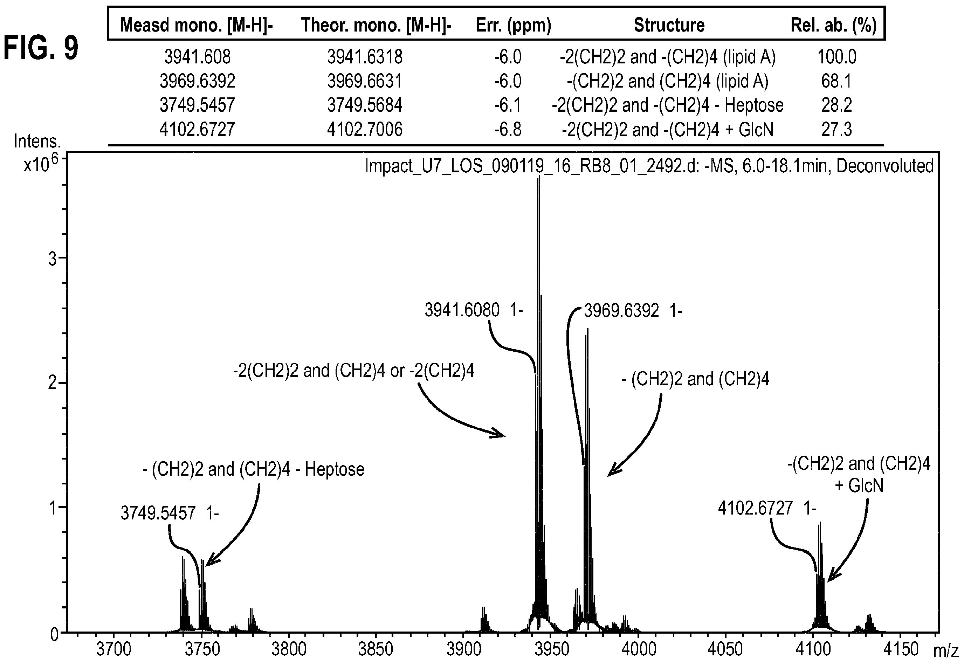

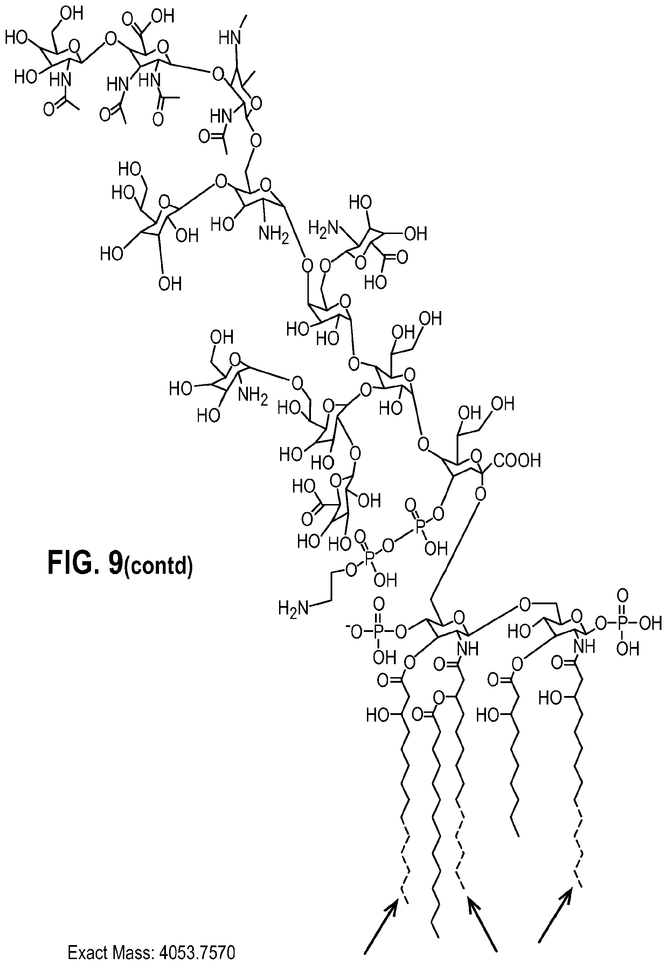

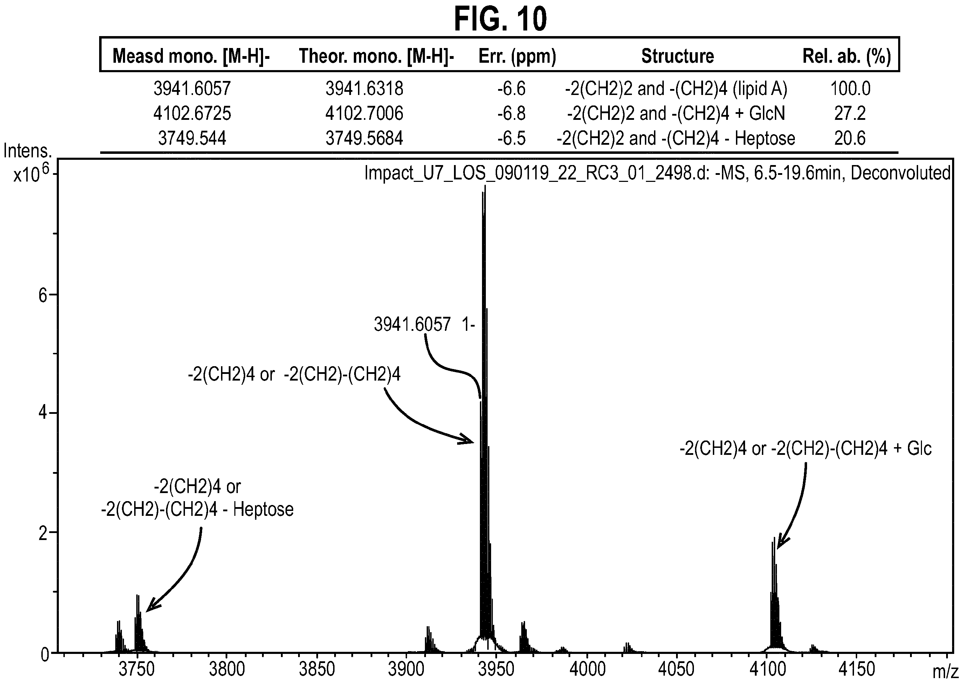

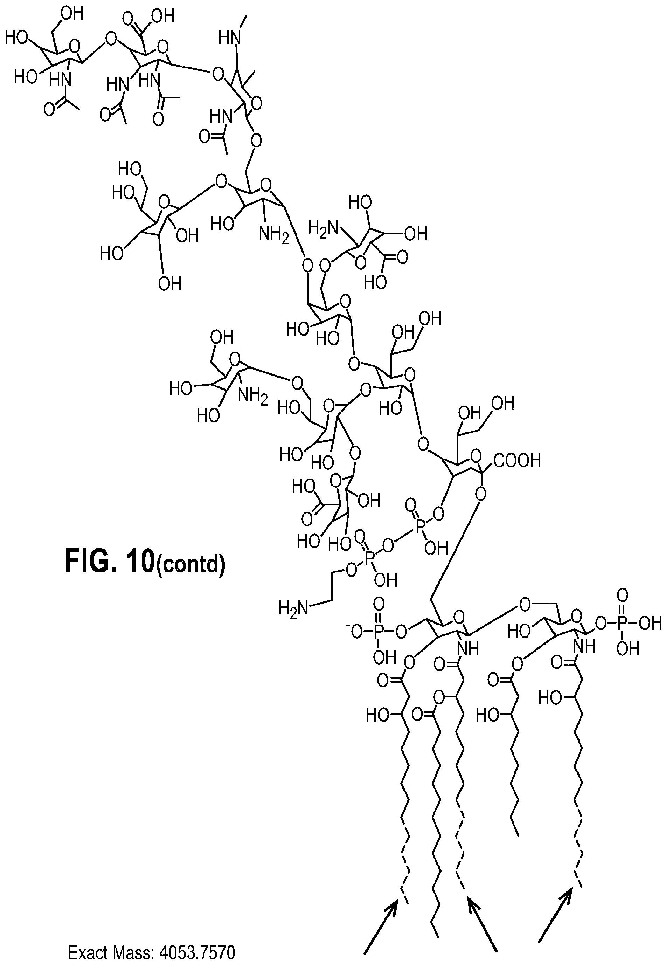

Importantly, genomic integration and expression of LpxA_{Bpa} also had the same effect on the length of the C3' acyl chain in strains ΔLpxA_{Bpe}/LpxA_{Bpa}/ΔDNT/LpxD_{Pa} and ΔLpxA_{Bpe}/LpxA_{Bpa}/ΔDNT/LpxD_{Pa}/ΔLpxD_{Bpe}, in which LpxD from *Pseudomonas* was also expressed (Figures 9 and 10). Expression of the exogenous LpxD gene, in combination with the inactivation of the respective endogenous LpxD had no detrimental effect on the activity of the exogenous LpxA gene. Additionally, no significant growth differences were observed.

### LpxD recombinant strains

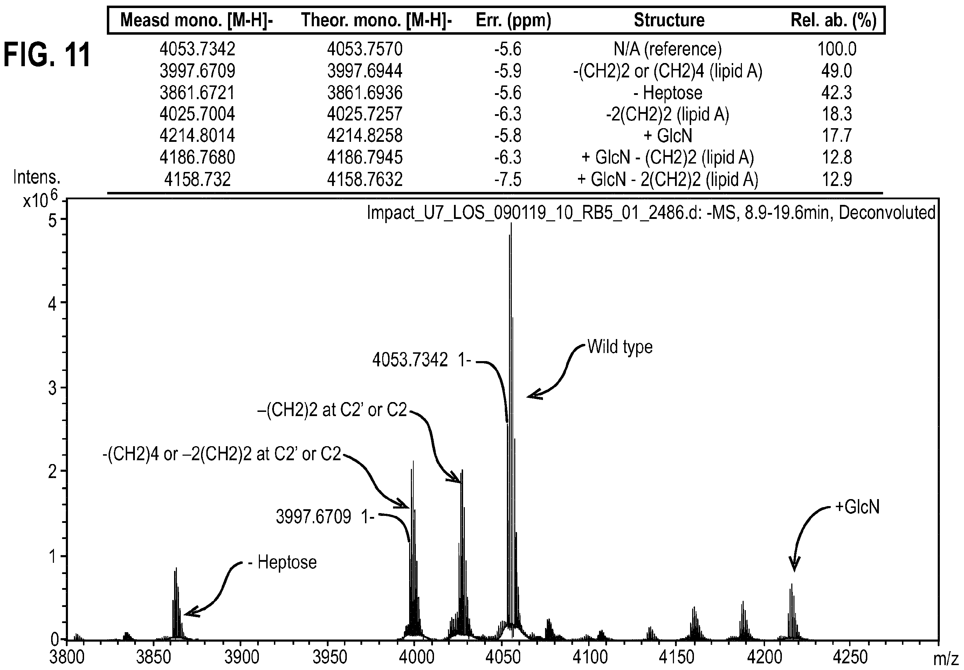

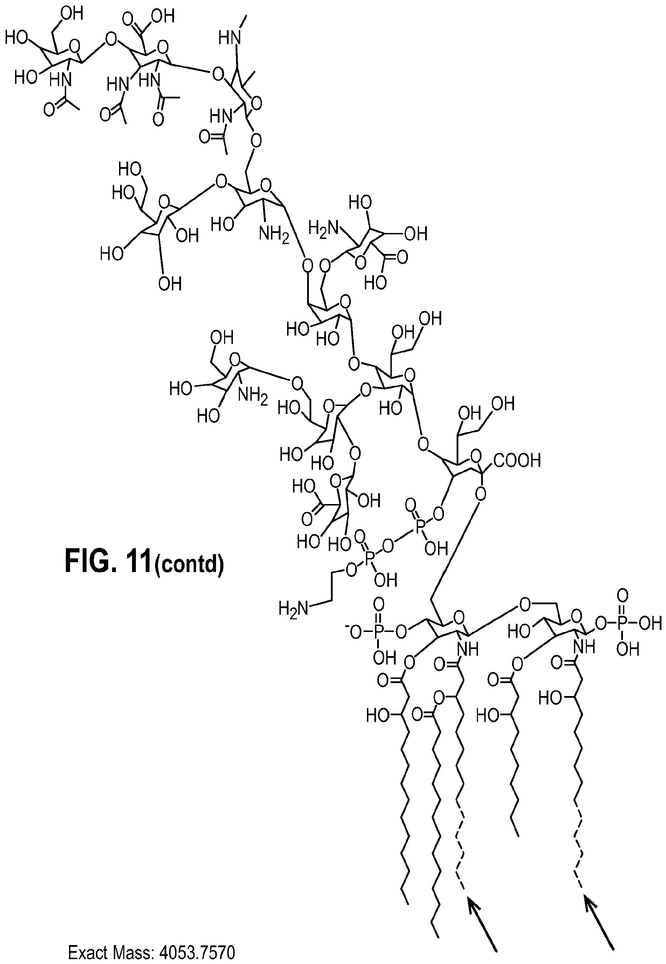

Episomal expression of LpxD from *P. aeruginosa* (LpxD_{Pa}) only had a partial impact on the LPS structure of the OMV from recombinant *B. pertussis* (Figure 11), with just 36% of the total LPS displaying a reduced length at either both of the C2 and C2' acyl chains (24%) or only one of the C2 or C2' acyl chains (12%).

Genomic expression of LpxD_{Pa} increased the impact on LPS, wherein the length of both C2 and C2' acyl chains was reduced in 70% of LPS, and the length of only one of C2 or C2' was reduced in 30% of LPS. Removal of the remaining activity of LpxD_{Bpe} led to 100% chain length reduction at both positions (ΔLpxA_{Bpe}/LpxA_{Bpa}/ΔDNT/LpxD_{Pa}/ΔLpxD_{Bpe}, Figure 10).

### ΔArnT/PagL recombinant strain

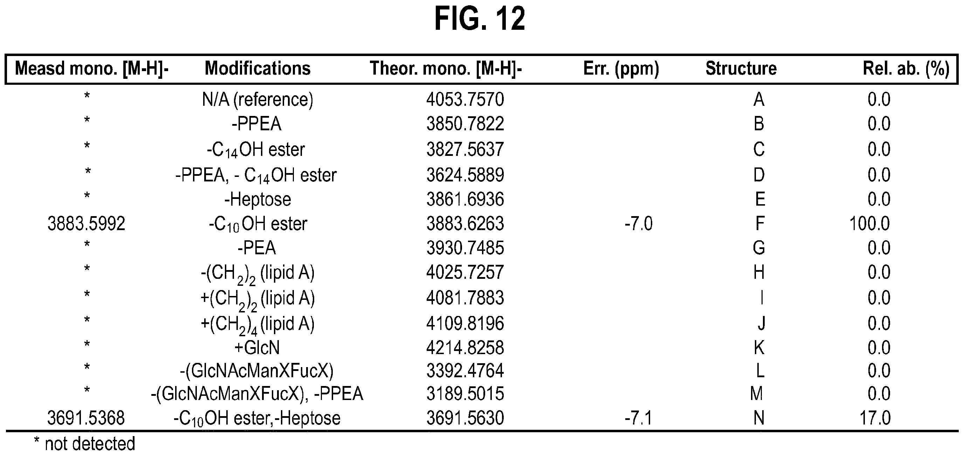

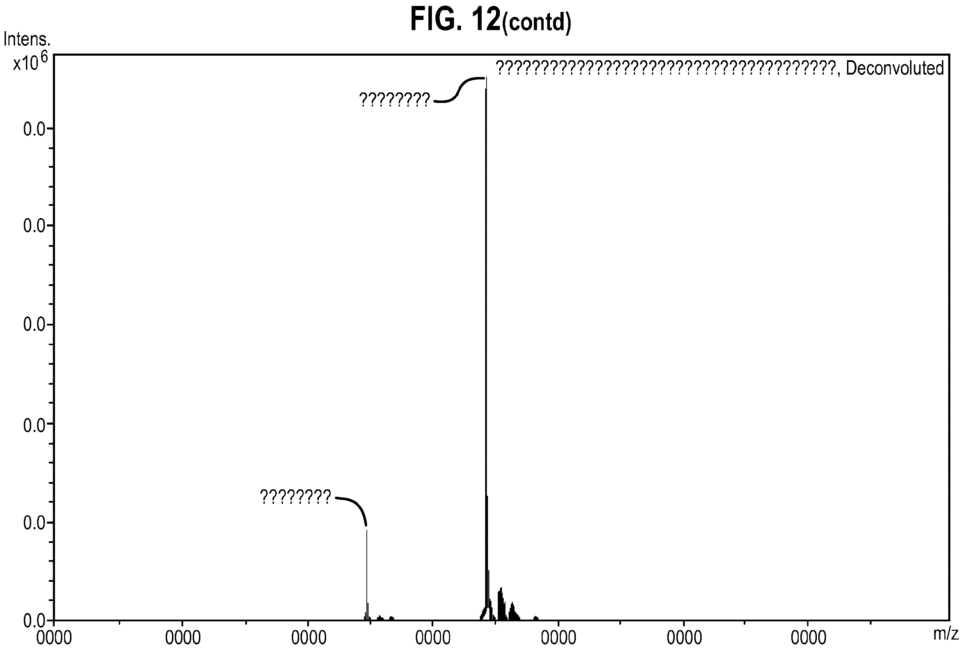

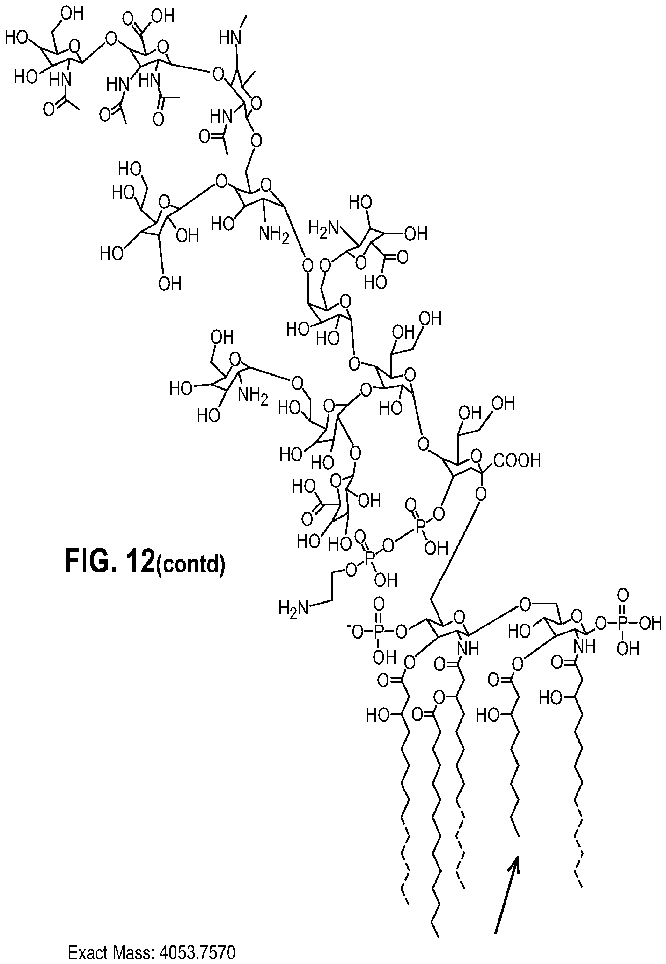

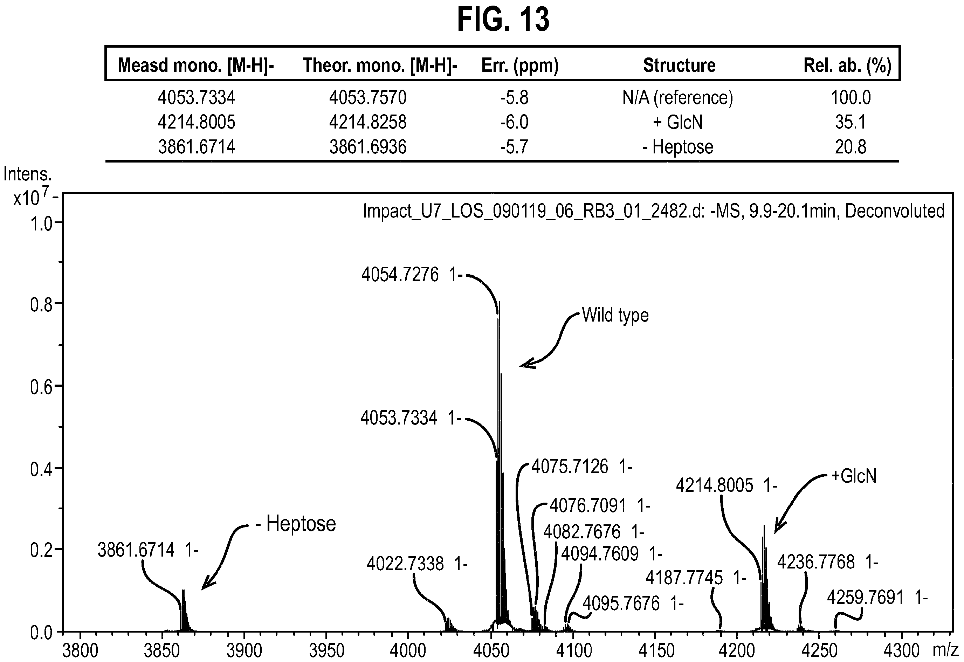

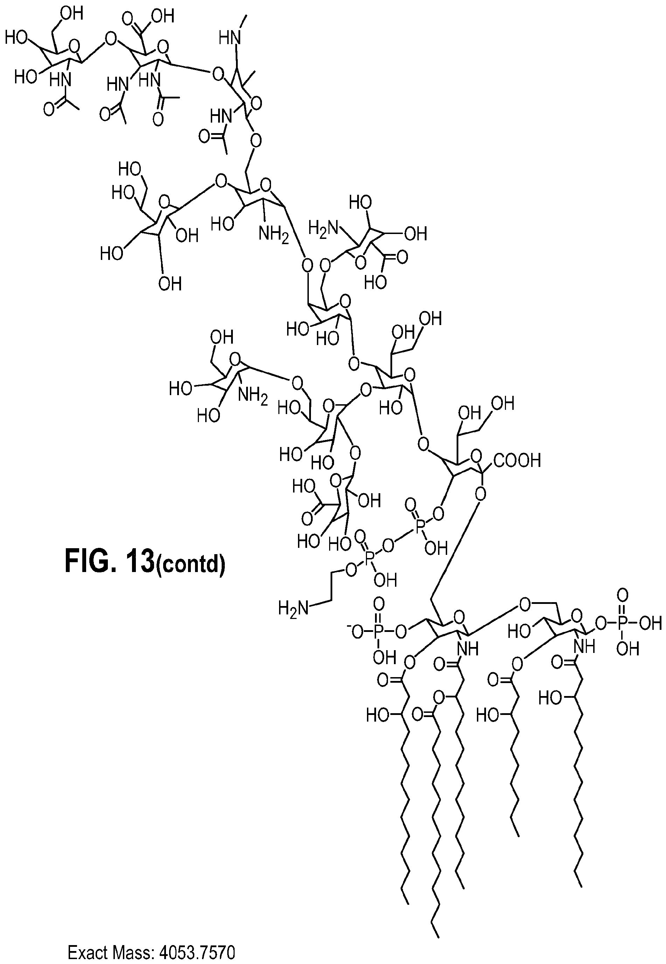

Inactivation of ArnT and genomic integration of PagL led to 100% removal of the C10 ester at C3 (Figure 12). For reference, the structural analysis of OMVs from wild type *B. pertussis* Tomaha I Ptg is shown in Figure 13.

### In vitro evaluation of TLR4 signalling activation on HEK-hTLR4 cells

Stimulation of the TLR4 signalling cascade can lead to the activation of NF-κB and/or activator protein 1 (AP-1) transcription factors, which contribute to the expression of several pro-inflammatory cytokines e.g. IL-1, IL-6, IL-8 and TNF-α and thus play a key role in inflammation.

To analyse whether the signalling activation of the TLR4 pathway decreased when using purified OMVs from recombinant *B. pertussis,* the TLR4 dependant transcriptional activity in HEK-Blue™-hTLR4 cells (Invivogen) was determined.

Upon stimulation with a TLR4 ligand, activation of NF-kB and AP-1 induces the secretion of alkaline phosphatase, which can be detected in the supernatant using the QUANTI-Blue™ colorimetric assay. TLR4 receptor response was determined measuring NF-κB- and AP-1-dependent SEAP production in supernatant measured at 655nm. For each sample, three different dilutions were tested in triplicate. The proportion of signal attributable to TLR4 stimulation is visualized through specific blocking of the TLR4 receptor with an anti-TLR4 antibody, with an unrelated antibody (anti-M72) used as control. The capacity of the OMV obtained from the various recombinant *B. pertussis* strains to activate the TLR4 signalling was compared to that of the OMVs obtained from the parental Tohama I PTg strain (Figures 3 to 6).

For assays normalized by protein content, BEXSERO (MenB vaccine containing MenB OMV), QUINVAXEM (wP vaccine) and INFANRIX HEXA (aP vaccine) were included as controls. For assays normalized by LPS content, a purified LPS from *B. pertussis,* a detoxified (LpxL1 mutant) purified LPS from *Neisseria* and a negative control without stimulation were included.

The HEK-hTLR4 data showed a marked decrease in the activation of the TLR4 signalling cascade by OMVs from the LpxA standalone mutant ΔLpxA_{Bpe}/LpxA_{Bpa}. The activation profile was comparable (± 0.5 OD) to that of INFANRIX HEXA i.e. low activation of the TLR4 signalling cascade.

Similar results were obtained with all the recombinant *B. pertussis* strains with genomic insertion of LpxA_{Bpa}, namely ΔArnT/ΔLpxA_{Bpe}/LpxA_{Bpa}, ΔLpxA_{Bpe}/LpxA_{Bpa}/ΔDNT/LpxD_{Pa}, and ΔLpxA_{Bpe}/LpxA_{Bpa}/ΔDNT/LpxD_{Pa}/ΔLpxD_{Bpe}.

In accordance with their partial effect on the LPS structure, those LpxD mutants in which LpxD was episomally expressed induced a more modest decrease in the TLR4 signalling cascade activation. More specifically, the OD₆₅₅ values observed with the LpxD mutants were higher (≥0.5 OD₆₅₅) than the OD₆₅₅ values observed in the non-stimulated cells and/or to the OD₆₅₅ values observed with cells stimulated with aP vaccine, but lower than the OD₆₅₅ values observed with the cells stimulated with OMV derived from wild type gram-negative bacterium. The reduction was observed in assays using both normalized protein and LPS.

The effect of the ΔArnT mutation in strain ΔArnT/ΔLpxA_{Bpe}/LpxA_{Bpa} cannot be easily derived since the decrease in TLR4 stimulation provided by the ΔLpxA_{Bpe}/LpxA_{Bpa} mutation already saturates the read-out and no further decrease can be observed. With OMV from strain ΔArnT/PagL_{Bbr} on the other hand, an increase in TLR4 stimulation is observed. Given such an increase is not observed in both ΔArnT mutant strains, this increase is hypothesized to be caused by the removal of the C3 acyl chain through PagL_{Bbr} activity.

It will be understood that the invention has been described by way of example only and modifications may be made whilst remaining within the scope and spirit of the invention as defined in the appended claims.

### REFERENCES

[1] Acevedo R. et al., Bacterial outer membrane vesicles and vaccine applications. Front. Immunol. 2014 Mar 24;5:121.
[2] Maeshima N., Fernandez R.C. (2013) Recognition of lipid A variants by the TLR4/MD-2 receptor complex. Frontiers in Cellular and Infection Microbiology 3:3.
[3] WO2018/167061
[4] Sweet C.R., Preston A., Toland E., Ramirez S.M., Cotter R.J., Maskell D.J., Raetz C.R. (2002) Relaxed acyl chain specificity of Bordetella UDP-N-acetylglucosamine acyltransferases. Journal of Biological Chemistry 277(21):18281-90.
[5] Bartling C.M., Raetz C.R. (2009) Crystal structure and acyl chain selectivity of Escherichia coli LpxD, the N-acyltransferase of lipid A biosynthesis. Biochemistry 48(36):8672-83
[6] Steeghs L, Berns M, ten Hove J, de Jong A, Roholl P, van Alphen L, Tommassen J, van der Ley P. (2002) Expression of foreign LpxA acyltransferases in Neisseria meningitidis results in modified lipid A with reduced toxicity and retained adjuvant activity. Cell Microbiol. 2002 Sep;4(9):599-611.
[7] EP0011243
[8] Fredriksen et al. (1991) Production, characterization and control of MenB-vaccine "Folkehelsa": an outer membrane vesicle vaccine against group B meningococcal disease..NIPH Ann. 14(2):67-80
[9] WO2004/019977
[10] US6,558,677
[11] WO2005/004908
[12] WO2011/036562
[13] Gennaro (2000) Remington: The Science and Practice of Pharmacy. 20th edition, ISBN: 0683306472.
[14] Vaccine Design (1995) eds. Powell & Newman. ISBN: 030644867X. Plenum.
[15] WO90/14837.
[16] Module 1 of WHO's The immunological basis for immunization series (Galazka)
[17] Lyng (1990) Biologicals 18:11-17
[18] NIBSC code: 69/017
[19] Kuhmlann & Rieger (1995) Immunol Infect Dis 5:10-4
[20] Sesardic et al. (2002) Biologicals 30:49-68
[21] NIBSC code: 98/552
[22] Lindberg (1999) Vaccine 17 Suppl 2:S28-36
[23] Buttery & Moxon (2000) J R Coll Physicians Lond 34:163-168
[24] Ahmad & Chapnick (1999) Infect Dis Clin North Am 13:113-133, vii
[25] Goldblatt (1998) J. Med. Microbiol. 47:563-567
[26] EP-A-0477508
[27] US patent 5,306,492
[28] WO1998/42721
[29] Conjugate Vaccines (eds. Cruse et al.) ISBN 3805549326, particularly vol. 10:48-114
[30] Lees et al. (1996) Vaccine 14:190-198
[31] WO1995/08348
[32] WO1998/42721
[33] US patent 4,882,317
[34] US patent 4,695,624
[35] EP-B-0477508
[36] Mol. Immunol. 1985, 22, 907-919
[37] EP-A-0208375
[38] WO2000/10599
[39] Gever et al. (1979) Med. Microbiol. Immunol. 165:171-288
[40] US patent 4,057,685
[41] US patent 4,673,574
[42] US patent 4,761,283;
[43] US patent 4,808,700
[44] US patent 4,459,286
[45] US patent 4,965,338
[46] US patent 4,663,160
[47] US patent 4,356,170
[48] Research Disclosure, 453077 (Jan 2002)
[49] Anderson (1983) Infect Immun 39(1):233-238
[50] Anderson et al. (1985) J Clin Invest 76(1):52-59
[51] Kanra et al. (1999) The Turkish Journal of Paediatrics 42:421-427
[52] Ravenscroft et al. (2000) Dev Biol (Basel) 103: 35-47
[53] WO96/40242
[54] Verez-Bencomo V, Fernández-Santana V, Hardy E, Toledo ME, Rodríguez MC, Heynngnezz L, Rodriguez A, Baly A, Herrera L, Izquierdo M, Villar A, Valdés Y, Cosme K, Deler ML, Montane M, Garcia E, Ramos A, Aguilar A, Medina E, Toraño G, Sosa I, Hernandez I, Martínez R, Muzachio A, Carmenates A, Costa L, Cardoso F, Campa C, Diaz M, Roy R. A synthetic conjugate polysaccharide vaccine against Haemophilus influenzae type b. Science. 2004 Jul 23;305(5683):522-5.
[55] WO2018/020046
[56] Vanlandschoot et al. (2005) J Gen Virol 86:323-31
[57] WO2007/054820
[58] WO2003/066094
[59] Module 6 of WHO's The immunological basis for immunization series (Robertson)
[60] WO2008/028956
[61] WO2008/028957
[62] Liao et al. (2012) J Infect Dis. 205:237-43
[63] Stibitz S, Black W, Falkow S. The construction of a cloning vector designed for gene replacement in Bordetella pertussis. Gene. 1986;50(1-3):133-140.
[64] Goffin P, Slock T, Smessaert V, De Rop P, Dehottay P. A versatile, non genetically modified organism (GMO)-based strategy for controlling low-producer mutants in Bordetella pertussis cultures using antigenic modulation. Biotechnol J. 2015 Aug;10(8):1269-80.

## Claims

1. A recombinant *Bordetella pertussis* bacterium which comprises:
(i) at least one genomic LpxA gene encoding an LpxA protein, wherein the LpxA protein comprises a mutation at position 170 and/or a mutation at position 229 relative to SEQ ID NO: 1; and/or
(ii)at least one genomic insertion of a heterologous LpxD gene.

2. The recombinant Bordetella pertussis bacterium of Claim 1 which comprises at least one genomic LpxA gene encoding an LpxA protein, wherein the LpxA protein comprises a substitution at position 170 and/or a substitution at position 229 relative to SEQ ID NO: 1.

3. The recombinant Bordetella pertussis bacterium of Claim 1 or 2 which comprises a Serine residue at position 170 and/or an Alanine residue at position 229 relative to SEQ ID NO: 1.

4. The recombinant Bordetella pertussis bacterium of Claim 3, which comprises (i) a Serine residue at position 170 and (ii) an Alanine residue at position 229 when numbered in accordance with SEQ ID NO:1.

5. The recombinant Bordetella pertussis bacterium of Claim 4 which comprises an LpxA gene that encodes an LpxA protein having at least 80% amino acid sequence identity to SEQ ID NO: 1 or 2.

6. The recombinant Bordetella pertussis bacterium of any preceding claim, wherein the heterologous LpxD gene encodes an LpxD protein having at least 90% amino acid sequence identity with SEQ ID NO:3 or SEQ ID NO: 4.

7. The recombinant Bordetella pertussis bacterium of Claim 6, wherein the heterologous LpxD gene encodes an LpxD protein having at least 95% amino acid sequence identity with SEQ ID NO:3 or SEQ ID NO: 4.

8. The recombinant Bordetella pertussis bacterium of Claim 7, wherein the heterologous LpxD gene encodes an LpxD protein having an amino acid sequence selected from the group consisting of SEQ ID NO:3 or SEQ ID NO: 4.

9. The recombinant Bordetella pertussis bacterium of any preceding claim, wherein the endogenous LpxA gene is inactivated and/or the endogenous LpxD gene is inactivated.

10. The recombinant Bordetella pertussis bacterium of any one of the preceding claims which produces lipid A wherein (i) at least 30% of the C3' acyl chains are from about C₁₀ to about C₁₂ in length; and/or (ii) at least 30% of the C2' acyl chains are from about C₁₀ to about C₁₂ in length; and/or (iii) at least 30% of the C2 acyl chains are from about C₁₀ to about C₁₂ in length.

11. The recombinant Bordetella pertussis bacterium of any one of the preceding claims that produces Lipid A with reduced endotoxic activity compared to that of the Lipid A produced by the parental strain, particularly when measured using TLR4 stimulation assays, particularly human TLR4 stimulation assays.

12. The recombinant Bordetella pertussis bacterium of any one of the preceding claims, wherein the growth curve of a bacterial population of said bacteria is comparable to the growth curve of a population of the parental strain.

13. An isolated outer membrane vesicle (OMV) derived from the recombinant Bordetella pertussis bacterium of any one of Claims 1 to 12 which comprises modified Lipid A incorporated into the membrane wherein substantially all of C3' acyl chains of the Lipid A have a length of C₁₀ and/or wherein substantially all of C2 and C2' acyl chains of the Lipid A have a length of C₁₂.

14. The isolated outer membrane vesicle according to Claim 13 which is substantially free of dermonecrotic toxin and/or which contains endogenous genetically detoxified pertussis toxoid.

15. An immunogenic composition comprising at least one isolated OMV according to Claim 13 or 14 and a pharmaceutically acceptable excipient.

16. The immunogenic composition of Claim 15, further comprising at least one additional antigen selected from the group consisting of (1) pertussis toxoid (PT), (2) FHA, (3) pertactin (PRN), (4) FIM2/FIM3, (5) adenylate cyclase, (6) diphtheria toxoid (DT), (7) tetanus toxoid (TT), (8) inactivated polio virus (IPV), (9) hepatitis B surface antigen and (10) Hib PRP.

17. The OMV of claim 13 or 14 or the immunogenic composition of claim 15 or 16 for use in inducing an immune response in a suitable mammal, for example a human.

18. The OMV of claim 13 or 14 or the immunogenic composition of claim 15 or 16 for use in prophylaxis or as a vaccine.

19. A method of modulating the reactogenicity of the Lipid A of a *Bordetella pertussis* bacterium, comprising stably integrating into the genome of the bacterium at least one gene selected from the group consisting of LpxA and LpxD, wherein:
(i) the LpxA gene encodes an LpxA protein having at least 80% sequence identity with SEQ ID NO: 1 or 2 and wherein the protein comprises Serine at position 170 (S170) and/or Alanine at position 229 (A229) when numbered in accordance with SEQ ID NO: 1 or 2; and/or
(ii) the LpxD gene encodes an LpxD protein having at least 90% amino acid sequence identity with SEQ ID NO:3 or SEQ ID NO: 4.
